# EUROPEAN PATENT SPECIFICATION

(11) **EP 3 174 897 B1**
(45) Date of publication and mention of the grant of the patent: **12.02.2020**
(21) Application number: 15750278.2
(22) Date of filing: 29.07.2015
(51) Int. Cl.: C07K 16/00, C07K 16/22, C07K 16/24, C07K 16/28, C07K 16/32, C07K 16/46

(54) **MULTISPECIFIC ANTIBODIES**
MULTISPEZIFISCHE ANTIKÖRPER
ANTICORPS MULTI-SPÉCIFIQUES

(30) Priority: 29.07.2014 EP 14178908
(43) Date of publication of application: 07.06.2017
(73) Proprietor: F. Hoffmann-La Roche AG, 4070 Basel (CH)
(72) Inventor: IMHOF-JUNG, Sabine, 82152 Planegg (DE); KLEIN, Christian, CH-8906 Bonstetten (CH); KLOSTERMANN, Stefan, 82061 Neuried (DE); REGULA, Joerg, Thomas, 81377 Muenchen (DE); SCHAEFER,Wolfgang, 68199 Mannheim (DE)
(74) Representative: Burger, Alexander
(86) International application number: PCT/EP2015/067369
(87) International publication number: WO 2016/016299

(56) References cited:
- EP-A1- 2 647 707
- WO-A1-2013/174873
- WO-A1-2014/049003
- WO-A1-2014/081955
- STEVEN M LEWIS ET AL: "Generation of bispecific IgG antibodies by structure-based design of an orthogonal Fab interface", NATURE BIOTECHNOLOGY, vol. 32, no. 2, 1 February 2014 (2014-02-01), pages 191-198, XP055122831, ISSN: 1087-0156, DOI: 10.1038/nbt.2797

### SEQUENCE LISTING

<110> F. Hoffmann-La Roche AG
<120> Multispecific antibodies
<130> P32237-WO
<150> EP 14178908.1
   <151> 2014-07-29
<160> 51
<170> PatentIn version 3.5
<210> 1
   <211> 439
   <212> PRT
   <213> Artificial Sequence
<220>
   <223> heavy chain (HC) <VEGF> with VL-VH/CL-CH1 domain exchange wild type (wt)
<400> 1
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Asp Lys Thr His Thr Cys Pro Pro Cys Pro
   210 215 220
   Ala Pro Glu Leu Leu Gly Gly Pro Ser Val Phe Leu Phe Pro Pro Lys
   225 230 235 240
   Pro Lys Asp Thr Leu Met Ile Ser Arg Thr Pro Glu Val Thr Cys Val
   245 250 255
   Val Val Asp Val Ser His Glu Asp Pro Glu Val Lys Phe Asn Trp Tyr
   260 265 270
   Val Asp Gly Val Glu Val His Asn Ala Lys Thr Lys Pro Arg Glu Glu
   275 280 285
   Gln Tyr Asn Ser Thr Tyr Arg Val Val Ser Val Leu Thr Val Leu His
   290 295 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Cys Thr Leu Pro Pro Ser Arg Asp Glu Leu
   340 345 350
   Thr Lys Asn Gln Val Ser Leu Ser Cys Ala Val Lys Gly Phe Tyr Pro
   355 360 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Val Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro
   435
<210> 2
   <211> 226
   <212> PRT
   <213> artificial
<220>
   <223> light chain (LC) <VEGF> with VL-VH/CL-CH1 domain exchange wild type (wt)
<400> 2
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Thr Phe Thr Asn Tyr
   20 25 30
   Gly Met Asn Trp Val Arg Gln Ala Pro Gly Lys Gly Leu Glu Trp Val
   35 40 45
   Gly Trp Ile Asn Thr Tyr Thr Gly Glu Pro Thr Tyr Ala Ala Asp Phe
   50 55 60
   Lys Arg Arg Phe Thr Phe Ser Leu Asp Thr Ser Lys Ser Thr Ala Tyr
   65 70 75 80
   Leu Gln Met Asn Ser Leu Arg Ala Glu Asp Thr Ala Val Tyr Tyr Cys
   85 90 95
   Ala Lys Tyr Pro His Tyr Tyr Gly Ser Ser His Trp Tyr Phe Asp Val
   100 105 110
   Trp Gly Gln Gly Thr Leu Val Thr Val Ser Ser Ala Ser Thr Lys Gly
   115 120 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys
   225
<210> 3
   <211> 457
   <212> PRT
   <213> Artificial Sequence
<220>
   <223> heavy chain (HC) <Ang-2> wild type (wt)
<400> 3
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
   130 135 140
   Lys Ser Thr Ser Gly Gly Thr Ala Ala Leu Gly Cys Leu Val Lys Asp
   145 150 155 160
   Tyr Phe Pro Glu Pro Val Thr Val Ser Trp Asn Ser Gly Ala Leu Thr
   165 170 175
   Ser Gly Val His Thr Phe Pro Ala Val Leu Gln Ser Ser Gly Leu Tyr
   180 185 190
   Ser Leu Ser Ser Val Val Thr Val Pro Ser Ser Ser Leu Gly Thr Gln
   195 200 205
   Thr Tyr Ile Cys Asn Val Asn His Lys Pro Ser Asn Thr Lys Val Asp
   210 215 220
   Lys Lys Val Glu Pro Lys Ser Cys Asp Lys Thr His Thr Cys Pro Pro
   225 230 235 240
   Cys Pro Ala Pro Glu Leu Leu Gly Gly Pro Ser Val Phe Leu Phe Pro
   245 250 255
   Pro Lys Pro Lys Asp Thr Leu Met Ile Ser Arg Thr Pro Glu Val Thr
   260 265 270
   Cys Val Val Val Asp Val Ser His Glu Asp Pro Glu Val Lys Phe Asn
   275 280 285
   Trp Tyr Val Asp Gly Val Glu Val His Asn Ala Lys Thr Lys Pro Arg
   290 295 300
   Glu Glu Gln Tyr Asn Ser Thr Tyr Arg Val Val Ser Val Leu Thr Val
   305 310 315 320
   Leu His Gln Asp Trp Leu Asn Gly Lys Glu Tyr Lys Cys Lys Val Ser
   325 330 335
   Asn Lys Ala Leu Pro Ala Pro Ile Glu Lys Thr Ile Ser Lys Ala Lys
   340 345 350
   Gly Gln Pro Arg Glu Pro Gln Val Tyr Thr Leu Pro Pro Cys Arg Asp
   355 360 365
   Glu Leu Thr Lys Asn Gln Val Ser Leu Trp Cys Leu Val Lys Gly Phe
   370 375 380
   Tyr Pro Ser Asp Ile Ala Val Glu Trp Glu Ser Asn Gly Gln Pro Glu
   385                 390                 395                 400
   Asn Asn Tyr Lys Thr Thr Pro Pro Val Leu Asp Ser Asp Gly Ser Phe
                   405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro
       450                 455
<210> 4
   <211> 215
   <212> PRT
   <213> Artificial Sequence
<220>
   <223> kappa light chain (LC) <Ang-2> wild type (wt)
<400> 4
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 5
   <211> 457
   <212> PRT
   <213> Artificial Sequence
<220>
   <223> heavy chain (HC) <Ang-2> with K213E and K147E substitutions
<400> 5
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Glu Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Glu Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
               340                 345                 350
   Gly Gln Pro Arg Glu Pro Gln Val Tyr Thr Leu Pro Pro Cys Arg Asp
           355                 360                 365
   Glu Leu Thr Lys Asn Gln Val Ser Leu Trp Cys Leu Val Lys Gly Phe
   370 375 380
   Tyr Pro Ser Asp Ile Ala Val Glu Trp Glu Ser Asn Gly Gln Pro Glu
   385 390 395 400
   Asn Asn Tyr Lys Thr Thr Pro Pro Val Leu Asp Ser Asp Gly Ser Phe
   405 410 415
   Phe Leu Tyr Ser Lys Leu Thr Val Asp Lys Ser Arg Trp Gln Gln Gly
   420 425 430
   Asn Val Phe Ser Cys Ser Val Met His Glu Ala Leu His Asn His Tyr
   435 440 445
   Thr Gln Lys Ser Leu Ser Leu Ser Pro
   450 455
<210> 6
   <211> 215
   <212> PRT
   <213> Artificial Sequence
<220>
   <223> kappa light chain (LC) <Ang-2> with E123K and Q124K substitutions
<400> 6
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Arg Thr Val Ala
               100                 105                 110
   Ala Pro Ser Val Phe Ile Phe Pro Pro Ser Asp Lys Lys Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 7
   <211> 439
   <212> PRT
   <213> Artificial Sequence
<220>
   <223> heavy chain (HC) <VEGF> with VL-VH/CL-CH1 domain exchange with Q124E substitution
<400> 7
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Glu Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Asp Lys Thr His Thr Cys Pro Pro Cys Pro
   210 215 220
   Ala Pro Glu Leu Leu Gly Gly Pro Ser Val Phe Leu Phe Pro Pro Lys
   225 230 235 240
   Pro Lys Asp Thr Leu Met Ile Ser Arg Thr Pro Glu Val Thr Cys Val
   245 250 255
   Val Val Asp Val Ser His Glu Asp Pro Glu Val Lys Phe Asn Trp Tyr
   260 265 270
   Val Asp Gly Val Glu Val His Asn Ala Lys Thr Lys Pro Arg Glu Glu
   275 280 285
   Gln Tyr Asn Ser Thr Tyr Arg Val Val Ser Val Leu Thr Val Leu His
   290 295 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Cys Thr Leu Pro Pro Ser Arg Asp Glu Leu
   340 345 350
   Thr Lys Asn Gln Val Ser Leu Ser Cys Ala Val Lys Gly Phe Tyr Pro
   355 360 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Val Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro
   435
<210> 8
   <211> 439
   <212> PRT
   <213> Artificial Sequence
<220>
   <223> heavy chain (HC) <VEGF> with VL-VH/CL-CH1 domain exchange with Q124E and Q38E substitutions
<400> 8
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Glu Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Glu Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Asn Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Cys Thr Leu Pro Pro Ser Arg Asp Glu Leu
               340                 345                 350
   Thr Lys Asn Gln Val Ser Leu Ser Cys Ala Val Lys Gly Phe Tyr Pro
           355                 360                 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Val Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro
   435
<210> 9
   <211> 226
   <212> PRT
   <213> Artificial Sequence
<220>
   <223> light chain (LC) <VEGF> with VL-VH/CL-CH1 domain exchange with Q39K substitution
<400> 9
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Thr Phe Thr Asn Tyr
   20 25 30
   Gly Met Asn Trp Val Arg Lys Ala Pro Gly Lys Gly Leu Glu Trp Val
   35 40 45
   Gly Trp Ile Asn Thr Tyr Thr Gly Glu Pro Thr Tyr Ala Ala Asp Phe
   50 55 60
   Lys Arg Arg Phe Thr Phe Ser Leu Asp Thr Ser Lys Ser Thr Ala Tyr
   65 70 75 80
   Leu Gln Met Asn Ser Leu Arg Ala Glu Asp Thr Ala Val Tyr Tyr Cys
   85 90 95
   Ala Lys Tyr Pro His Tyr Tyr Gly Ser Ser His Trp Tyr Phe Asp Val
   100 105 110
   Trp Gly Gln Gly Thr Leu Val Thr Val Ser Ser Ala Ser Thr Lys Gly
   115 120 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Lys Lys Val Glu Pro Lys
   210 215 220
   Ser Cys
   225
<210> 10
   <211> 457
   <212> PRT
   <213> Artificial Sequence
<220>
   <223> heavy chain (HC) <Ang-2> with K213E, K147E and Q39E substitutions
<400> 10
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Glu Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Glu Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Glu Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
               340                 345                 350
   Gly Gln Pro Arg Glu Pro Gln Val Tyr Thr Leu Pro Pro Cys Arg Asp
   355 360 365
   Glu Leu Thr Lys Asn Gln Val Ser Leu Trp Cys Leu Val Lys Gly Phe
   370 375 380
   Tyr Pro Ser Asp Ile Ala Val Glu Trp Glu Ser Asn Gly Gln Pro Glu
   385 390 395 400
   Asn Asn Tyr Lys Thr Thr Pro Pro Val Leu Asp Ser Asp Gly Ser Phe
   405 410 415
   Phe Leu Tyr Ser Lys Leu Thr Val Asp Lys Ser Arg Trp Gln Gln Gly
   420 425 430
   Asn Val Phe Ser Cys Ser Val Met His Glu Ala Leu His Asn His Tyr
   435 440 445
   Thr Gln Lys Ser Leu Ser Leu Ser Pro
   450 455
<210> 11
   <211> 215
   <212> PRT
   <213> Artificial Sequence
<220>
   <223> kappa light chain (LC) <Ang-2> with E123K, Q124K and Q38K substitutions
<400> 11
   Gln Pro Gly Leu Thr Gln Pro Pro Ser Val Ser Val Ala Pro Gly Gln
   1 5 10 15
   Thr Ala Arg Ile Thr Cys Gly Gly Asn Asn Ile Gly Ser Lys Ser Val
   20 25 30
   His Trp Tyr Gln Lys Lys Pro Gly Gln Ala Pro Val Leu Val Val Tyr
   35 40 45
   Asp Asp Ser Asp Arg Pro Ser Gly Ile Pro Glu Arg Phe Ser Gly Ser
   50 55 60
   Asn Ser Gly Asn Thr Ala Thr Leu Thr Ile Ser Arg Val Glu Ala Gly
   65 70 75 80
   Asp Glu Ala Asp Tyr Tyr Cys Gln Val Trp Asp Ser Ser Ser Asp His
   85 90 95
   Tyr Val Phe Gly Thr Gly Thr Lys Val Thr Val Leu Arg Thr Val Ala
   100 105 110
   Ala Pro Ser Val Phe Ile Phe Pro Pro Ser Asp Lys Lys Leu Lys Ser
   115 120 125
   Gly Thr Ala Ser Val Val Cys Leu Leu Asn Asn Phe Tyr Pro Arg Glu
   130 135 140
   Ala Lys Val Gln Trp Lys Val Asp Asn Ala Leu Gln Ser Gly Asn Ser
   145 150 155 160
   Gln Glu Ser Val Thr Glu Gln Asp Ser Lys Asp Ser Thr Tyr Ser Leu
   165 170 175
   Ser Ser Thr Leu Thr Leu Ser Lys Ala Asp Tyr Glu Lys His Lys Val
   180 185 190
   Tyr Ala Cys Glu Val Thr His Gln Gly Leu Ser Ser Pro Val Thr Lys
   195 200 205
   Ser Phe Asn Arg Gly Glu Cys
   210 215
<210> 12
   <211> 214
   <212> PRT
   <213> Artificial Sequence
<220>
   <223> lambda light chain (LC) <Ang-2> wild type (wt)
<400> 12
   Gln Pro Gly Leu Thr Gln Pro Pro Ser Val Ser Val Ala Pro Gly Gln
   1 5 10 15
   Thr Ala Arg Ile Thr Cys Gly Gly Asn Asn Ile Gly Ser Lys Ser Val
   20 25 30
   His Trp Tyr Gln Gln Lys Pro Gly Gln Ala Pro Val Leu Val Val Tyr
   35 40 45
   Asp Asp Ser Asp Arg Pro Ser Gly Ile Pro Glu Arg Phe Ser Gly Ser
   50 55 60
   Asn Ser Gly Asn Thr Ala Thr Leu Thr Ile Ser Arg Val Glu Ala Gly
   65 70 75 80
   Asp Glu Ala Asp Tyr Tyr Cys Gln Val Trp Asp Ser Ser Ser Asp His
   85 90 95
   Tyr Val Phe Gly Thr Gly Thr Lys Val Thr Val Leu Gly Gln Pro Lys
   100 105 110
   Ala Ala Pro Ser Val Thr Leu Phe Pro Pro Ser Ser Glu Glu Leu Gln
   115 120 125
   Ala Asn Lys Ala Thr Leu Val Cys Leu Ile Ser Asp Phe Tyr Pro Gly
   130 135 140
   Ala Val Thr Val Ala Trp Lys Ala Asp Ser Ser Pro Val Lys Ala Gly
   145 150 155 160
   Val Glu Thr Thr Thr Pro Ser Lys Gln Ser Asn Asn Lys Tyr Ala Ala
   165 170 175
   Ser Ser Tyr Leu Ser Leu Thr Pro Glu Gln Trp Lys Ser His Arg Ser
   180 185 190
   Tyr Ser Cys Gln Val Thr His Glu Gly Ser Thr Val Glu Lys Thr Val
   195 200 205
   Ala Pro Thr Glu Cys Ser
   210
<210> 13
   <211> 214
   <212> PRT
   <213> Artificial Sequence
<220>
   <223> lambda light chain (LC) <Ang-2> with E123K and E124K substitutions
<400> 13
   Gln Pro Gly Leu Thr Gln Pro Pro Ser Val Ser Val Ala Pro Gly Gln
   1 5 10 15
   Thr Ala Arg Ile Thr Cys Gly Gly Asn Asn Ile Gly Ser Lys Ser Val
   20 25 30
   His Trp Tyr Gln Gln Lys Pro Gly Gln Ala Pro Val Leu Val Val Tyr
   35 40 45
   Asp Asp Ser Asp Arg Pro Ser Gly Ile Pro Glu Arg Phe Ser Gly Ser
   50 55 60
   Asn Ser Gly Asn Thr Ala Thr Leu Thr Ile Ser Arg Val Glu Ala Gly
   65 70 75 80
   Asp Glu Ala Asp Tyr Tyr Cys Gln Val Trp Asp Ser Ser Ser Asp His
   85 90 95
   Tyr Val Phe Gly Thr Gly Thr Lys Val Thr Val Leu Gly Gln Pro Lys
   100 105 110
   Ala Ala Pro Ser Val Thr Leu Phe Pro Pro Ser Ser Lys Lys Leu Gln
   115 120 125
   Ala Asn Lys Ala Thr Leu Val Cys Leu Ile Ser Asp Phe Tyr Pro Gly
   130 135 140
   Ala Val Thr Val Ala Trp Lys Ala Asp Ser Ser Pro Val Lys Ala Gly
   145 150 155 160
   Val Glu Thr Thr Thr Pro Ser Lys Gln Ser Asn Asn Lys Tyr Ala Ala
   165 170 175
   Ser Ser Tyr Leu Ser Leu Thr Pro Glu Gln Trp Lys Ser His Arg Ser
   180 185 190
   Tyr Ser Cys Gln Val Thr His Glu Gly Ser Thr Val Glu Lys Thr Val
   195 200 205
   Ala Pro Thr Glu Cys Ser
   210
<210> 14
   <211> 440
   <212> PRT
   <213> Artificial Sequence
<220>
   <223> heavy chain (HC) <TWEAK> with VL-VH/CL-CH1 domain exchange wild type (wt)
<400> 14
   Asp Ile Gln Met Thr Gln Ser Pro Ser Ser Val Ser Ala Ser Val Gly
   1 5 10 15
   Asp Arg Val Thr Ile Thr Cys Gln Ala Ser Gln Asn Ile Tyr Ser Asn
   20 25 30
   Leu Ala Trp Tyr Gln Gln Lys Pro Gly Lys Ala Pro Lys Leu Leu Ile
   35 40 45
   Tyr Thr Ala Ser Tyr Leu Ala Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Thr Ala Tyr Tyr Asn Ser Arg
                   85                  90                  95
   Pro Asp Thr Val Ala Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys Pro Pro Cys Pro Pro Cys
       210                 215                 220
   Pro Ala Pro Glu Phe Glu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser Gln Glu Asp Pro Glu Val Gln Phe Asn Trp
               260                 265                 270
   Tyr Val Asp Gly Val Glu Val His Asn Ala Lys Thr Lys Pro Arg Glu
           275                 280                 285
   Glu Gln Phe Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305 310 315 320
   Lys Gly Leu Pro Ser Ser Ile Glu Lys Thr Ile Ser Lys Ala Lys Gly
   325 330 335
   Gln Pro Arg Glu Pro Gln Val Tyr Thr Leu Pro Pro Cys Gln Glu Glu
   340 345 350
   Met Thr Lys Asn Gln Val Ser Leu Trp Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Arg Leu Thr Val Asp Lys Ser Arg Trp Gln Glu Gly Asn
   405 410 415
   Val Phe Ser Cys Ser Val Met His Glu Ala Leu His Asn His Tyr Thr
   420 425 430
   Gln Lys Ser Leu Ser Leu Ser Leu
   435 440
<210> 15
   <211> 223
   <212> PRT
   <213> Artificial Sequence
<220>
   <223> light chain (LC) <TWEAK> with VL-VH/CL-CH1 domain exchange wild type (wt)
<400> 15
   Gln Val Gln Leu Val Gln Ser Gly Ala Glu Val Lys Lys Pro Gly Ser
   1 5 10 15
   Ser Val Lys Val Ser Cys Lys Ala Ser Gly Phe Asp Phe Ser Thr Tyr
   20 25 30
   Tyr Met Ser Trp Val Arg Gln Ala Pro Gly Gln Gly Leu Glu Trp Met
   35 40 45
   Gly Thr Val Tyr Val Arg Gln Gly Thr Thr Tyr Tyr Ala Ser Trp Leu
   50 55 60
   Asn Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Tyr Asn Tyr Asp Asp Ala Phe Val Ile Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Cys Ser Arg Ser Thr Ser Glu Ser Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Lys Thr Tyr Thr Cys Asn Val Asp His Lys
           195                 200                 205
   Pro Ser Asn Thr Lys Val Asp Lys Arg Val Glu Ser Lys Tyr Gly
       210                 215                 220
<210> 16
   <211> 446
   <212> PRT
   <213> Artificial Sequence
<220>
   <223> heavy chain (HC) <IL17> wild type (wt)
<400> 16
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Asp Ser Tyr
               20                  25                  30
   Gly Val His Trp Val Arg Gln Ala Thr Gly Lys Gly Leu Glu Trp Val
           35                  40                  45
   Ser Val Ile Trp Ser Asp Gly Thr Thr Thr Tyr Asn Ser Ala Leu Lys
       50                  55                  60
   Ser Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Asp Thr His Tyr Arg Leu Tyr Tyr Tyr Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Cys Ser Arg Ser Thr Ser Glu Ser Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Lys Thr Tyr Thr Cys Asn Val Asp His
           195                 200                 205
   Lys Pro Ser Asn Thr Lys Val Asp Lys Arg Val Glu Ser Lys Tyr Gly
       210                 215                 220
   Pro Pro Cys Pro Pro Cys Pro Ala Pro Glu Phe Glu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser Gln Glu Asp Pro
               260                 265                 270
   Glu Val Gln Phe Asn Trp Tyr Val Asp Gly Val Glu Val His Asn Ala
           275                 280                 285
   Lys Thr Lys Pro Arg Glu Glu Gln Phe Asn Ser Thr Tyr Arg Val Val
       290                 295                 300
   Ser Val Leu Thr Val Leu His Gln Asp Trp Leu Asn Gly Lys Glu Tyr
   305 310 315 320
   Lys Cys Lys Val Ser Asn Lys Gly Leu Pro Ser Ser Ile Glu Lys Thr
   325 330 335
   Ile Ser Lys Ala Lys Gly Gln Pro Arg Glu Pro Gln Val Cys Thr Leu
   340 345 350
   Pro Pro Ser Gln Glu Glu Met Thr Lys Asn Gln Val Ser Leu Ser Cys
   355 360 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu
   435 440 445
<210> 17
   <211> 219
   <212> PRT
   <213> Artificial Sequence
<220>
   <223> kappa light chain (LC) <IL17> wild type (wt)
<400> 17
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Val His Ser
   20 25 30
   Asn Gly Asp Thr Tyr Phe His Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Thr
                   85                  90                  95
   Thr His Ala Pro Phe Thr Phe Gly Gln Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 18
   <211> 446
   <212> PRT
   <213> Artificial Sequence
<220>
   <223> heavy chain (HC) <IL17> with K213E and K147E substitutions
<400> 18
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Asp Ser Tyr
               20                  25                  30
   Gly Val His Trp Val Arg Gln Ala Thr Gly Lys Gly Leu Glu Trp Val
           35                  40                  45
   Ser Val Ile Trp Ser Asp Gly Thr Thr Thr Tyr Asn Ser Ala Leu Lys
   50 55 60
   Ser Arg Phe Thr Ile Ser Arg Glu Asn Ala Lys Asn Ser Leu Tyr Leu
   65 70 75 80
   Gln Met Asn Ser Leu Arg Ala Gly Asp Thr Ala Val Tyr Tyr Cys Ala
   85 90 95
   Arg Asp Thr His Tyr Arg Leu Tyr Tyr Tyr Ala Met Asp Tyr Trp Gly
   100 105 110
   Gln Gly Thr Thr Val Thr Val Ser Ser Ala Ser Thr Lys Gly Pro Ser
   115 120 125
   Val Phe Pro Leu Ala Pro Cys Ser Arg Ser Thr Ser Glu Ser Thr Ala
   130 135 140
   Ala Leu Gly Cys Leu Val Glu Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Lys Thr Tyr Thr Cys Asn Val Asp His
   195 200 205
   Lys Pro Ser Asn Thr Lys Val Asp Glu Arg Val Glu Ser Lys Tyr Gly
   210 215 220
   Pro Pro Cys Pro Pro Cys Pro Ala Pro Glu Phe Glu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser Gln Glu Asp Pro
   260 265 270
   Glu Val Gln Phe Asn Trp Tyr Val Asp Gly Val Glu Val His Asn Ala
   275 280 285
   Lys Thr Lys Pro Arg Glu Glu Gln Phe Asn Ser Thr Tyr Arg Val Val
   290 295 300
   Ser Val Leu Thr Val Leu His Gln Asp Trp Leu Asn Gly Lys Glu Tyr
   305                 310                 315                 320
   Lys Cys Lys Val Ser Asn Lys Gly Leu Pro Ser Ser Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Gln Glu Glu Met Thr Lys Asn Gln Val Ser Leu Ser Cys
           355                 360                 365
   Ala Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Val Ser Arg Leu Thr Val Asp Lys Ser
                   405                 410                 415
   Arg Trp Gln Glu Gly Asn Val Phe Ser Cys Ser Val Met His Glu Ala
               420                 425                 430
   Leu His Asn His Tyr Thr Gln Lys Ser Leu Ser Leu Ser Leu
           435                 440                 445
<210> 19
   <211> 219
   <212> PRT
   <213> Artificial Sequence
<220>
   <223> kappa light chain (LC) <IL17> E123K and Q124K substitutions
<400> 19
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Val His Ser
               20                  25                  30
   Asn Gly Asp Thr Tyr Phe His Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Thr
                   85                  90                  95
   Thr His Ala Pro Phe Thr Phe Gly Gln Gly Thr Lys Leu Glu Ile Lys
               100                 105                 110
   Arg Thr Val Ala Ala Pro Ser Val Phe Ile Phe Pro Pro Ser Asp Lys
           115                 120                 125
   Lys Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 20
   <211> 440
   <212> PRT
   <213> Artificial Sequence
<220>
   <223> heavy chain (HC) <TWEAK> with VL-VH/CL-CH1 domain exchange with Q124E substitution
<400> 20
   Asp Ile Gln Met Thr Gln Ser Pro Ser Ser Val Ser Ala Ser Val Gly
   1               5                   10                  15
   Asp Arg Val Thr Ile Thr Cys Gln Ala Ser Gln Asn Ile Tyr Ser Asn
               20                  25                  30
   Leu Ala Trp Tyr Gln Gln Lys Pro Gly Lys Ala Pro Lys Leu Leu Ile
           35                  40                  45
   Tyr Thr Ala Ser Tyr Leu Ala Ser Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Gln Thr Ala Tyr Tyr Asn Ser Arg
   85 90 95
   Pro Asp Thr Val Ala Phe Gly Gly Gly Thr Lys Val Glu Ile Lys Arg
   100 105 110
   Thr Val Ala Ala Pro Ser Val Phe Ile Phe Pro Pro Ser Asp Glu Glu
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys Pro Pro Cys Pro Pro Cys
   210 215 220
   Pro Ala Pro Glu Phe Glu Gly Gly Pro Ser Val Phe Leu Phe Pro Pro
   225 230 235 240
   Lys Pro Lys Asp Thr Leu Met Ile Ser Arg Thr Pro Glu Val Thr Cys
   245 250 255
   Val Val Val Asp Val Ser Gln Glu Asp Pro Glu Val Gln Phe Asn Trp
   260 265 270
   Tyr Val Asp Gly Val Glu Val His Asn Ala Lys Thr Lys Pro Arg Glu
   275 280 285
   Glu Gln Phe Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Gly Leu Pro Ser Ser Ile Glu Lys Thr Ile Ser Lys Ala Lys Gly
   325 330 335
   Gln Pro Arg Glu Pro Gln Val Tyr Thr Leu Pro Pro Cys Gln Glu Glu
   340 345 350
   Met Thr Lys Asn Gln Val Ser Leu Trp Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Arg Leu Thr Val Asp Lys Ser Arg Trp Gln Glu Gly Asn
   405 410 415
   Val Phe Ser Cys Ser Val Met His Glu Ala Leu His Asn His Tyr Thr
   420 425 430
   Gln Lys Ser Leu Ser Leu Ser Leu
   435 440
<210> 21
   <211> 439
   <212> PRT
   <213> Artificial Sequence
<220>
   <223> heavy chain (HC) <cMet> with VL-VH/CL-CH1 domain exchange wild type (wt)
<400> 21
   Asp Val Val Met Thr Gln Ser Gln Lys Phe Met Ser Thr Ser Val Gly
   1 5 10 15
   Asp Arg Val Ser Ile Ser Cys Lys Ala Ser Gln Asn Val Gly Asn Ile
   20 25 30
   Ile Ala Trp Phe Leu Gln Lys Pro Gly Gln Ser Pro Gln Ala Leu Ile
   35 40 45
   Tyr Leu Ala Ser Tyr Arg Tyr Ser Gly Val Pro Asp Arg Phe Thr Gly
   50 55 60
   Ser Gly Ser Gly Thr Asp Phe Thr Leu Thr Ile Ser Asn Val Gln Ser
   65 70 75 80
   Glu Asp Leu Ala Glu Tyr Phe Cys Gln Gln Tyr Gly Ser Ser Pro Val
   85 90 95
   Thr Phe Gly Thr Gly Thr Lys Leu Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Asp Lys Thr His Thr Cys Pro Pro Cys Pro
   210 215 220
   Ala Pro Glu Leu Leu Gly Gly Pro Ser Val Phe Leu Phe Pro Pro Lys
   225 230 235 240
   Pro Lys Asp Thr Leu Met Ile Ser Arg Thr Pro Glu Val Thr Cys Val
   245 250 255
   Val Val Asp Val Ser His Glu Asp Pro Glu Val Lys Phe Asn Trp Tyr
   260 265 270
   Val Asp Gly Val Glu Val His Asn Ala Lys Thr Lys Pro Arg Glu Glu
   275 280 285
   Gln Tyr Asn Ser Thr Tyr Arg Val Val Ser Val Leu Thr Val Leu His
   290 295 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Cys Thr Leu Pro Pro Ser Arg Asp Glu Leu
   340 345 350
   Thr Lys Asn Gln Val Ser Leu Ser Cys Ala Val Lys Gly Phe Tyr Pro
   355 360 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Val Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro
   435
<210> 22
   <211> 224
   <212> PRT
   <213> Artificial Sequence
<220>
   <223> light chain (LC) <cMet> with VL-VH/CL-CH1 domain exchange wild type (wt)
<400> 22
   Gln Ile Gln Leu Val Gln Ser Gly Pro Glu Leu Lys Lys Pro Gly Glu
   1 5 10 15
   Thr Val Lys Ile Ser Cys Lys Ala Ser Gly Tyr Thr Phe Thr Asp Tyr
   20 25 30
   Ser Met His Trp Val Lys Gln Ala Pro Gly Lys Gly Leu Lys Trp Met
   35 40 45
   Gly Trp Ile Asn Thr Lys Thr Gly Glu Pro Thr Tyr Ala Asp Asp Phe
   50 55 60
   Lys Gly Arg Phe Ala Phe Ser Leu Glu Thr Ser Ala Ser Thr Ala Tyr
   65 70 75 80
   Leu Gln Ile Asn Asn Leu Lys Asn Glu Asp Thr Ala Thr Tyr Phe Cys
                   85                  90                  95
   Ala Arg Glu Glu Gly Gly Asn Tyr Tyr Tyr Gly Met Asp Tyr Trp Gly
               100                 105                 110
   Gln Gly Thr Ser Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
<210> 23
   <211> 450
   <212> PRT
   <213> Artificial Sequence
<220>
   <223> heavy chain (HC) <Her1Her3> wild type (wt)
<400> 23
   Ser Glu Val Gln Leu Val Glu Ser Gly Gly Gly Leu Val Gln Pro Gly
   1               5                   10                  15
   Gly Ser Leu Arg Leu Ser Cys Ala Ala Ser Gly Phe Thr Leu Ser Gly
               20                  25                  30
   Asp Trp Ile His Trp Val Arg Gln Ala Pro Gly Lys Gly Leu Glu Trp
           35                  40                  45
   Val Gly Glu Ile Ser Ala Ala Gly Gly Tyr Thr Asp Tyr Ala Asp Ser
       50                  55                  60
   Val Lys Gly Arg Phe Thr Ile Ser Ala Asp Thr Ser Lys Asn Thr Ala
   65                  70                  75                  80
   Tyr Leu Gln Met Asn Ser Leu Arg Ala Glu Asp Thr Ala Val Tyr Tyr
                   85                  90                  95
   Cys Ala Arg Glu Ser Arg Val Ser Phe Glu Ala Ala Met Asp Tyr Trp
               100                 105                 110
   Gly Gln Gly Thr Leu Val Thr Val Ser Ser Ala Ser Thr Lys Gly Pro
           115                 120                 125
   Ser Val Phe Pro Leu Ala Pro Ser Ser Lys Ser Thr Ser Gly Gly Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Gln Thr Tyr Ile Cys Asn Val Asn
           195                 200                 205
   His Lys Pro Ser Asn Thr Lys Val Asp Lys Lys Val Glu Pro Lys Ser
       210                 215                 220
   Cys Asp Lys Thr His Thr Cys Pro Pro Cys Pro Ala Pro Glu Leu Leu
   225                 230                 235                 240
   Gly Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Lys Phe Asn Trp Tyr Val Asp Gly Val Glu
           275                 280                 285
   Val His Asn Ala Lys Thr Lys Pro Arg Glu Glu Gln Tyr Asn Ser Thr
       290                 295                 300
   Tyr Arg Val Val Ser Val Leu Thr Val Leu His Gln Asp Trp Leu Asn
   305                 310                 315                 320
   Gly Lys Glu Tyr Lys Cys Lys Val Ser Asn Lys Ala Leu Pro Ala Pro
   325 330 335
   Ile Glu Lys Thr Ile Ser Lys Ala Lys Gly Gln Pro Arg Glu Pro Gln
   340 345 350
   Val Tyr Thr Leu Pro Pro Cys Arg Asp Glu Leu Thr Lys Asn Gln Val
   355 360 365
   Ser Leu Trp Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Val Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro
   450
<210> 24
   <211> 214
   <212> PRT
   <213> Artificial Sequence
<220>
   <223> kappa light chain (LC) <Her1Her3> wild type (wt)
<400> 24
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asn Ile Ala Thr Asp
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Glu Pro Glu Pro Tyr
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 25
   <211> 439
   <212> PRT
   <213> Artificial Sequence
<220>
   <223> heavy chain (HC) <cMet> with VL-VH/CL-CH1 domain exchange with Q124E substitution
<400> 25
   Asp Val Val Met Thr Gln Ser Gln Lys Phe Met Ser Thr Ser Val Gly
   1 5 10 15
   Asp Arg Val Ser Ile Ser Cys Lys Ala Ser Gln Asn Val Gly Asn Ile
   20 25 30
   Ile Ala Trp Phe Leu Gln Lys Pro Gly Gln Ser Pro Gln Ala Leu Ile
   35 40 45
   Tyr Leu Ala Ser Tyr Arg Tyr Ser Gly Val Pro Asp Arg Phe Thr Gly
       50                  55                  60
   Ser Gly Ser Gly Thr Asp Phe Thr Leu Thr Ile Ser Asn Val Gln Ser
   65                  70                  75                  80
   Glu Asp Leu Ala Glu Tyr Phe Cys Gln Gln Tyr Gly Ser Ser Pro Val
                   85                  90                  95
   Thr Phe Gly Thr Gly Thr Lys Leu Glu Ile Lys Arg Thr Val Ala Ala
               100                 105                 110
   Pro Ser Val Phe Ile Phe Pro Pro Ser Asp Glu Glu Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Asn Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305 310 315 320
   Ala Leu Pro Ala Pro Ile Glu Lys Thr Ile Ser Lys Ala Lys Gly Gln
   325 330 335
   Pro Arg Glu Pro Gln Val Cys Thr Leu Pro Pro Ser Arg Asp Glu Leu
   340 345 350
   Thr Lys Asn Gln Val Ser Leu Ser Cys Ala Val Lys Gly Phe Tyr Pro
   355 360 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Val Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro
   435
<210> 26
   <211> 449
   <212> PRT
   <213> Artificial Sequence
<220>
   <223> heavy chain (HC) <Her1Her3> with K213E and K147E substitutions
<400> 26
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Leu Ser Gly Asp
   20 25 30
   Trp Ile His Trp Val Arg Gln Ala Pro Gly Lys Gly Leu Glu Trp Val
   35 40 45
   Gly Glu Ile Ser Ala Ala Gly Gly Tyr Thr Asp Tyr Ala Asp Ser Val
   50 55 60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Glu Ser Arg Val Ser Phe Glu Ala Ala Met Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Glu Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Glu Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Cys Arg Asp Glu Leu Thr Lys Asn Gln Val Ser
   355 360 365
   Leu Trp Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro
<210> 27
   <211> 214
   <212> PRT
   <213> Artificial Sequence
<220>
   <223> kappa light chain (LC) <Her1Her3> E123K and Q124K substitutions
<400> 27
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asn Ile Ala Thr Asp
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Glu Pro Glu Pro Tyr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Lys Lys Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 28
   <211> 449
   <212> PRT
   <213> Artificial Sequence
<220>
   <223> heavy chain (HC) <Her1Her3> with K213D and K147E substitutions
<400> 28
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Leu Ser Gly Asp
               20                  25                  30
   Trp Ile His Trp Val Arg Gln Ala Pro Gly Lys Gly Leu Glu Trp Val
           35                  40                  45
   Gly Glu Ile Ser Ala Ala Gly Gly Tyr Thr Asp Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Glu Ser Arg Val Ser Phe Glu Ala Ala Met Asp Tyr Trp Gly
   100 105 110
   Gln Gly Thr Leu Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Glu Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Asp Lys Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Cys Arg Asp Glu Leu Thr Lys Asn Gln Val Ser
   355 360 365
   Leu Trp Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro
<210> 29
   <211> 214
   <212> PRT
   <213> Artificial Sequence
<220>
   <223> kappa light chain (LC) <Her1Her3> E123R and Q124K substitutions
<400> 29
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asn Ile Ala Thr Asp
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Glu Pro Glu Pro Tyr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Arg Lys Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 30
   <211> 123
   <212> PRT
   <213> Artificial Sequence
<220>
   <223> variable heavy chain domain <VEGF> 289
<400> 30
<210> 31
   <211> 107
   <212> PRT
   <213> Artificial Sequence
<220>
   <223> variable light chain domain <VEGF> 289
<400> 31
<210> 32
   <211> 129
   <212> PRT
   <213> Artificial Sequence
<220>
   <223> variable heavy chain domain <Ang-2> 289
<400> 32
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Ser Pro Asn Pro Tyr Tyr Tyr Asp Ser Ser Gly Tyr Tyr Tyr
               100                 105                 110
   Pro Gly Ala Phe Asp Ile Trp Gly Gln Gly Thr Met Val Thr Val Ser
           115                 120                 125
   Ser
<210> 33
   <211> 108
   <212> PRT
   <213> Artificial Sequence
<220>
   <223> variable light chain domain <Ang-2> 289
<400> 33
   Gln Pro Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
   50 55 60
   Asn Ser Gly Asn Thr Ala Thr Leu Thr Ile Ser Arg Val Glu Ala Gly
   65 70 75 80
   Asp Glu Ala Asp Tyr Tyr Cys Gln Val Trp Asp Ser Ser Ser Asp His
   85 90 95
   Tyr Val Phe Gly Thr Gly Thr Lys Val Thr Val Leu
   100 105
<210> 34
   <211> 120
   <212> PRT
   <213> Artificial Sequence
<220>
   <223> variable heavy chain domain <TWEAK> 305-LC2
<400> 34
   Gln Val Gln Leu Val Gln Ser Gly Ala Glu Val Lys Lys Pro Gly Ser
   1 5 10 15
   Ser Val Lys Val Ser Cys Lys Ala Ser Gly Phe Asp Phe Ser Thr Tyr
   20 25 30
   Tyr Met Ser Trp Val Arg Gln Ala Pro Gly Gln Gly Leu Glu Trp Met
   35 40 45
   Gly Thr Val Tyr Val Arg Gln Gly Thr Thr Tyr Tyr Ala Ser Trp Leu
   50 55 60
   Asn Gly Arg Val Thr Ile Thr Ala Asp Glu Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Gly Gly Tyr Asn Tyr Asp Asp Ala Phe Val Ile Trp Gly Gln
   100 105 110
   Gly Thr Leu Val Thr Val Ser Ser
   115 120
<210> 35
   <211> 111
   <212> PRT
   <213> Artificial Sequence
<220>
   <223> variable light chain domain <TWEAK> 305-LC2
<400> 35
   Asp Ile Gln Met Thr Gln Ser Pro Ser Ser Val Ser Ala Ser Val Gly
   1               5                   10                  15
   Asp Arg Val Thr Ile Thr Cys Gln Ala Ser Gln Asn Ile Tyr Ser Asn
               20                  25                  30
   Leu Ala Trp Tyr Gln Gln Lys Pro Gly Lys Ala Pro Lys Leu Leu Ile
           35                  40                  45
   Tyr Thr Ala Ser Tyr Leu Ala Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Thr Ala Tyr Tyr Asn Ser Arg
                   85                  90                  95
   Pro Asp Thr Val Ala Phe Gly Gly Gly Thr Lys Val Glu Ile Lys
               100                 105                 110
<210> 36
   <211> 121
   <212> PRT
   <213> Artificial Sequence
<220>
   <223> variable heavy chain domain <IL17> LC136
<400> 36
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Asp Ser Tyr
               20                  25                  30
   Gly Val His Trp Val Arg Gln Ala Thr Gly Lys Gly Leu Glu Trp Val
           35                  40                  45
   Ser Val Ile Trp Ser Asp Gly Thr Thr Thr Tyr Asn Ser Ala Leu Lys
       50                  55                  60
   Ser Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Asp Thr His Tyr Arg Leu Tyr Tyr Tyr Ala Met Asp Tyr Trp Gly
               100                 105                 110
   Gln Gly Thr Thr Val Thr Val Ser Ser
           115                 120
<210> 37
   <211> 112
   <212> PRT
   <213> Artificial Sequence
<220>
   <223> variable light chain domain <IL17> LC136
<400> 37
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Val His Ser
   20 25 30
   Asn Gly Asp Thr Tyr Phe His Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ser Gln Thr
   85 90 95
   Thr His Ala Pro Phe Thr Phe Gly Gln Gly Thr Lys Leu Glu Ile Lys
   100 105 110
<210> 38
   <211> 121
   <212> PRT
   <213> Artificial Sequence
<220>
   <223> variable heavy chain domain <cMet>
<400> 38
   Gln Ile Gln Leu Val Gln Ser Gly Pro Glu Leu Lys Lys Pro Gly Glu
   1 5 10 15
   Thr Val Lys Ile Ser Cys Lys Ala Ser Gly Tyr Thr Phe Thr Asp Tyr
   20 25 30
   Ser Met His Trp Val Lys Gln Ala Pro Gly Lys Gly Leu Lys Trp Met
   35 40 45
   Gly Trp Ile Asn Thr Lys Thr Gly Glu Pro Thr Tyr Ala Asp Asp Phe
   50 55 60
   Lys Gly Arg Phe Ala Phe Ser Leu Glu Thr Ser Ala Ser Thr Ala Tyr
   65 70 75 80
   Leu Gln Ile Asn Asn Leu Lys Asn Glu Asp Thr Ala Thr Tyr Phe Cys
   85 90 95
   Ala Arg Glu Glu Gly Gly Asn Tyr Tyr Tyr Gly Met Asp Tyr Trp Gly
   100 105 110
   Gln Gly Thr Ser Val Thr Val Ser Ser
   115 120
<210> 39
   <211> 107
   <212> PRT
   <213> Artificial Sequence
<220>
   <223> variable light chain domain <cMet>
<400> 39
   Asp Val Val Met Thr Gln Ser Gln Lys Phe Met Ser Thr Ser Val Gly
   1               5                   10                  15
   Asp Arg Val Ser Ile Ser Cys Lys Ala Ser Gln Asn Val Gly Asn Ile
               20                  25                  30
   Ile Ala Trp Phe Leu Gln Lys Pro Gly Gln Ser Pro Gln Ala Leu Ile
           35                  40                  45
   Tyr Leu Ala Ser Tyr Arg Tyr Ser Gly Val Pro Asp Arg Phe Thr Gly
       50                  55                  60
   Ser Gly Ser Gly Thr Asp Phe Thr Leu Thr Ile Ser Asn Val Gln Ser
   65                  70                  75                  80
   Glu Asp Leu Ala Glu Tyr Phe Cys Gln Gln Tyr Gly Ser Ser Pro Val
                   85                  90                  95
   Thr Phe Gly Thr Gly Thr Lys Leu Glu Ile Lys
               100                 105
<210> 40
   <211> 121
   <212> PRT
   <213> Artificial Sequence
<220>
   <223> variable heavy chain domain <Her1Her3>
<400> 40
<210> 41
   <211> 107
   <212> PRT
   <213> Artificial Sequence
<220>
   <223> variable light chain domain <Her1Her3>
<400> 41
<210> 42
   <211> 440
   <212> PRT
   <213> Artificial Sequence
<220>
   <223> heavy chain (HC) <TWEAK> with VL-VH/CL-CH1 domain exchange wild type (wt) with Q38E and Q124E substitution
<400> 42
   Asp Ile Gln Met Thr Gln Ser Pro Ser Ser Val Ser Ala Ser Val Gly
   1               5                   10                  15
   Asp Arg Val Thr Ile Thr Cys Gln Ala Ser Gln Asn Ile Tyr Ser Asn
               20                  25                  30
   Leu Ala Trp Tyr Gln Glu Lys Pro Gly Lys Ala Pro Lys Leu Leu Ile
           35                  40                  45
   Tyr Thr Ala Ser Tyr Leu Ala Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Thr Ala Tyr Tyr Asn Ser Arg
                   85                  90                  95
   Pro Asp Thr Val Ala Phe Gly Gly Gly Thr Lys Val Glu Ile Lys Arg
               100                 105                 110
   Thr Val Ala Ala Pro Ser Val Phe Ile Phe Pro Pro Ser Asp Glu Glu
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys Pro Pro Cys Pro Pro Cys
   210 215 220
   Pro Ala Pro Glu Phe Glu Gly Gly Pro Ser Val Phe Leu Phe Pro Pro
   225 230 235 240
   Lys Pro Lys Asp Thr Leu Met Ile Ser Arg Thr Pro Glu Val Thr Cys
   245 250 255
   Val Val Val Asp Val Ser Gln Glu Asp Pro Glu Val Gln Phe Asn Trp
   260 265 270
   Tyr Val Asp Gly Val Glu Val His Asn Ala Lys Thr Lys Pro Arg Glu
   275 280 285
   Glu Gln Phe Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Gly Leu Pro Ser Ser Ile Glu Lys Thr Ile Ser Lys Ala Lys Gly
   325 330 335
   Gln Pro Arg Glu Pro Gln Val Tyr Thr Leu Pro Pro Cys Gln Glu Glu
   340 345 350
   Met Thr Lys Asn Gln Val Ser Leu Trp Cys Leu Val Lys Gly Phe Tyr
   355 360 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Arg Leu Thr Val Asp Lys Ser Arg Trp Gln Glu Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Leu
           435                 440
<210> 43
   <211> 223
   <212> PRT
   <213> Artificial Sequence
<220>
   <223> light chain (LC) <TWEAK> with VL-VH/CL-CH1 domain exchange wild type (wt) with Q39K substitution
<400> 43
   Gln Val Gln Leu Val Gln Ser Gly Ala Glu Val Lys Lys Pro Gly Ser
   1               5                   10                  15
   Ser Val Lys Val Ser Cys Lys Ala Ser Gly Phe Asp Phe Ser Thr Tyr
               20                  25                  30
   Tyr Met Ser Trp Val Arg Lys Ala Pro Gly Gln Gly Leu Glu Trp Met
           35                  40                  45
   Gly Thr Val Tyr Val Arg Gln Gly Thr Thr Tyr Tyr Ala Ser Trp Leu
       50                  55                  60
   Asn Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Tyr Asn Tyr Asp Asp Ala Phe Val Ile Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Cys Ser Arg Ser Thr Ser Glu Ser Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Lys Thr Tyr Thr Cys Asn Val Asp His Lys
           195                 200                 205
   Pro Ser Asn Thr Lys Val Asp Lys Arg Val Glu Ser Lys Tyr Gly
       210                 215                 220
<210> 44
   <211> 446
   <212> PRT
   <213> Artificial Sequence
<220>
   <223> heavy chain (HC) <IL17> wild type (wt) with Q39E, K147E and K213E substitution
<400> 44
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Asp Ser Tyr
               20                  25                  30
   Gly Val His Trp Val Arg Glu Ala Thr Gly Lys Gly Leu Glu Trp Val
           35                  40                  45
   Ser Val Ile Trp Ser Asp Gly Thr Thr Thr Tyr Asn Ser Ala Leu Lys
       50                  55                  60
   Ser Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Asp Thr His Tyr Arg Leu Tyr Tyr Tyr Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Cys Ser Arg Ser Thr Ser Glu Ser Thr Ala
       130                 135                 140
   Ala Leu Gly Cys Leu Val Glu Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Lys Thr Tyr Thr Cys Asn Val Asp His
           195                 200                 205
   Lys Pro Ser Asn Thr Lys Val Asp Glu Arg Val Glu Ser Lys Tyr Gly
       210                 215                 220
   Pro Pro Cys Pro Pro Cys Pro Ala Pro Glu Phe Glu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser Gln Glu Asp Pro
               260                 265                 270
   Glu Val Gln Phe Asn Trp Tyr Val Asp Gly Val Glu Val His Asn Ala
           275                 280                 285
   Lys Thr Lys Pro Arg Glu Glu Gln Phe Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Gly Leu Pro Ser Ser Ile Glu Lys Thr
                   325                 330                 335
   Ile Ser Lys Ala Lys Gly Gln Pro Arg Glu Pro Gln Val Cys Thr Leu
               340                 345                 350
   Pro Pro Ser Gln Glu Glu Met Thr Lys Asn Gln Val Ser Leu Ser Cys
           355                 360                 365
   Ala Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Val Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu
   435 440 445
<210> 45
   <211> 219
   <212> PRT
   <213> Artificial Sequence
<220>
   <223> kappa light chain (LC) <IL17> wild type (wt) with Q38K, E123K and Q124K substitution
<400> 45
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Val His Ser
   20 25 30
   Asn Gly Asp Thr Tyr Phe His Trp Tyr Leu Lys Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ser Gln Thr
   85 90 95
   Thr His Ala Pro Phe Thr Phe Gly Gln Gly Thr Lys Leu Glu Ile Lys
   100 105 110
   Arg Thr Val Ala Ala Pro Ser Val Phe Ile Phe Pro Pro Ser Asp Lys
   115 120 125
   Lys Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 46
   <211> 439
   <212> PRT
   <213> Artificial Sequence
<220>
   <223> heavy chain (HC) <VEGF> with VL-VH/CL-CH1 domain exchange with Q124E substitution with wild type CH3
<400> 46
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Glu Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Asn Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Tyr Thr Leu Pro Pro Ser Arg Asp Glu Leu
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Tyr Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro
   435
<210> 47
   <211> 457
   <212> PRT
   <213> Artificial Sequence
<220>
   <223> heavy chain (HC) <Ang-2> with K231E, K147E substitution with wild type CH3
<400> 47
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Ser Pro Asn Pro Tyr Tyr Tyr Asp Ser Ser Gly Tyr Tyr Tyr
   100 105 110
   Pro Gly Ala Phe Asp Ile Trp Gly Gln Gly Thr Met Val Thr Val Ser
   115 120 125
   Ser Ala Ser Thr Lys Gly Pro Ser Val Phe Pro Leu Ala Pro Ser Ser
       130                 135                 140
   Lys Ser Thr Ser Gly Gly Thr Ala Ala Leu Gly Cys Leu Val Glu Asp
   145                 150                 155                 160
   Tyr Phe Pro Glu Pro Val Thr Val Ser Trp Asn Ser Gly Ala Leu Thr
                   165                 170                 175
   Ser Gly Val His Thr Phe Pro Ala Val Leu Gln Ser Ser Gly Leu Tyr
               180                 185                 190
   Ser Leu Ser Ser Val Val Thr Val Pro Ser Ser Ser Leu Gly Thr Gln
           195                 200                 205
   Thr Tyr Ile Cys Asn Val Asn His Lys Pro Ser Asn Thr Lys Val Asp
       210                 215                 220
   Glu Lys Val Glu Pro Lys Ser Cys Asp Lys Thr His Thr Cys Pro Pro
   225                 230                 235                 240
   Cys Pro Ala Pro Glu Leu Leu Gly Gly Pro Ser Val Phe Leu Phe Pro
                   245                 250                 255
   Pro Lys Pro Lys Asp Thr Leu Met Ile Ser Arg Thr Pro Glu Val Thr
               260                 265                 270
   Cys Val Val Val Asp Val Ser His Glu Asp Pro Glu Val Lys Phe Asn
           275                 280                 285
   Trp Tyr Val Asp Gly Val Glu Val His Asn Ala Lys Thr Lys Pro Arg
       290                 295                 300
   Glu Glu Gln Tyr Asn Ser Thr Tyr Arg Val Val Ser Val Leu Thr Val
   305                 310                 315                 320
   Leu His Gln Asp Trp Leu Asn Gly Lys Glu Tyr Lys Cys Lys Val Ser
                   325                 330                 335
   Asn Lys Ala Leu Pro Ala Pro Ile Glu Lys Thr Ile Ser Lys Ala Lys
               340                 345                 350
   Gly Gln Pro Arg Glu Pro Gln Val Tyr Thr Leu Pro Pro Ser Arg Asp
           355                 360                 365
   Glu Leu Thr Lys Asn Gln Val Ser Leu Thr Cys Leu Val Lys Gly Phe
               370                 375                 380
   Tyr Pro Ser Asp Ile Ala Val Glu Trp Glu Ser Asn Gly Gln Pro Glu
   385                 390                 395                 400
   Asn Asn Tyr Lys Thr Thr Pro Pro Val Leu Asp Ser Asp Gly Ser Phe
                   405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro
       450                 455
<210> 48
   <211> 439
   <212> PRT
   <213> Artificial Sequence
<220>
   <223> heavy chain (HC) <VEGF> with VL-VH/CL-CH1 domain exchange with wild type CH3
<400> 48
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
               115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Asn Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Tyr Thr Leu Pro Pro Ser Arg Asp Glu Leu
               340                 345                 350
   Thr Lys Asn Gln Val Ser Leu Thr Cys Leu Val Lys Gly Phe Tyr Pro
           355                 360                 365
   Ser Asp Ile Ala Val Glu Trp Glu Ser Asn Gly Gln Pro Glu Asn Asn
   370 375 380
   Tyr Lys Thr Thr Pro Pro Val Leu Asp Ser Asp Gly Ser Phe Phe Leu
   385 390 395 400
   Tyr Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro
   435
<210> 49
   <211> 439
   <212> PRT
   <213> Artificial Sequence
<220>
   <223> heavy chain (HC) <VEGF> with VL-VH/CL-CH1 domain exchange with E123K, Q124K substitution
<400> 49
   Asp Ile Gln Met Thr Gln Ser Pro Ser Ser Leu Ser Ala Ser Val Gly
   1 5 10 15
   Asp Arg Val Thr Ile Thr Cys Ser Ala Ser Gln Asp Ile Ser Asn Tyr
   20 25 30
   Leu Asn Trp Tyr Gln Gln Lys Pro Gly Lys Ala Pro Lys Val Leu Ile
   35 40 45
   Tyr Phe Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Thr Val Pro Trp
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Lys Lys Leu Lys Ser Gly
   115 120 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys Asp Lys Thr His Thr Cys Pro Pro Cys Pro
       210                 215                 220
   Ala Pro Glu Leu Leu Gly Gly Pro Ser Val Phe Leu Phe Pro Pro Lys
   225                 230                 235                 240
   Pro Lys Asp Thr Leu Met Ile Ser Arg Thr Pro Glu Val Thr Cys Val
                   245                 250                 255
   Val Val Asp Val Ser His Glu Asp Pro Glu Val Lys Phe Asn Trp Tyr
               260                 265                 270
   Val Asp Gly Val Glu Val His Asn Ala Lys Thr Lys Pro Arg Glu Glu
           275                 280                 285
   Gln Tyr Asn Ser Thr Tyr Arg Val Val Ser Val Leu Thr Val Leu His
       290                 295                 300
   Gln Asp Trp Leu Asn Gly Lys Glu Tyr Lys Cys Lys Val Ser Asn Lys
   305                 310                 315                 320
   Ala Leu Pro Ala Pro Ile Glu Lys Thr Ile Ser Lys Ala Lys Gly Gln
                   325                 330                 335
   Pro Arg Glu Pro Gln Val Cys Thr Leu Pro Pro Ser Arg Asp Glu Leu
               340                 345                 350
   Thr Lys Asn Gln Val Ser Leu Ser Cys Ala Val Lys Gly Phe Tyr Pro
           355                 360                 365
   Ser Asp Ile Ala Val Glu Trp Glu Ser Asn Gly Gln Pro Glu Asn Asn
       370                 375                 380
   Tyr Lys Thr Thr Pro Pro Val Leu Asp Ser Asp Gly Ser Phe Phe Leu
   385 390 395 400
   Val Ser Lys Leu Thr Val Asp Lys Ser Arg Trp Gln Gln Gly Asn Val
   405 410 415
   Phe Ser Cys Ser Val Met His Glu Ala Leu His Asn His Tyr Thr Gln
   420 425 430
   Lys Ser Leu Ser Leu Ser Pro
   435
<210> 50
   <211> 226
   <212> PRT
   <213> Artificial Sequence
<220>
   <223> light chain (LC) <VEGF> with VL-VH/CL-CH1 domain exchange with K213E, K147E substitution
<400> 50
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Thr Phe Thr Asn Tyr
   20 25 30
   Gly Met Asn Trp Val Arg Gln Ala Pro Gly Lys Gly Leu Glu Trp Val
   35 40 45
   Gly Trp Ile Asn Thr Tyr Thr Gly Glu Pro Thr Tyr Ala Ala Asp Phe
   50 55 60
   Lys Arg Arg Phe Thr Phe Ser Leu Asp Thr Ser Lys Ser Thr Ala Tyr
   65 70 75 80
   Leu Gln Met Asn Ser Leu Arg Ala Glu Asp Thr Ala Val Tyr Tyr Cys
   85 90 95
   Ala Lys Tyr Pro His Tyr Tyr Gly Ser Ser His Trp Tyr Phe Asp Val
   100 105 110
   Trp Gly Gln Gly Thr Leu Val Thr Val Ser Ser Ala Ser Thr Lys Gly
   115 120 125
   Pro Ser Val Phe Pro Leu Ala Pro Ser Ser Lys Ser Thr Ser Gly Gly
   130 135 140
   Thr Ala Ala Leu Gly Cys Leu Val Glu Asp Tyr Phe Pro Glu Pro Val
   145 150 155 160
   Thr Val Ser Trp Asn Ser Gly Ala Leu Thr Ser Gly Val His Thr Phe
   165 170 175
   Pro Ala Val Leu Gln Ser Ser Gly Leu Tyr Ser Leu Ser Ser Val Val
   180 185 190
   Thr Val Pro Ser Ser Ser Leu Gly Thr Gln Thr Tyr Ile Cys Asn Val
   195 200 205
   Asn His Lys Pro Ser Asn Thr Lys Val Asp Glu Lys Val Glu Pro Lys
   210 215 220
   Ser Cys
   225
<210> 51
   <211> 215
   <212> PRT
   <213> Artificial Sequence
<220>
   <223> kappa light chain (LC) <Ang-2> with Q124E substitution
<400> 51
   Gln Pro Gly Leu Thr Gln Pro Pro Ser Val Ser Val Ala Pro Gly Gln
   1 5 10 15
   Thr Ala Arg Ile Thr Cys Gly Gly Asn Asn Ile Gly Ser Lys Ser Val
   20 25 30
   His Trp Tyr Gln Gln Lys Pro Gly Gln Ala Pro Val Leu Val Val Tyr
   35 40 45
   Asp Asp Ser Asp Arg Pro Ser Gly Ile Pro Glu Arg Phe Ser Gly Ser
   50 55 60
   Asn Ser Gly Asn Thr Ala Thr Leu Thr Ile Ser Arg Val Glu Ala Gly
   65 70 75 80
   Asp Glu Ala Asp Tyr Tyr Cys Gln Val Trp Asp Ser Ser Ser Asp His
   85 90 95
   Tyr Val Phe Gly Thr Gly Thr Lys Val Thr Val Leu Arg Thr Val Ala
   100 105 110
   Ala Pro Ser Val Phe Ile Phe Pro Pro Ser Asp Glu Glu Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215

## Description

### FIELD OF THE INVENTION

The present invention relates to novel multispecific antibodies, methods for their production, pharmaceutical compositions containing said antibodies and uses thereof.

### BACKGROUND OF THE INVENTION

Engineered proteins, such as bi- or multispecific antibodies capable of binding two or more antigens are known in the art. Such multispecific binding proteins can be generated using cell fusion, chemical conjugation, or recombinant DNA techniques.

A wide variety of recombinant multispecific antibody formats have been developed in the recent past, e.g. tetravalent bispecific antibodies by fusion of, e.g. an IgG antibody format and single chain domains (see e.g. Coloma, M.J., et. al., Nature Biotech. 15 (1997) 159-163; WO 2001/077342; and Morrison, S.L., Nature Biotech. 25 (2007) 1233-1234).

Also several other new formats, wherein the antibody core structure (IgA, IgD, IgE, IgG or IgM) is no longer retained, have been developed; such as dia-, tria- or tetrabodies, minibodies and several single chain formats (scFv, Bis-scFv), which are capable of binding two or more antigens (Holliger, P., et. al, Nature Biotech. 23 (2005) 1126-1136; Fischer, N., and Léger, O., Pathobiology 74 (2007) 3-14; Shen, J., et. al., J. Immunol. Methods 318 (2007) 65-74; Wu, C., et al., Nature Biotech. 25 (2007) 1290-1297).

All such formats use linkers either to fuse the antibody core (IgA, IgD, IgE, IgG or IgM) to a further binding protein (e.g. scFv) or to fuse e.g. two Fab fragments or scFv (Fischer, N., and Léger, O., Pathobiology 74 (2007) 3-14). While it is obvious that linkers have advantages for the engineering of bispecific antibodies, they may also cause problems in therapeutic settings. Indeed, these foreign peptides might elicit an immune response against the linker itself or the junction between the protein and the linker. Furthermore, the flexible nature of these peptides makes them more prone to proteolytic cleavage, potentially leading to poor antibody stability, aggregation and increased immunogenicity. In addition one may want to retain effector functions, such as e.g. complement-dependent cytotoxicity (CDC) or antibody dependent cellular cytotoxicity (ADCC), which are mediated through the Fc-part by maintaining a high degree of similarity to naturally occurring antibodies.

Thus, ideally, one should aim at developing bispecific antibodies that are very similar in general structure to naturally occurring antibodies (like IgA, IgD, IgE, IgG or IgM) with minimal deviation from human sequences.

In one approach bispecific antibodies that are very similar to natural antibodies have been produced using the quadroma technology (see Milstein, C., and Cuello, A.C., Nature 305 (1983) 537-540) based on the somatic fusion of two different hybridoma cell lines expressing murine monoclonal antibodies with the desired specificities of the bispecific antibody. Because of the random pairing of two different antibody heavy and light chains within the resulting hybrid-hybridoma (or quadroma) cell line, up to ten different antibody species are generated of which only one is the desired, functional bispecific antibody. Due to the presence of mispaired byproducts, and significantly reduced production yields, sophisticated purification procedures are required (see e.g. Morrison, S.L., Nature Biotech. 25 (2007) 1233-1234). In general the same problem of mispaired by-products remains if recombinant expression techniques are used.

An approach to circumvent the problem of mispaired byproducts, which is known as "knob-into-hole technology", aims at forcing the pairing of two different antibody heavy chains by introducing mutations into the CH3 domains to modify the contact interface. On one chain bulky amino acids were replaced by amino acids with short side chains to create a "hole". Conversely, amino acids with large side chains were introduced into the other CH3 domain, to create a "knob". By coexpressing these two heavy chains (and two identical light chains, which have to be appropriate for both heavy chains), high yields of heterodimer formation ("knob-hole") versus homodimer formation ("hole-hole" or "knob-knob") was observed (Ridgway, J.B., et al., Protein Eng. 9 (1996) 617-621; and WO 96/027011). The percentage of heterodimer could be further increased by remodeling the interaction surfaces of the two CH3 domains using a phage display approach and the introduction of a disulfide bridge to stabilize the heterodimers (Merchant, A.M., et al., Nature Biotech. 16 (1998) 677-681; Atwell, S., et al., J. Mol. Biol. 270 (1997) 26-35). New approaches for the knob-into-hole technology are described in e.g. in EP 1 870 459 A1. Although this format appears very attractive, no data describing progression towards the clinic are currently available. One important constraint of this strategy is that the light chains of the two parent antibodies have to be identical to prevent mispairing and formation of inactive molecules. Thus this technique is not appropriate as a basis for easily developing recombinant, tri-or tetraspecific antibodies against three or four antigens starting from two antibodies against the first and the second antigen, as either the heavy chains of these antibodies and/or the identical light chains have to be optimized first and then further antigen binding peptides against the third and fourth antigen have to be added.

WO 2006/093794 relates to heterodimeric protein binding compositions. WO 99/37791 describes multipurpose antibody derivatives. Morrison, S.L., et al., J. Immunol. 160 (1998) 2802-2808 refers to the influence of variable region domain exchange on the functional properties of IgG.

WO 2013/02362 relates to heterodimerized polypeptides. WO 2013/12733 relates to polypeptides comprising heterodimeric Fc regions. WO 2012/131555 relates to engineered hetero-dimeric immunoglobulins. EP 2647707 relates to engineered hetero-dimeric immunoglobulins.

WO 2009/080251, WO 2009/080252, WO 2009/080253, WO 2009/080254 and Schaefer, W. et al, PNAS, 108 (2011) 11187-1191 relate to bivalent, bispecific IgG antibodies with a domain crossover.

The multispecific antibodies with a VL-VH/CL-CH1 replacement in one binding arm (CrossMabFab), which are described in WO 2009/080251 and Schaefer, W. et al, PNAS, 108 (2011) 11187-1191 clearly reduce the byproduct formation caused by a mismatch of a light chain of a first antibody that specifically binds to a first antigen with the wrong heavy chain of a second antibody that specifically binds to a second antigen (when compared to approaches without such domain exchanges). However their preparation is not completely free of side products. The side product profile depends on the structure of the multispecific antibody with a VL-VH/CL-CH1 replacement in one binding arm. The main side products are non functional light chain dimers (see also Schaefer, W. et al, PNAS, 108 (2011) 11187-1191; in Fig. S1G of the Supplement) and non functional heavy chain dimers (see also Schaefer, W. et al, PNAS, 108 (2011) 11187-1191; in Fig. S1F of the Supplement).

Therefore there is still a need for approaches to further reduce unwanted side products in order to improve e.g. the yield of multispecific antibodies.

### SUMMARY OF THE INVENTION

The present invention relates to a multispecific antibody, comprising a first light chain and a first heavy chain derived from a first antibody which specifically binds to a first antigen; and a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
- wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D; or
- wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.
   One embodiment of the invention relates to a multispecific antibody,
- wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D, and wherein in the constant domain CL of the second heavy chain the amino acid at position 124 (numbering according to Kabat) is substituted by an amino acid selected from E and D; or
- wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and wherein in the constant domain CL of the first light chain the amino acid at position 124 (numbering according to Kabat) is substituted by an amino acid selected from E and D.

One embodiment of the invention relates to a multispecific antibody, wherein in the variable domain VL of the first light chain the amino acid at position 38 (numbering according to Kabat) is substituted by an amino acid selected from K, R and H; and wherein in the variable domain VH of the first heavy chain the amino acid at position 39 (numbering according to Kabat) is substituted by an amino acid selected from E and D; and wherein in the variable domain VL of the second heavy chain the amino acid at position 38 (numbering according to Kabat) is substituted by an amino acid selected from E and D (in one preferred embodiment E); and wherein in the variable domain VH of the second light chain the amino acid at position 39 (numbering according to Kabat) is substituted by an amino acid selected from K, R and H (in one preferred embodiment by K or R, in one embodiment by K).

Another aspect of the invention is a method for the preparation of a multispecific antibody according to the invention, comprising the steps of
- transforming a host cell with vectors comprising nucleic acids encoding
   a) the first light chain as defined for a multispecific antibody according to the invention derived from a first antibody which specifically binds to a first antigen;
   b) the first heavy chain as defined for a multispecific antibody according to the invention derived from a first antibody which specifically binds to a first antigen;
   c) the second light chain as defined for a multispecific antibody according to the invention derived from a second antibody which specifically binds to a second antigen; and
   d) the second heavy chain as defined for a multispecific antibody according to the invention derived from a second antibody which specifically binds to a second antigen,
- culturing said host cell under conditions that allow synthesis of said multispecific antibody; and
- recovering said multispecific antibody from said host cell culture.

Another aspect of the invention is a nucleic acid encoding the multispecific antibody according to the invention.

Another aspect of the invention is a vector comprising a nucleic acid according to the invention, wherein the vector is capable of expressing said nucleic acid in a host cell.

Another aspect of the invention is a host cell comprising a vector according to the invention.

Another aspect of the invention is a pharmaceutical composition comprising a multispecific antibody according to the invention in combination with at least one pharmaceutically acceptable carrier.

Also described herein is an immunoconjugate comprising the multispecific antibody according to the invention coupled to a cytotoxic agent.

Also described herein is the use of a multispecific antibody according to the invention for the manufacture of a pharmaceutical composition.

Another aspect of the invention is the multispecific antibody according to the invention for use as a medicament.

Also described herein is a pharmaceutical composition comprising a multispecific antibody according to the invention in combination with at least one pharmaceutically acceptable carrier for use as a medicament.

Also described herein is the use of a multispecific antibody according to the invention for the manufacture of a medicament.

Also described herein is a method of treatment of a patient suffering from a disease by administering a multispecific antibody according to the invention to the patient in the need of such treatment.

According to the invention the formation of undesired side products during the production of the multispecific antibodies can be reduced due to the introduction of oppositely charged amino acids at specific positions in the CH1 and CL domains. Thereby, the yield of the desired multispecific antibody can be increased.

### DESCRIPTION OF THE FIGURES

**Figure 1****:** Some examples of multispecific antibodies according to the invention with VL-VH/CL-CH1 domain exchange (CrossMAb^{Fab}) in one antibody binding arm and specific amino acid substitutions in one CH1/CL domain interface:
   **Figure 1a****:** VL-VH/CL-CH1 domain exchange in one antibody binding arm and specific mutations in the CH1/CL domain interface of the other ("uncrossed") antibody binding arm.
   **Figure 1b****:** VL-VH/CL-CH1 domain exchange in one antibody binding arm and specific mutations in the CH1/CL domain interface of the same ("crossed") antibody binding arm.
   **Figure 1c****:** VL-VH/CL-CH1 domain exchange in one antibody binding arm with specific mutation in the same ("crossed") antibody binding arm and additional specific mutations in the CH1/CL domain interface of the other ("uncrossed") antibody binding arm.
   **Figure 1d****:** VL-VH/CL-CH1 domain exchange in one antibody binding arm and specific mutations in the CH1/CL domain interface of the same ("crossed") antibody binding arm with additional specific mutation in the CH1/CL domain interface of the other ("uncrossed") antibody binding arm.
   **Figure 1e****:** VL-VH/CL-CH1 domain exchange in one antibody binding arm and specific mutations in the CH1/CL domain interface of the other ("uncrossed") antibody binding arm and additional specific mutations in the VH/VL interface of both antibody binding arms.
   **Figure 1f****:** VL-VH/CL-CH1 domain exchange in one antibody binding arm and specific mutations in the CH1/CL domain interface of the same ("crossed") antibody binding arm and additional specific mutations in the VH/VL interface of both antibody binding arms.
   **Figure 1g****:** VL-VH/CL-CH1 domain exchange in one antibody binding arm with specific mutation in the same ("crossed") antibody binding arm and additional specific mutations in the CH1/CL domain interface of the other ("uncrossed") antibody binding arm and further specific mutations in the VH/VL interface of both antibody binding arms.
   **Figure 1h****:** VL-VH/CL-CH1 domain exchange in one antibody binding arm with specific mutations in the CH1/CL domain interface of the same ("crossed") antibody binding arm with additional specific mutation in the CH1/CL domain interface of the other ("uncrossed") antibody binding arm and further specific mutations in the VH/VL interface of both antibody binding arms.
   **Figure 1i**:VL-VH/CL-CH1 domain exchange in one antibody binding arm and specific mutations in the CH1/CL domain interface of the other ("uncrossed") antibody binding arm, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1j****:** VL-VH/CL-CH1 domain exchange in one antibody binding arm and specific mutations in the CH1/CL domain interface of the same ("crossed") antibody binding arm, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1k****:** VL-VH/CL-CH1 domain exchange in one antibody binding arm and specific mutations in the CH1/CL domain interface of the both antibody binding arms (double mutation on the "uncrossed" binding arm), and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1l****:** VL-VH/CL-CH1 domain exchange in one antibody binding arm and specific mutations in the CH1/CL domain interface of the both antibody binding arms (double mutation on the "crossed" binding arm), and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1m****:** VL-VH/CL-CH1 domain exchange in one antibody binding arm and specific mutations in the CH1/CL domain interface of the other ("uncrossed") antibody binding arm and additional specific mutations in the VH/VL interface of both antibody binding arms, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1n****:** VL-VH/CL-CH1 domain exchange in one antibody binding arm and specific mutations in the CH1/CL domain interface of the same ("crossed") antibody binding arm and additional specific mutations in the VH/VL interface of both antibody binding arms, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1o****:** VL-VH/CL-CH1 domain exchange in one antibody binding arm and specific mutations in the CH1/CL domain interface of the both antibody binding arms (double mutation on the "uncrossed" binding arm) and additional specific mutations in the VH/VL interface of both antibody binding arms, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1p****:** VL-VH/CL-CH1 domain exchange in one antibody binding arm and specific mutations in the CH1/CL domain interface of the both antibody binding arms (double mutation on the "crossed" binding arm) and additional specific mutations in the VH/VL interface of both antibody binding arms, and modifications of the CH3/CH3 domain interface to enforce heavy chain heterodimerization (like e.g. knob-into-hole technology or alternative heterodimerization technologies like e.g. substitution of charged amino acids with their respective opposite charge).

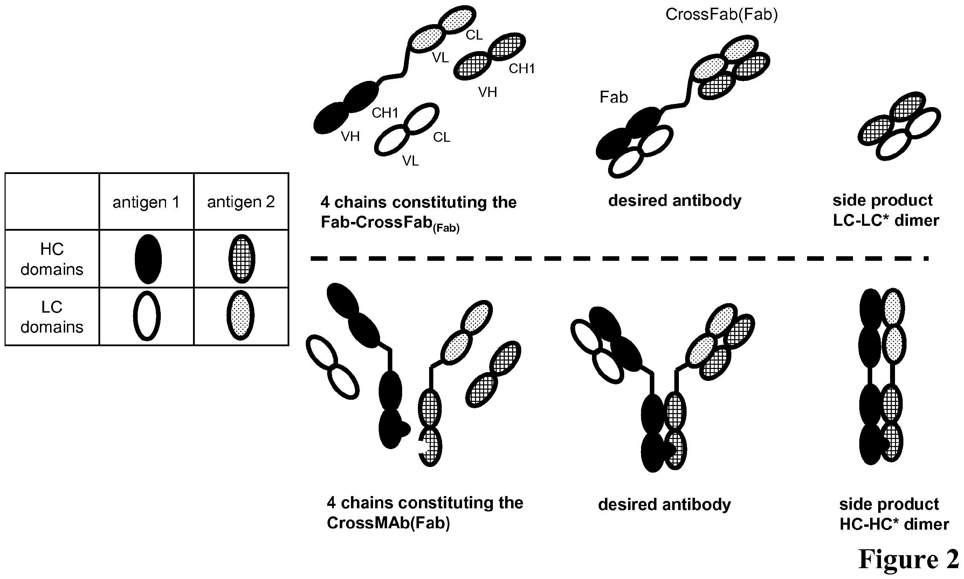

**Figure 2****:** Example of multispecific antibody with VL-VH/CL-CH1 domain exchange in one antibody binding arm and **without** specific amino acid substitutions in one CH1/CL domain interface and the side products of this multispecific antibody. Upper field: Multispecific antibody with VL-VH/CL-CH1 domain exchange in one antibody binding arm comprising only Fab fragments of the first and second antibody (Fab-CrossFab_{(Fab)}) and main side product, a dimer formed between the light chain (LC: VL-CL) of the first antibody and the modified light chain (LC*: VH-CH1) of the second antibody. Lower field: Multispecific antibody with VL-VH/CL-CH1 domain exchange in one antibody binding arm additionally comprising Fc fragments (CrossMAbFab) and main side product, a non functional heavy chain dimer formed between the heavy chain (HC: VH-CH1-CH2-CH3) of the first antibody and the modified heavy chain (HC*: VL-CL-CH2-CH3) of the second antibody.

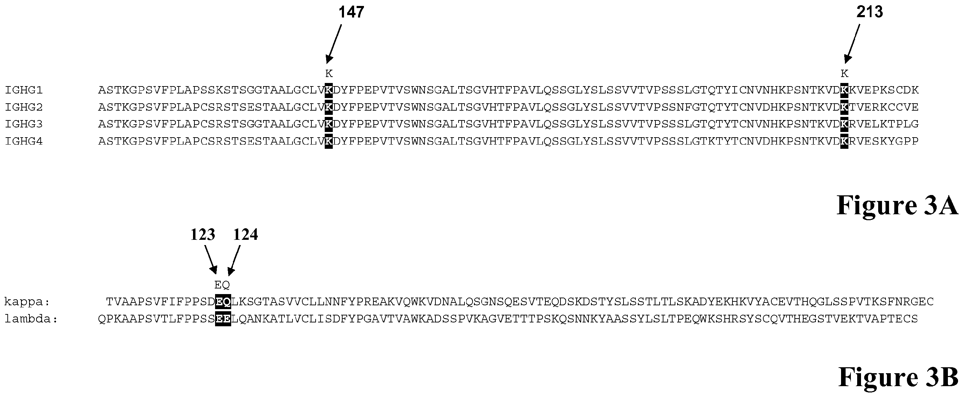

**Figure 3****:** Positions for amino acid substitutions in CH1 and CL domains
   **Figure 3A****:** wild type (wt) amino acid sequences in CH1 domain (four IgG isotypes are shown) with highlighted amino acid positions 147 and 213 (according to Kabat numbering).
   **Figure 3B****:** wild type (wt) amino acid sequences in the CL domain of kappa and lambda isotype with underlined and highlighted amino acid positions 123 and 124 (according to Kabat EU index numbering).
**Figure 4****:** Reduction of side product by charged amino acids substitutions in anti-Ang2-VEGF bispecific antibodies.
   **Figure 4A****:** Sequences (SEQ ID NOs) of the bispecific antibodies for which the results are shown in Figure 4B.
   **Figure 4B****:** Reduction of side product (non functional heavy hcain dimer) by charged amino acids substitutions according to the invention in the CH1/CL interface using examples of anti-Ang2-VEGF bispecific antibodies according to the invention with VL-VH/CL-CH1 domain exchange (CrossMAbFab).
**Figure 5****:** Reduction of side product by charged amino acids substitutions in anti-Tweak-IL17 bispecific antibodies.
   **Figure 5A****:** Sequences (SEQ ID NOs) of the bispecific antibodies for which the results are shown in Figure 5B.
   **Figure 5B****:.** Reduction of side product (non functional heavy hcain dimer) by single charged amino acids substitutions according to the invention in the CH1/CL interface using examples of anti-Tweak-IL17 bispecific antibodies according to the invention with VL-VH/CL-CH1 domain exchange (CrossMAbFab).
**Figure 6****:** Reduction of side product by charged amino acids substitutions in anti-Her1Her3-cMet trispecific antibodies.
   **Figure 6A****:** Sequences (SEQ ID NOs) of the bispecific antibodies for which the results are shown in Figure 6B.
   **Figure 6B****:.** Reduction of side product by single charged amino acids substitutions according to the invention in the CH1/CL interface using examples of anti-Her1Her3-cMet trispecific antibodies according to the invention with VL-VH/CL-CH1 domain exchange (CrossDAF-Fab).
**Figure 7****:** Reduction of side product by charged amino acids substitutions in anti-Ang2-VEGF bispecific antibodies.
   **Figure 7A****:** Sequences (SEQ ID NO) of the bispecific antibody "Ang2VEGF 044" for which the result is shown in Figure 7B.
   **Figure 7B****:** Reduction of side product by charged amino acids substitutions according to the invention in the CH1/CL interface (amino acid sequences according to figure 4A) and additional charged amino acid substitutions in the VL/VH interface using examples of anti-Ang2-VEGF bispecific antibodies according to the invention with VL-VH/CL-CH1 domain exchange (CrossMAbFab).

### DETAILED DESCRIPTION OF THE INVENTION

### I) DEFINITIONS

The terms "a", "an" and "the" generally include plural referents, unless the context clearly indicates otherwise.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

"Multispecific antibodies" bind two or more different epitopes (for example, two, three, four, or more different epitopes). The epitopes may be on the same or different antigens. An example of a multispecific antibody is a "bispecific antibody" which binds two different epitopes.

When an antibody possesses more than one specificity, the recognized epitopes may be associated with a single antigen or with more than one antigen.

The term "valent" as used herein denotes the presence of a specified number of binding sites in an antibody molecule. A natural antibody for example has two binding sites and is bivalent. As such, the term "trivalent" denotes the presence of three binding sites in an antibody molecule.

"Antibody specificity" refers to selective recognition of a particular epitope of an antigen by the antibody. Natural antibodies, for example, are monospecific. The term "monospecific antibody" as used herein denotes an antibody that has one or more binding sites each of which bind to the same epitope of the same antigen.

An epitope is a region of an antigen that is bound by an antibody. The term "epitope" includes any polypeptide determinant capable of specific binding to an antibody. In certain embodiments, epitope determinants include chemically active surface groupings of molecules such as amino acids, glycan side chains, phosphoryl, or sulfonyl, and, in certain embodiments, may have specific three dimensional structural characteristics, and/or specific charge characteristics. In certain embodiments, an antibody is said to specifically bind an antigen when it preferentially recognizes its target antigen in a complex mixture of proteins and/or macromolecules.

As used herein, the terms "binding" and "specific binding" refer to the binding of the antibody to an epitope of the antigen in an *in vitro* assay, preferably in a plasmon resonance assay (BIAcore, GE-Healthcare Uppsala, Sweden) with purified wild-type antigen.

The affinity of the binding of an antibody to an antigen is defined by the terms kₐ (rate constant for the association of the antibody from the antibody/antigen complex), k_{D} (dissociation constant), and K_{D} (k_{D}/ka). In one embodiment binding or that/which specifically binds to means a binding affinity (K_{D}) of 10⁻⁸ mol/l or less, in one embodiment 10⁻⁸ M to 10⁻¹³ mol/l. Thus, an multispecific antibody according to the invention specifically binds to each antigen for which it is specific with a binding affinity (K_{D}) of 10⁻⁸ mol/l or less, e.g. with a binding affinity (K_{D}) of 10⁻⁸ to 10⁻¹³ mol/l. in one embodiment with a binding affinity (K_{D}) of 10⁻⁹ to 10⁻¹³ mol/l.

Binding of the antibody to the FcyRIII can be investigated by a BIAcore assay (GE-Healthcare Uppsala, Sweden). The affinity of the binding is defined by the terms ka (rate constant for the association of the antibody from the antibody/antigen complex), k_{D} (dissociation constant), and K_{D} (k_{D}/ka).

The terms "monoclonal antibody" or "monoclonal antibody composition" as used herein refer to a preparation of antibody molecules of a single amino acid composition.

The term "chimeric" antibody refers to an antibody in which a portion of the heavy and/or light chain is derived from a particular source or species, while the remainder of the heavy and/or light chain is derived from a different source or species.

The term "humanized antibody" refers to antibodies in which the framework or the CDRs have been modified to comprise the CDR of an immunoglobulin of different specificity as compared to that of the parent immunoglobulin. In one preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Other forms of humanized antibodies encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 33-40). Human antibodies can also be produced in phage display libraries (Hoogenboom, H.R., and Winter, G., J. Mol. Biol. 227 (1992) 381-388; Marks, J.D., et al., J. Mol. Biol. 222 (1991) 581-597). The techniques of Cole et al. and Boerner et al. are also available for the preparation of human monoclonal antibodies (Cole, et al., Monoclonal Antibodies and Cancer Therapy, Alan R. Liss, p. 77 (1985); and Boerner, P., et al., J. Immunol. 147 (1991) 86-95). As already mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation).

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The terms "binding site" or "antigen-binding site" as used herein denotes the region(s) of an antibody molecule to which a ligand (e.g. the antigen or antigen fragment of it) actually binds and which is derived from an antibody. The antigen-binding site includes antibody heavy chain variable domains (VH) and/or antibody light chain variable domains (VL), or pairs of VH/VL.

The antigen-binding sites that specifically bind to the desired antigen can be derived a) from known antibodies specifically binding to the antigen or b) from new antibodies or antibody fragments obtained by de novo immunization methods using inter alia either the antigen protein or nucleic acid or fragments thereof or by phage display.

An antigen-binding site of an antibody according to the invention can contain six complementarity determining regions (CDRs) which contribute in varying degrees to the affinity of the binding site for antigen. There are three heavy chain variable domain CDRs (CDRH1, CDRH2 and CDRH3) and three light chain variable domain CDRs (CDRL1, CDRL2 and CDRL3). The extent of CDR and framework regions (FRs) is determined by comparison to a compiled database of amino acid sequences in which those regions have been defined according to variability among the sequences. Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

The "light chains" of antibodies (immunoglobulins) from any vertebrate species can be assigned to one of two distinct types, called kappa (κ) and lambda (λ), based on the amino acid sequences of their constant domains. A wild type light chain typically contains two immunoglobulin domains, usually one variable domain (VL) that is important for binding to an antigen and a constant domain (CL).

Several different types of "heavy chains" exist that define the class or isotype of an antibody. A wild type heavy chain contains a series of immunoglobulin domains, usually with one variable domain (VH) that is important for binding antigen and several constant domains (CHI, CH2, CH3, etc.).

The term "Fc domain" is used herein to define a C-terminal region of an immunoglobulin heavy chain that contains at least a portion of the constant region. For example in natural antibodies, the Fc domain is composed of two identical protein fragments, derived from the second and third constant domains of the antibody's two heavy chains in IgG, IgA and IgD isotypes; IgM and IgE Fc domains contain three heavy chain constant domains (CH domains 2-4) in each polypeptide chain. "Devoid of the Fc domain" as used herein means that the bispecific antibodies of the invention do not comprise a CH2, CH3 and CH4 domain; i.e. the constant heavy chain consists solely of one or more CH1 domains.

The "variable domains" or "variable region" as used herein denotes each of the pair of light and heavy chains which is involved directly in binding the antibody to the antigen. The variable domain of a light chain is abbreviated as "VL" and the variable domain of a light chain is abbreviated as "VH". The variable domains of human light chains and heavy chains have the same general structure. Each variable domain comprises four framework (FR) regions, the sequences of which are widely conserved. The FR are connected by three "hypervariable regions" (or "complementarity determining regions", CDRs). CDRs on each chain are separated by such framework amino acids. Therefore, the light and heavy chains of an antibody comprise from N- to C-terminal direction the domains FR1, CDR1, FR2, CDR2, FR3, CDR3, and FR4. The FR adopt a β-sheet conformation and the CDRs may form loops connecting the β-sheet structure. The CDRs in each chain are held in their three-dimensional structure by the FR and form together with the CDRs from the other chain an "antigen binding site". Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, MD (1991).

The term "constant domains" or "constant region" as used within the current application denotes the sum of the domains of an antibody other than the variable region. The constant region is not directly involved in binding of an antigen, but exhibits various effector functions.

Depending on the amino acid sequence of the constant region of their heavy chains, antibodies are divided in the classes: IgA, IgD, IgE, IgG and IgM, and several of these may are further divided into subclasses, such as IgG1, IgG2, IgG3, and IgG4, IgA1 and IgA2. The heavy chain constant regions that correspond to the different classes of antibodies are called α, δ, ε, γ and µ, respectively, respectively. The light chain constant regions (CL) which can be found in all five antibody classes are called κ (kappa) and λ (lambda). The "constant domains" as used herein are from human origin, which is from a constant heavy chain region of a human antibody of the subclass IgG1, IgG2, IgG3, or IgG4 and/or a constant light chain kappa or lambda region. Such constant domains and regions are well known in the state of the art and e.g. described by Kabat, et al., Sequences of Proteins of Immunological Interest, 5th ed., Public Health Service, National Institutes of Health, Bethesda, MD (1991).

The term "tertiary structure" as used herein refers to the geometric shape of the antibody according to the invention. The tertiary structure comprises a polypeptide chain backbone comprising the antibody domains, while amino acid side chains interact and bond in a number of ways.

The term "amino acid" as used herein denotes an organic molecule possessing an amino moiety located at α-position to a carboxylic group. Examples of amino acids include: arginine, glycine, ornithine, lysine, histidine, glutamic acid, asparagic acid, isoleucine, leucine, alanine, phenylalanine, tyrosine, tryptophane, methionine, serine, proline. The amino acid employed is optionally in each case the L-form. The term "positively charged" or "negatively charged" amino acid refers to the amino acid side-chain charge at pH 7.4. Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

**Table - Amino acids with specific properties**

| **Amino Acid** | **3-Letter** | **1-Letter** | **Side-chain polarity** | **Side-chain charge (pH 7.4)** |
|---|---|---|---|---|
| Alanine | Ala | A | nonpolar | neutral |
| Arginine | Arg | R | basic polar | positive |
| Asparagine | Asn | N | polar | neutral |
| Aspartic acid | Asp | D | acidic polar | negative |
| Cysteine | Cys | C | nonpolar | neutral |
| Glutamic acid | Glu | E | acidic polar | negative |
| Glutamine | Gln | Q | polar | neutral |
| Glycine | Gly | G | nonpolar | neutral |
| Histidine | His | H | basic polar | positive (10%) |
| | | | | neutral (90%) |
| Isoleucine | Ile | I | nonpolar | neutral |
| Leucine | Leu | L | nonpolar | neutral |
| Lysine | Lys | K | basic polar | positive |
| Methionine | Met | M | nonpolar | neutral |
| Phenylalanine | Phe | F | nonpolar | neutral |
| Proline | Pro | P | nonpolar | neutral |
| Serine | Ser | S | polar | neutral |
| Threonine | Thr | T | polar | neutral |
| Tryptophan | Trp | w | nonpolar | neutral |
| Tyrosine | Tyr | Y | polar | neutral |
| Valine | Val | V | nonpolar | neutral |

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991), which is referred to herein as "numbering according to Kabat et al.". In particular, for variable domains and for the light chain constant domain CL of kappa and lambda isotype, the Kabat numbering system (see pages 647-660) of Kabat, et al., Sequences of Proteins of Immunological Interest, 5th ed., Public Health Service, National Institutes of Health, Bethesda, MD (1991) is used and is herein referred to as "numbering according to Kabat" and, nonwithstanding this, for the constant heavy chain domains (CHI, Hinge, CH2 and CH3) the Kabat EU index numbering system (see pages 661-723) is used and is herein referred to as "numbering according to EU index of Kabat".

Amino acid substitutions (or mutations) within the polypeptide chains of the multispecific antibody are prepared by introducing appropriate nucleotide changes into the antibody DNA, or by nucleotide synthesis. Such modifications can be performed, however, only in a very limited range, e.g. as described above. For example, the modifications do not alter the above mentioned antibody characteristics such as the IgG isotype and antigen binding, but may further improve the yield of the recombinant production, protein stability or facilitate the purification. In certain embodiments, antibody variants having one or more conservative amino acid substitutions are provided.

The antibody according to the invention is produced by recombinant means. Methods for recombinant production of antibodies are widely known in the state of the art and comprise protein expression in prokaryotic and eukaryotic cells with subsequent isolation of the antibody and usually purification to a pharmaceutically acceptable purity. For the expression of the antibodies as aforementioned in a host cell, nucleic acids encoding the respective (modified) light and heavy chains are inserted into expression vectors by standard methods. Expression is performed in appropriate prokaryotic or eukaryotic host cells, like CHO cells, NS0 cells, SP2/0 cells, HEK293 cells, COS cells, PER.C6 cells, yeast, or E. coli cells, and the antibody is recovered from the cells (supernatant or cells after lysis). General methods for recombinant production of antibodies are well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

"Polynucleotide" or "nucleic acid" as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR2 ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a vector are capable of directing the expression of nucleic acids to which they are operatively linked. When the expression vector is introduced into an appropriate host cell, it can be transcribed and translated into a polypeptide. When transforming host cells in methods according to the invention, "expression vectors" are used; thereby the term "vector" in connection with transformation of host cells as described herein means "expression vector". An "expression system" usually refers to a suitable host cell comprised of an expression vector that can function to yield a desired expression product.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

The term "transformation" as used herein refers to process of transfer of a vectors/nucleic acid into a host cell. If cells without formidable cell wall barriers are used as host cells, transfection is carried out e.g. by the calcium phosphate precipitation method as described by Graham and Van der Eh, Virology 52 (1978) 546ff. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen, F.N, et al., PNAS 69 (1972) 7110 et seq.

The term "host cell" as used in the current application denotes any kind of cellular system which can be engineered to generate the antibodies according to the current invention.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included. Where distinct designations are intended, it will be clear from the context.

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK 293) is described by Schlaeger, E.-J., and Christensen, K., in Cytotechnology 30 (1999) 71-83 and by Schlaeger, E.-J., J. Immunol. Methods 194 (1996) 191-199.

Antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain (also referred to herein as a cleaved variant heavy chain). This may be the case where the final two C-terminal amino acids of the heavy chain are glycine (G446) and lysine (K447, numbering according to Kabat EU index).

Therefore, amino acid sequences of heavy chains including CH3 domains are denoted herein without C-terminal glycine-lysine dipeptide if not indicated otherwise.

Compositions of the invention, such as the pharmaceutical compositions described herein, comprise a population of antibodies of the invention. The population of antibodies may comprise antibodies having a full-length heavy chain and antibodies having a cleaved variant heavy chain. In one embodiment, the population of antibodies consists of a mixture of antibodies having a full-length heavy chain and antibodies having a cleaved variant heavy chain, wherein at least 50%, at least 60%, at least 70%, at least 80% or at least 90% of the antibodies have a cleaved variant heavy chain.

Purification of antibodies (recovering the antibodies from the host cell culture) is performed in order to eliminate cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987). Different methods are well established and widespread used for protein purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

The term "pharmaceutical composition" refers to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. To administer an antibody according to the invention by certain routes of administration, it may be necessary to coat the antibody with, or co-administer the antibody with, a material to prevent its inactivation. For example, the antibody may be administered to a subject in an appropriate carrier, for example, liposomes, or a diluent. Pharmaceutically acceptable diluents include saline and aqueous buffer solutions.

A "pharmaceutically acceptable carrier" refers to an ingredient in a pharmaceutical formulation, other than an active ingredient, which is nontoxic to a subject. Pharmaceutically acceptable carriers includes any and all solvents, dispersion media, coatings, antibacterial and antifungal agents, isotonic and absorption delaying agents, and the like that are physiologically compatible. In one preferred embodiment, the carrier is suitable for intravenous, intramuscular, subcutaneous, parenteral, spinal or epidermal administration (e.g. by injection or infusion).

The pharmaceutical compositions according to the invention may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, in one embodiment the carrier is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

An "immunoconjugate" is an antibody conjugated to one or more heterologous molecule(s), including but not limited to a cytotoxic agent.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "cancer" as used herein refers to proliferative diseases, such as lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

"Human VEGF" as used herein refers to human vascular endothelial growth factor (VEGF/VEGF-A) which is described in e.g. Leung, D.W., et al., Science 246 (1989) 1306-9; Keck, P.J., et al., Science 246 (1989) 1309-12 and Connolly, D.T., et al., J. Biol. Chem. 264 (1989) 20017-24. VEGF is involved in the regulation of normal and abnormal angiogenesis and neovascularization associated with tumors and intraocular disorders (Ferrara, N., et al., Endocr. Rev. 18 (1997) 4-25; Berkman, R.A., et al., J. Clin. Invest. 91 (1993) 153-159; Brown, L.F., et al., Human Pathol. 26 (1995) 86-91; Brown, L.F., et al., Cancer Res. 53 (1993) 4727-4735; Mattern, J., et al., Brit. J. Cancer. 73 (1996) 931-934; and Dvorak, H., et al., Am. J. Pathol. 146 (1995) 1029-1039). VEGF is a homodimeric glycoprotein that has been isolated from several sources. VEGF shows highly specific mitogenic activity for endothelial cells.

Human "ANG-2" as used herein refers to human angiopoietin-2 (ANG-2) (alternatively abbreviated with ANGPT2 or ANG2) which is described in Maisonpierre, P.C., et al, Science 277 (1997) 55-60 and Cheung, A.H., et al., Genomics 48 (1998) 389-91. The angiopoietins-1 and -2 were discovered as ligands for the Ties, a family of tyrosine kinases that is selectively expressed within the vascular endothelium (Yancopoulos, G.D., et al., Nature 407 (2000) 242-48). There are now four definitive members of the angiopoietin family. Angiopoietin-3 and -4 (Ang-3 and Ang-4) may represent widely diverged counterparts of the same gene locus in mouse and man (Kim, I., et al., FEBS Let, 443 (1999) 353-56; Kim, I., et al., J Biol Chem 274 (1999) 26523-28).

Human TWEAK (UniProtKB O43508, TNF-related weak inducer of apoptosis) is a cell surface associated type II transmembrane protein. TWEAK is described in Chicheportiche, Y., et al., J. Biol. Chem. 272 (1997) 32401-32410; Marsters, S.A., et al., Curr. Biol. 8 (1998) 525-528; Lynch, C.N., et al., J. Biol. Chem. 274 (1999) 8455-8459. The active form of TWEAK is a soluble homotrimer. Human and murine TWEAK show 93 % sequence identity in receptor binding domain. The TWEAK receptor Fn14 (fibroblast growth factor inducible 14 kDa protein) is a 129 aa type I transmembane protein consisting of one single cystein rich domain in ligand binding domain. Signaling of TWEAK occurs via NF-KB pathway activation. TWEAK mRNA is expressed in a variety of tissues and found in most major organs like heart, brain, skeletal muscle, and pancreas, tissues related to the immune system like spleen, lymph nodes, and thymus. Fn14 mRNA has been detected in heart, brain, lung, placenta, vascular EC and smooth muscle cells. TWEAK-null and Fnl4-null knockout mice are viable, healthy and fertile and have more natural killer cells and display an enhanced innate inflammatory response. TWEAK is involved in apoptosis, proliferation, angiogenesis, ischemic penumbra, cerebral edema, multiple sclerosis.

Human IL-17 (also named IL17-A; CTLA-8, Swiss Prot Q16552, IL17) is a pro-inflammatory cytokine produced by a subset of memory T cells (called Th17) that has been implicated e.g. in the pathogenesis of multiple sclerosis. IL-17A plays a role in the induction of other inflammatory cytokines, chemokines and adhesion molecules. Treatment of animals with IL-17A neutralizing antibodies decreases disease incidence and severity in autoimmune encephalomyelitis (Komiyama, Y. et al., J. Immunol. 177 (2006) 566-573). IL-17A is over-expressed in the cerebrospinal fluid of MS patients (Hellings, P.W. et al., Am. J. Resp. Cell Mol. Biol. 28 (2003) 42-50; Matusevicius, D. et al., Multiple Sclerosis 5 (1999) 101-104; WO 2005/051422). In addition, IL-17A neutralizing antibodies reduce severity and incidence of mouse rheumatoid arthritis model of collagen induced arthritis, and high levels of IL-17A can be detected in the synovial fluid of inflamed joints from RA patients (Ziolkowska, M. et al., J. Immunol. 164 (2000) 2832-2838; Kotake, S., et al., J. Clin. Invest. 103 (1999) 1345-1352; Hellings, P.W. et al., Am. J. Resp. Cell Mol. Biol. 28 (2003) 42-50).

### II) DETAILED DESCRIPTION OF THE EMBODIMENTS OF THE INVENTION

Multispecific antibodies with a VL-VH/CL-CH1 replacement in one binding arm (CrossMabFab) are described in detail in WO 2009/080251 and Schaefer, W. et al, PNAS, 108 (2011) 11187-1191. With these antibodies, the byproduct formation caused by a mismatch of a light chain of a first antibody that specifically binds to a first antigen with the wrong heavy chain of a second antibody that specifically binds to a second antigen can be considerably reduced (when compared to approaches without such domain exchanges). However their preparation is not completely free of side products. The side product profile depends on the structure of the multispecific antibody with a VL-VH/CL-CH1 replacement in one binding arm. For multispecific antibodies comprising only Fab fragments (Fab-CrossFab, for example as described in WO 2013/026835) the main side product is a non functional light chain dimer (see also Schaefer, W. et al, PNAS, 108 (2011) 11187-1191; in Fig. S1G of the Supplement). For multispecific antibodies including the respective Fc regions of the at least two different antibodies the main side product is a non functional heavy chain dimer (see also Schaefer, W. et al, PNAS, 108 (2011) 11187-1191; in Fig. S1F of the Supplement).

The invention provides an approach to further reduce the formation of unwanted side products to improve the yield by substituting particular amino acids in the CH1 and CL domains by oppositely charged amino acids, respectively. A further improvement of the yield of the multispecific antibody and a reduction of side product formation can be achieved by additionally substituting particular amino acids in the VH and VL domains of the multispecific antibodies.

Therefore, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid; or
   ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In accordance with the concept of the invention, the antibody according to the invention comprises only one of the modifications as indicated under i) and ii) above and below. Hence, the multispecific antibody according to the invention comprises either
i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid;
   or
ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid,
with the proviso that the multispecific antibody does not comprise both of the modifications mentioned under i) and ii).

In one embodiment, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid.

In one embodiment, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In one embodiment of the invention, the negatively charged amino acid is selected from E and D. In one embodiment of the invention, the negatively charged amino acid is E.

In one embodiment of the invention, the positively charged amino acid is selected from K, R and H. In one embodiment of the invention, the positively charged amino acid is selected from K and R. In one embodiment of the invention, the positively charged amino acid is K.

The invention also relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D; or
   ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.

In one embodiment, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D.

In one embodiment, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.

In one embodiment of the invention, in the constant domain CL the amino acids at position 124 and 123 (numbering according to Kabat) are substituted independently from each other by an amino acid selected from K and R.

In one embodiment of the invention, in the constant domain CL the amino acids at position 124 and 123 (numbering according to Kabat) are substituted by K.

In one embodiment of the invention, in the constant domain CH1 the amino acids at position 147 and 213 (numbering according to EU index of Kabat) are substituted by E.

In one embodiment of the invention, in the constant domain CL the amino acids at position 124 and 123 (numbering according to Kabat) are substituted independently from each other by an amino acid selected from K and R, and in the constant domain CH1 the amino acids at position 147 and 213 (numbering according to EU index of Kabat) are substituted independently from each other by an amino acid selected from E or D.

In one embodiment of the invention, in the constant domain CL the amino acids at position 124 and 123 (numbering according to Kabat) are substituted by K, and in the constant domain CH1 the amino acids at position 147 and 213 (numbering according to EU index of Kabat) are substituted by E.

In one embodiment of the invention, in the constant domain CL of the first light chain the amino acids at position 124 and 123 (numbering according to Kabat) are substituted independently from each other by an amino acid selected from K and R.

In one embodiment of the invention, in the constant domain CL of the first light chain the amino acids at position 124 and 123 (numbering according to Kabat) are substituted by K.

In one embodiment of the invention, in the constant domain CH1 of the first heavy chain the amino acids at position 147 and 213 (numbering according to EU index of Kabat) are substituted by E.

In one embodiment of the invention, in the constant domain CL of the first light chain the amino acids at position 124 and 123 (numbering according to Kabat) are substituted independently from each other by an amino acid selected from K and R, and in the constant domain CH1 of the first heavy chain the amino acids at position 147 and 213 (numbering according to EU index of Kabat) are substituted independently from each other by an amino acid selected from E or D.

In one embodiment of the invention, in the constant domain CL of the first light chain the amino acids at position 124 and 123 (numbering according to Kabat) are substituted by K, and in the constant domain CH1 of the first heavy chain the amino acids at position 147 and 213 (numbering according to EU index of Kabat) are substituted by E.

In one embodiment of the invention, in the constant domain CL of the second heavy chain the amino acids at position 124 and 123 (numbering according to Kabat) are substituted independently from each other by an amino acid selected from K and R.

In one embodiment of the invention, in the constant domain CL of the second heavy chain the amino acids at position 124 and 123 (numbering according to Kabat) are substituted by K.

In one embodiment of the invention, in the constant domain CH1 of the second light chain the amino acids at position 147 and 213 (numbering according to EU index of Kabat) are substituted by E.

In one embodiment of the invention, in the constant domain CL of the second heavy chain the amino acids at position 124 and 123 (numbering according to Kabat) are substituted independently from each other by an amino acid selected from K and R, and in the constant domain CH1 of the second light chain the amino acids at position 147 and 213 (numbering according to EU index of Kabat) are substituted independently from each other by an amino acid selected from E or D.

In one embodiment of the invention, in the constant domain CL of the second heavy chain the amino acids at position 124 and 123 (numbering according to Kabat) are substituted by K, and in the constant domain CH1 of the second light chain the amino acids at position 147 and 213 (numbering according to EU index of Kabat) are substituted by E.

The "light chain of an antibody" as used herein is a polypeptide comprising in N-terminal to C-terminal direction an antibody light chain variable domain (VL), and an antibody light chain constant domain (CL), abbreviated as VL-CL. The "heavy chain of an antibody" as used herein is a polypeptide comprising in N-terminal to C-terminal direction an antibody heavy chain variable domain (VH) and an antibody constant heavy chain domain 1 (CHI). In one preferred embodiment of the invention a heavy chain of an antibody comprises in N-terminal to C-terminal direction an antibody heavy chain variable domain (VH), an antibody constant heavy chain domain 1 (CHI), an antibody heavy chain constant domain 2 (CH2) and an antibody heavy chain constant domain 3 (CH3), abbreviated as VH-CH1-CH2-CH3.

Thus, in a multispecific antibody according to the invention in said first light chain derived from said first antibody the sequential arrangement of the domains of the light chain (CL-VL) remains unaltered. In said first heavy chain derived from said first antibody the sequential arrangement of the domains of the heavy chain (CH1-CL or CH3-CH2-CH1-CL) remains unaltered. Because the Fab region derived from said first antibody does not include a domain crossover, this region is herein also referred to as "non-crossed Fab region" of the multispecific antibody according to the invention.

In one embodiment of the invention, the multispecific antibody comprises a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH and CH1 domains (i.E. VH-CH1 from N-terminal to C-terminal direction), wherein the first light chain and the first heavy chain are derived from a first antibody which specifically binds to a first antigen.

In one embodiment of the invention, the multispecific antibody comprises a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH, CH1, CH2 and CH3 domains (i.E. VH-CH1-CH2-CH3 from N-terminal to C-terminal direction), wherein the first light chain and the first heavy chain are derived from a first antibody which specifically binds to a first antigen.

In contrast, within said second light chain (which is herein also referred to as "modified second light chain" or "LC*") the original variable domain VL is replaced by the variable domain VH of the (original) second heavy chain and the original constant domain CL is replaced by the constant domain CH1 of the second heavy chain. Therefore, the modified second light chain is built up of the CH1 and VH domains of the second heavy chain derived from said second antibody. In consequence, the sequential arrangement of the light chain domains of said modified second light chain is VH-CH1 (in N-terminal to C-terminal direction).

Additionally, within said second heavy chain (which is herein also refered to as "modified second heavy chain" or "HC*") the original variable domain VH is replaced by the variable domain VL of the (original) second light chain and the original constant domain CH1 is replaced by the constant domain CL of the (original) second light chainTherefore, the modified second heavy chain comprises at least a sequential arrangement of the CL and VL domains of the second light chain derived from said second antibody. In consequence, the sequential arrangement of the heavy chain domains of said modified second heavy chains is at least VL-CL (in N-terminal to C-terminal direction).

In summary, within said second heavy chain and said second light chain derived from said second antibody the variable domains VL and VH are replaced by each other, and the constant domains CL and CH1 are replaced by each other. Because the Fab region derived from said second antibody includes a domain crossover, as described above, this region is herein also referred to as "crossed Fab region" of the multispecific antibody according to the invention. Thus, the multispecific antibody according to the invention includes at least one non-crossed Fab region and at least one crossed Fab region.

In one embodiment of the invention the modified heavy chain of a multispecific antibody according to the invention consists of a sequential arrangement (N-terminal to C-terminal direction) of the VL and CL domains of the second light chain (however particular amino acid substitutions in said VL and CL domains as described for the invention are possible). Thus, in this embodiment the multispecific antibody comprises at least two Fab fragments, including a first Fab fragment derived from said first antibody (non-crossed Fab fragment) and a second Fab fragment derived from said second antibody (crossed Fab fragment), wherein the multispecific antibody according to this embodiment does not comprise the respective Fc domains of said first and said second antibody (hence, the multispecific antibody is devoid of an Fc domain). In one embodiment, a multispecific antibody comprises two to five Fab fragments. In one embodiment of a multispecific antibody the Fab fragments are connected with each other via a peptide linker. The term "peptide linker" as used herein denotes a peptide with amino acid sequences, which is preferably of synthetic origin. In one embodiment a peptide linker is used to connect one of the Fab fragments to the C- or N-terminus of the other Fab fragment in order to form a multispecific antibody according to the invention. In one preferred embodiment said peptide linker is a peptide with an amino acid sequence with a length of at least 5 amino acids, in one embodiment with a length of 5 to 100, in a further embodiment of 10 to 50 amino acids. In one embodiment said peptide linker is (GxS)ₙ or (GxS)ₙGₘ with G = glycine, S = serine, and (x = 3, n= 3, 4, 5 or 6, and m= 0, 1, 2 or 3) or (x=4, n=2, 3, 4 or 5 and m= 0, 1, 2 or 3), in one embodiment x=4 and n=2 or 3, in a further embodiment x=4 and n=2. In one embodiment said peptide linker is (G₄S)₂. The peptide linker is used to connect the first and the second Fab fragment. In one embodiment the first Fab fragment is connected to the C- or N-terminus of the second Fab fragment. An exemplary scheme of a multispecific antibody of this embodiment, which is devoid of an Fc domain, is depicted in Figure 2 (upper field). The multispecific antibody according to Figure 2 (upper field) is herein also referred to as "Fab-CrossFab". Multispecific antibodies of this format have been previously described in WO 2013/026835.

In one embodiment of the invention the modified heavy chain of a multispecific antibody according to the invention comprises a sequential arrangement (in N-terminal to C-terminal direction) of the VL, CL, CH2 and CH3 domains derived from said second antibody which specifically binds to said second antigen. Thus, in this embodiment the multispecific antibody comprises the respective Fc regions of said first and said second antibody. An exemplary scheme of a multispecific antibody of this embodiment of the invention is depicted in Figure 2 (lower field). The multispecific antibody according to Figure 2 (lower field) is herein also referred to as "CrossMAb-Fab".

In one embodiment of the invention, the multispecific antibody comprises a first light chain and a first heavy chain derived from a first antibody which specifically binds to said first antigen and a third antigen. This embodiments includes the variable light chain domain and the variable heavy chain domain in a so called dual-acting Fab, as described previously (WO 2013/174873).

In one embodiment of the invention, the multispecific antibody is devoid of an Fc domain and comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH and CH1 domains (i.e. VH-CH1 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid; or
   ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In one embodiment of the invention, the multispecific antibody is devoid of an Fc domain and comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH and CH1 domains (i.e. VH-CH1 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid.

In one embodiment of the invention, the multispecific antibody is devoid of an Fc domain and comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH and CH1 domains (i.E. VH-CH1 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In one embodiment of the invention, the multispecific antibody comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH, CH1, CH2 and CH3 domains (i.e. VH-CH1-CH2-CH3 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid; or
   ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In one embodiment of the invention, the multispecific antibody comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH, CH1, CH2 and CH3 domains (i.e. VH-CH1-CH2-CH3 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid.

In one embodiment of the invention, the multispecific antibody comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH, CH1, CH2 and CH3 domains (i.e. VH-CH1-CH2-CH3 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
   i) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

One embodiment of the invention relates to a multispecific antibody, wherein in addition to the above modifications, in the respective other CL domain (i.e. of the second heavy chain or the first light chain) the amino acid at position 124 (numbering according to Kabat) is substituted by a negatively charged amino acid. The introduced negative charge at position 124 supports pairing of the CL domain with the corresponding CH1 domain (both domains derived from either the first or the second antibody). This is due to the fact that the introduced negatively charged amino acid at position 124 (numbering according to Kabat) in the CL domain faces the positively charged amino acid K at position 124 on the corresponding CH1 domain in the tertiary structure of the multispecific antibody. As a result, the negative charge originally present in this environment caused by the glutamic acid (E) residue at position 123 (numbering according to Kabat) in the CL domain is enhanced by introducing a second negatively charged amino acid, thereby improving the pairing of CL and CH1. By the amino acid substitution according to this embodiment, the yield of the multispecific antibody can be further improved and the formation of side products can be further impaired.

One embodiment of the invention relates to a multispecific antibody,
i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid and wherein in the constant domain CL of the second heavy chain the amino acid at position 124 (numbering according to Kabat) is substituted by a negatively charged amino acid; or
ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid and wherein in the constant domain CL of the first light chain the amino acid at position 124 (numbering according to Kabat) is substituted by a negatively charged amino acid.

One embodiment of the invention relates to a multispecific antibody
i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D and wherein in the constant domain CL of the second heavy chain the amino acid at position 124 (numbering according to Kabat) is substituted by an amino acid selected from E or D; or
ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and wherein in the constant domain CL of the first light chain the amino acid at position 124 (numbering according to Kabat) is substituted by an amino acid selected from E or D.

One embodiment of the invention relates to a multispecific antibody, wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid and wherein in the constant domain CL of the second heavy chain the amino acid at position 124 (numbering according to Kabat) is substituted by a negatively charged amino acid.

One embodiment of the invention relates to a multispecific antibody, wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid and wherein in the constant domain CL of the second heavy chain the amino acid at position 124 (numbering according to Kabat) is substituted by an amino acid selected from E or D, in one preferred embodiment by E.

One embodiment of the invention relates to a multispecific antibody wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D and wherein in the constant domain CL of the second heavy chain the amino acid at position 124 (numbering according to Kabat) is substituted by an amino acid selected from E or D, in one preferred embodiment by E.

In one embodiment of the invention, in the constant domain CL of the first light chain the amino acids at position 124 and 123 (numbering according to Kabat) are substituted independently from each other by an amino acid selected from K and R; and in the constant domain CH1 of the first heavy chain the amino acids at position 147 and 213 (numbering according to EU index of Kabat) are substituted independently from each other by an amino acid selected from E or D; and in the constant domain CL of the second heavy chain the amino acid at position 124 (numbering according to Kabat) is substituted by an amino acid selected from E and D, in one preferred embodiment by E.

In one embodiment of the invention, in the constant domain CL of the first light chain the amino acids at position 124 and 123 (numbering according to Kabat) are substituted by K; and in the constant domain CH1 of the first heavy chain the amino acids at position 147 and 213 (numbering according to EU index of Kabat) are substituted by E; and in the constant domain CL of the second heavy chain the amino acid at position 124 (numbering according to Kabat) is substituted by an amino acid selected from E and D, in one preferred embodiment by E.

One embodiment of the invention relates to a multispecific antibody, wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid and wherein in the constant domain CL of the first light chain the amino acid at position 124 (numbering according to Kabat) is substituted by a negatively charged amino acid.

One embodiment of the invention relates to a multispecific antibody, wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid and wherein in the constant domain CL of the first light chain the amino acid at position 124 (numbering according to Kabat) is substituted by an amino acid selected from E or D, in one preferred embodiment by E.

One embodiment of the invention relates to a multispecific antibody, wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and wherein in the constant domain CL of the first light chain the amino acid at position 124 (numbering according to Kabat) is substituted by an amino acid selected from E or D, in one preferred embodiment by E.

In one embodiment of the invention, in the constant domain CL of the second heavy chain the amino acids at position 124 and 123 (numbering according to Kabat) are substituted independently from each other by an amino acid selected from K and R; and in the constant domain CH1 of the second light chain the amino acids at position 147 and 213 (numbering according to EU index of Kabat) are substituted independently from each other by an amino acid selected from E or D; and in the constant domain CL of the first light chain the amino acid at position 124 (numbering according to Kabat) is substituted by an amino acid selected from E and D, in one preferred embodiment by E.

In one embodiment of the invention, in the constant domain CL of the second heavy chain the amino acids at position 124 and 123 (numbering according to Kabat) are substituted by K; and in the constant domain CH1 of the second light chain the amino acids at position 147 and 213 (numbering according to EU index of Kabat) are substituted by E; and in the constant domain CL of the first light chain the amino acid at position 124 (numbering according to Kabat) is substituted by an amino acid selected from E and D, in one preferred embodiment by E.

One embodiment of the invention relates to a multispecific antibody, wherein in the variable domain VL of the first light chain the amino acid at position 38 (numbering according to Kabat) is substituted by an amino acid selected from K, R and H; and wherein in the variable domain VH of the first heavy chain the amino acid at position 39 (numbering according to Kabat) is substituted by an amino acid selected from E and D; and wherein in the variable domain VL of the second heavy chain the amino acid at position 38 (numbering according to Kabat) is substituted by an amino acid selected from E and D; and wherein in the variable domain VH of the second light chain the amino acid at position 39 (numbering according to Kabat) is substituted by an amino acid selected from K, R and H.

In one embodiment of the invention, in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention
- in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D; or
- in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E; and
in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E; and in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E; and in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention
- in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by an amino acid selected from E and D; or
- in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted by an amino acid selected from E and D; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by an amino acid selected from E and D; or
- in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted by an amino acid selected from E and D; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by E; or
- in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted E; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by an amino acid selected from E and D; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by an amino acid selected from E and D; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by E; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by E; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by E; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by E; and
in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by E; and in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by E; and in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In case the multispecific antibody according to the invention comprises the an Fc region and/or [a first heavy chain including the domains VH-CH1-CH2-CH3 and a modified second heavy chain including the domains VL-CL-CH2-CH3], an additional aspect of the invention is to provide an approach in order to improve the ratio of the desired multispecific antibody compared to undesired side products (that may for example be formed by misparing of the first heavy chain with another first heavy chain, or mispairing of the second heavy chain with another second heavy chain). According to the invention this can be additionally supported by amino acid substitutions in the CH3 domains of the first heavy chain and the second heavy chain, respectively. By these approaches, which are to be described in further detail in the following paragraphs, heterodimerisation of the first heavy chain and the modified second heavy chain is improved.

Thus, one embodiment of the invention is a multispecific antibody according to the invention, which comprises a first heavy chain including a CH3 domain derived from said first antibody and a second heavy chain including a CH3 domain derived from said second antibody, wherein both CH3 domains are engineered in a complementary manner by respective amino acid substitutions, in order to support heterodimerisation of the first heavy chain and the modified second heavy chain.

Several approaches for CH3-modifications in order to support heterodimerization have been described, for example in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291. Typically, in the approaches known in the art, the CH3 domain of the first heavy chain and the CH3 domain of the second heavy chain are both engineered in a complementary manner so that the heavy chain comprising one engineered CH3 domain can no longer homodimerize with another heavy chain of the same structure (e.g. a CH3-engineered first heavy chain can no longer homodimerize with another CH3-engineered first heavy chain; and a CH3-engineered second heavy chain can no longer homodimerize with another CH3-engineered second heavy chain). Thereby the heavy chain comprising one engineered CH3 domain is forced to heterodimerize with another heavy chain comprising the CH3 domain, which is engineered in a complementary manner. For this embodiment of the invention, the CH3 domain of the first heavy chain and the CH3 domain of the second heavy chain are engineered in a complementary manner by amino acid substitutions, such that the first heavy chain and the second heavy chain are forced to heterodimerize, whereas the first heavy chain and the second heavy chain can no longer homodimerize (e.g. for sterical reasons).

The different approaches for supporting heavy chain heterodimerization known in the art, that were cited and included above, are contemplated as different alternatives used in a multispecific antibody according to the invention, which comprises a "non-crossed Fab region" derived from a first antibody, which specifically binds to a first antigen, and a "crossed Fab region" derived from a second antibody, which specifically binds to a second antigen, in combination with the particular amino acid substitutions described above for the invention.

In one embodiment of the multispecific antibody according to the invention, which comprises a first heavy chain including a CH3 domain derived from said first antibody and a second heavy chain including a CH3 domain derived from said second antibody, the CH3 domains of the first and second heavy chain are engineered by the so-called "knob-into-hole" technology, which is described in detail providing several examples in e.g. WO 96/027011, Ridgway, J.B., et al., Protein Eng. 9 (1996) 617-621; Merchant, A.M., et al., Nat. Biotechnol. 16 (1998) 677-681; and WO 98/ 050431. In the "knob-into-hole" technology, within the interface formed between the CH3 domain of the first heavy chain and the CH3 domain of the second heavy chain in the tertiary structure of the antibody, particular amino acids on each CH3 domain are engineered to produce a protuberance ("knob") in the CH3 domain of one heavy chain and a cavity ("hole") in the CH3 domain of the other heavy chain, respectively. In the tertiary structure of the multispecific antibody the introduced protuberance in the CH3 domain of the one heavy chain is positionable in the introduced cavity in the CH3 domain of the other heavy chain. Each of the heavy chains can comprise the "knob" in its CH3 domain while the other heavy chain comprises the "hole" in its CH3 domain.

Thus, one embodiment relates to a multispecific antibody according to the invention, wherein in the tertiary structure of the antibody the CH3 domain of the first heavy chain and the CH3 domain of the second heavy chain form an interface that is located between the respective antibody CH3 domains, wherein the respective amino acid sequences of the CH3 domain of the first heavy chain and the CH3 domain of the second heavy chain each comprise a set of amino acids that is located within said interface in the tertiary structure of the antibody,
- wherein from the set of amino acids that is located in the interface in the CH3 domain of one heavy chain at least one amino acid residue is substituted by an amino acid residue having a larger side chain volume than the original amino acid residue, thereby generating a protuberance within the interface, wherein the protuberance is located in the CH3 domain of the one heavy chain, and wherein the protuberance is positionable in a cavity located in the CH3 domain of the other heavy chain within the interface; and
- wherein from the set of amino acids that is located in the interface in the CH3 domain of the other heavy chain at least one amino acid residue is substituted by an amino acid residue having a smaller side chain volume than the original amino acid residue, thereby generating a cavity within the interface, wherein the cavity is located in the CH3 domain of the other heavy chain, and wherein in the cavity the protuberance within the interface located in the CH3 domain of the one heavy chain is positionable. The multispecific antibody according to this embodiment is herein also referred to as "CH3(KiH)-engineered multispecific antibody" (wherein the abbreviation "KiH" stands for the "knob-into-hole technology").

According to this embodiment of the invention relating to the CH3(KiH)-engineered multispecific antibody, the characteristics of the CH3(KiH)-engineered multispecific antibody, as well as the various embodiments of said CH3(KiH)-engineered multispecific antibody, which are described in further detail below, can be combined with either of the embodiments of said multispecific antibody as described above.

In one embodiment of said CH3(KiH)-engineered multispecific antibody according to the invention said amino acid residue having a larger side chain volume than the original amino acid residue is selected from R, F, Y and W.

In one embodiment of said CH3(KiH)-engineered multispecific antibody according to the invention said amino acid residue having a smaller side chain volume than the original amino acid residue is selected from A, S, T and V.

In one embodiment of said CH3(KiH)-engineered multispecific antibody according to the invention said amino acid residue having a larger side chain volume than the original amino acid residue is selected from R, F, Y and W; and said amino acid residue having a smaller side chain volume than the original amino acid residue is selected from A, S, T and V.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W, the amino acid R at position 409 (numbering according to EU index of Kabat) is substituted by D, and the amino acid K at position 370 (numbering according to EU index of Kabat) is substituted by E; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V, the amino acid D at position 399 (numbering according to EU index of Kabat) is substituted by K, and the amino acid E at position 357 (numbering according to EU index of Kabat) is substituted by K.

In addition to the engineering of the CH3 domains of the first and second heavy chain by the "knob-into-hole" technology, the introduction of a disulfide bridge further stabilizes the heterodimers (Atwell, S., et al., J. Mol. Biol. 270 (1997) 26-35; Merchant, A.M., et al., Nature Biotech. 16 (1998) 677-681). Thereby the additional introduction of a disulfice bridge further increases the yield of the multispecific antibody according to the invention.

Therefore, in one embodiment of said CH3(KiH)-engineered multispecific antibody according to the invention, from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.

In one embodiment of said CH3(KiH)-engineered multispecific antibody according to the invention said amino acid residue having a larger side chain volume than the original amino acid residue is selected from R, F, Y and W; and from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.

In one embodiment of said CH3(KiH)-engineered multispecific antibody according to the invention said amino acid residue having a smaller side chain volume than the original amino acid residue is selected from A, S, T and V; and from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.

In one embodiment of said CH3(KiH)-engineered multispecific antibody according to the invention said amino acid residue having a larger side chain volume than the original amino acid residue is selected from R, F, Y and W; said amino acid residue having a smaller side chain volume than the original amino acid residue is selected from A, S, T and V; and from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.

In one embodiment of said CH3(KiH)-engineered multispecific antibody according to the invention said amino acid residue having a larger side chain volume than the original amino acid residue is selected from R, F, Y and W; and in the CH3 domain of the one heavy chain (the heavy chain comprising the "knob") either the amino acid E at position 356 (numbering according to EU index of Kabat) or the amino acid S at position 354 (numbering according to EU index of Kabat) is substituted by C and in the CH3 domain of the other heavy chain (the heavy chain comprising the "hole") the amino acid Y at position 349 (numbering according to EU index of Kabat) is substituted by C.

In one embodiment of said CH3(KiH)-engineered multispecific antibody according to the invention said amino acid residue having a smaller side chain volume than the original amino acid residue is selected from A, S, T and V; and in the CH3 domain of the one heavy chain (the heavy chain comprising the "knob") either the amino acid E at position 356 (numbering according to EU index of Kabat) or the amino acid S at position 354 (numbering according to EU index of Kabat) is substituted by C and in the CH3 domain of the other heavy chain (the heavy chain comprising the "hole") the amino acid Y at position 349 (numbering according to EU index of Kabat) is substituted by C..

In one embodiment of said CH3(KiH)-engineered multispecific antibody according to the invention said amino acid residue having a larger side chain volume than the original amino acid residue is selected from R, F, Y and W; and said amino acid residue having a smaller side chain volume than the original amino acid residue is selected from A, S, T and V; and in the CH3 domain of the one heavy chain (the heavy chain comprising the "knob") either the amino acid E at position 356 (numbering according to EU index of Kabat) or the amino acid S at position 354 (numbering according to EU index of Kabat) is substituted by C and in the CH3 domain of the other heavy chain (the heavy chain comprising the "hole") the amino acid Y at position 349 (numbering according to EU index of Kabat) is substituted by C.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W and either the amino acid E at position 356 (numbering according to EU index of Kabat) or the amino acid S at position 354 (numbering according to EU index of Kabat) is substituted by C; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A, the amino acid Y at position 407 (numbering according to EU index of Kabat) is substituted by V and the amino acid Y at position 349 (numbering according to EU index of Kabat) is substituted by C.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W and the amino acid Y at position 349 (numbering according to EU index of Kabat) is substituted by C; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A, the amino acid Y at position 407 (numbering according to EU index of Kabat) is substituted by V and either the amino acid E at position 356 (numbering according to EU index of Kabat) or the amino acid S at position 354 (numbering according to EU index of Kabat) is substituted by C.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W, the amino acid R at position 409 (numbering according to EU index of Kabat) is substituted by D, the amino acid K at position 370 (numbering according to EU index of Kabat) is substituted by E and either the amino acid E at position 356 (numbering according to EU index of Kabat) or the amino acid S at position 354 (numbering according to EU index of Kabat) is substituted by C; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V, the amino acid D at position 399 (numbering according to EU index of Kabat) is substituted by K, the amino acid E at position 357 (numbering according to EU index of Kabat) is substituted by K and the amino acid Y at position 349 (numbering according to EU index of Kabat) is substituted by C.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W, the amino acid R at position 409 (numbering according to EU index of Kabat) is substituted by D, the amino acid K at position 370 (numbering according to EU index of Kabat) is substituted by E and the amino acid Y at position 349 (numbering according to EU index of Kabat) is substituted by C; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V, the amino acid D at position 399 (numbering according to EU index of Kabat) is substituted by K, the amino acid E at position 357 (numbering according to EU index of Kabat) is substituted by K and either the amino acid E at position 356 (numbering according to EU index of Kabat) or the amino acid S at position 354 (numbering according to EU index of Kabat) is substituted by C.

Apart from the "knob-into-hole technology" other techniques for modifying the CH3 domains of the heavy chains of a multispecific antibody to enforce heterodimerization are known in the art. These technologies, especially the ones described in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954 and WO 2013/096291 are contemplated herein as alternatives to the "knob-into-hole technology" in combination with a multispecific antibody according to the invention.

In one embodiment of a multispecific antibody according to the invention the approach described in EP 1870459 is used to support heterodimerization of the first heavy chain and the second heavy chain of the multispecific antibody. This approach is based on the introduction of charged amino acids with opposite charges at specific amino acid positions in the CH3/CH3-domain-interface between both, the first and the second heavy chain.

Accordingly, this embodiment relates to a multispecific antibody according to the invention, wherein in the tertiary structure of the antibody the CH3 domain of the first heavy chain and the CH3 domain of the second heavy chain form an interface that is located between the respective antibody CH3 domains, wherein the respective amino acid sequences of the CH3 domain of the first heavy chain and the CH3 domain of the second heavy chain each comprise a set of amino acids that is located within said interface in the tertiary structure of the antibody, wherein from the set of amino acids that is located in the interface in the CH3 domain of one heavy chain a first amino acid is substituted by a positively charged amino acid and from the set of amino acids that is located in the interface in the CH3 domain of the other heavy chain a second amino acid is substituted by a negatively charged amino acid. The multispecific antibody according to this embodiment is herein also referred to as "CH3(+/-)-engineered multispecific antibody" (wherein the abbreviation "+/-" stands for the oppositely charged amino acids that were introduced in the respective CH3 domains).

In one embodiment of said CH3(+/-)-engineered multispecific antibody according to the invention the positively charged amino acid is selected from K, R and H; and the negatively charged amino acid is selected from E or D.

In one embodiment of said CH3(+/-)-engineered multispecific antibody according to the invention the positively charged amino acid is selected from K and R; and the negatively charged amino acid is selected from E or D.

In one embodiment of said CH3(+/-)-engineered multispecific antibody according to the invention the positively charged amino acid is K; and the negatively charged amino acid is E.

In one embodiment of said CH3(+/-)-engineered multispecific antibody according to the invention in the CH3 domain of one heavy chain the amino acid R at position 409 (numbering according to EU index of Kabat) is substituted by D and the amino acid K at position 370 (numbering according to EU index of Kabat) is substituted by E; and in the CH3 domain of the other heavy chain the amino acid D at position 399 (numbering according to EU index of Kabat) is substituted by K and the amino acid E at position 357 (numbering according to EU index of Kabat) is substituted by K.

In one embodiment of a multispecific antibody according to the invention the approach described in WO2013/157953 is used to support heterodimerization of the first heavy chain and the second heavy chain of the multispecific antibody. In one embodiment of said CH3-engineered multispecific antibody according to the invention, in the CH3 domain of one heavy chain the amino acid T at position 366 (numbering according to EU index of Kabat) is substituted by K; and in the CH3 domain of the other heavy chain the amino acid L at position 351 (numbering according to EU index of Kabat) is substituted by D. In another embodiment of said CH3-engineered multispecific antibody according to the invention, in the CH3 domain of one heavy chain the amino acid T at position 366 (numbering according to EU index of Kabat) is substituted by K and the amino acid L at position 351 (numbering according to EU index of Kabat) is substituted by K; and in the CH3 domain of the other heavy chain the amino acid L at position 351 (numbering according to EU index of Kabat) is substituted by D.

In another embodiment of said CH3-engineered multispecific antibody according to the invention, in the CH3 domain of one heavy chain the amino acid T at position 366 (numbering according to EU index of Kabat) is substituted by K and the amino acid L at position 351 (numbering according to EU index of Kabat) is substituted by K; and in the CH3 domain of the other heavy chain the amino acid L at position 351 (numbering according to EU index of Kabat) is substituted by D. Additionally at least one of the following substitutions is comprised in the CH3 domain of the other heavy chain: the amino acid Y at position 349 (numbering according to EU index of Kabat) is substituted by E, the amino acid Y at position 349 (numbering according to EU index of Kabat) is substituted by D and the amino acid L at position 368 (numbering according to EU index of Kabat) is substituted by E. In one embodiment the amino acid L at position 368 (numbering according to EU index of Kabat) is substituted by E.

In one embodiment of a multispecific antibody according to the invention the approach described in WO2012/058768 is used to support heterodimerization of the first heavy chain and the second heavy chain of the multispecific antibody. In one embodiment of said CH3-engineered multispecific antibody according to the invention, in the CH3 domain of one heavy chain the amino acid L at position 351 (numbering according to EU index of Kabat) is substituted by Y and the amino acid Y at position 407 (numbering according to EU index of Kabat) is substituted by A; and in the CH3 domain of the other heavy chain the amino acid T at position 366 (numbering according to EU index of Kabat) is substituted by A and the amino acid K at position 409 (numbering according to EU index of Kabat) is substituted by F. In another embodiment, in addition to the aforementioned substitutions, in the CH3 domain of the other heavy chain at least one of the amino acids at positions 411 (originally T), 399 (originally D), 400 (originally S), 405 (originally F), 390 (originally N) and 392 (originally K) is substituted. Preferred substitutions are:
- substituting the amino acid T at position 411 (numbering according to EU index of Kabat) by an amino acid selected from N, R, Q, K, D, E and W;
- substituting the amino acid D at position 399 (numbering according to EU index of Kabat) by an amino acid selected from R, W, Y, and K;
- substituting the amino acid S at position 400 (numbering according to EU index of Kabat) by an amino acid selected from E, D, R and K;
- substituting the amino acid F at position 405 (numbering according to EU index of Kabat) by an amino acid selected from I, M, T, S, V and W;
- substituting the amino acid N at position 390 (numbering according to EU index of Kabat) by an amino acid selected from R, K and D; and
- substituting the amino acid K at position 392 (numbering according to EU index of Kabat) by an amino acid selected from V, M, R, L, F and E.

In another embodiment of said CH3-engineered multispecific antibody according to the invention (engineered according to WO2012/058768), in the CH3 domain of one heavy chain the amino acid L at position 351 (numbering according to EU index of Kabat) is substituted by Y and the amino acid Y at position 407 (numbering according to EU index of Kabat) is substituted by A; and in the CH3 domain of the other heavy chain the amino acid T at position 366 (numbering according to EU index of Kabat) is substituted by V and the amino acid K at position 409 (numbering according to EU index of Kabat) is substituted by F. In another embodiment of said CH3-engineered multispecific antibody according to the invention, in the CH3 domain of one heavy chain the amino acid Y at position 407 (numbering according to EU index of Kabat) is substituted by A; and in the CH3 domain of the other heavy chain the amino acid T at position 366 (numbering according to EU index of Kabat) is substituted by A and the amino acid K at position 409 (numbering according to EU index of Kabat) is substituted by F. In said last aforementioned embodiment, in the CH3 domain of said other heavy chain the amino acid K at position 392 (numbering according to EU index of Kabat) is substituted by E, the amino acid T at position 411 (numbering according to EU index of Kabat) is substituted by E, the amino acid D at position 399 (numbering according to EU index of Kabat) is substituted by R and the amino acid S at position 400 (numbering according to EU index of Kabat) is substituted by R.

In one embodiment of a multispecific antibody according to the invention the approach described in WO 2011/143545 is used to support heterodimerization of the first heavy chain and the second heavy chain of the multispecific antibody. In one embodiment of said CH3-engineered multispecific antibody according to the invention, amino acid modifications in the CH3 domains of both heavy chains are introduced at positions 368 and/or 409.

In one embodiment of a multispecific antibody according to the invention the approach described in WO 2011/090762 is used to support heterodimerization of the first heavy chain and the second heavy chain of the multispecific antibody. WO 2011/090762 relates to amino acid modifications according to the "knob-into-hole" technology. In one embodiment of said CH3(KiH)-engineered multispecific antibody according to the invention, in the CH3 domain of one heavy chain the amino acid T at position 366 (numbering according to EU index of Kabat) is substituted by W; and in the CH3 domain of the other heavy chain the amino acid Y at position 407 (numbering according to EU index of Kabat) is substituted by A. In another embodiment of said CH3(KiH)-engineered multispecific antibody according to the invention, in the CH3 domain of one heavy chain the amino acid T at position 366 (numbering according to EU index of Kabat) is substituted by Y; and in the CH3 domain of the other heavy chain the amino acid Y at position 407 (numbering according to EU index of Kabat) is substituted by T.

In one embodiment of a multispecific antibody according to the invention, which is of IgG2 isotype, the approach described in WO 2011/090762 is used to support heterodimerization of the first heavy chain and the second heavy chain of the multispecific antibody.

In one embodiment of a multispecific antibody according to the invention, the approach described in WO 2009/089004 is used to support heterodimerization of the first heavy chain and the second heavy chain of the multispecific antibody. In one embodiment of said CH3-engineered multispecific antibody according to the invention, in the CH3 domain of one heavy chain the amino acid K or N at position 392 (numbering according to EU index of Kabat) is substituted by a negatively charged amino acid (in one preferred embodiment by E or D, in one preferred embodiment by D); and in the CH3 domain of the other heavy chain the amino acid D at position 399 the amino acid E or D at position 356 or the amino acid E at position 357 (numberings according to EU index of Kabat) is substituted by a positively charged amino acid (in one preferred embodiment K or R, in one preferred embodiment by K, in one preferred embodiment the amino acids at positions 399 or 356 are substituted by K). In one further embodiment, in addition to the aforementioned substitutions, in the CH3 domain of the one heavy chain the amino acid K or R at position 409 (numbering according to EU index of Kabat) is substituted by a negatively charged amino acid (in one preferred embodiment by E or D, in one preferred embodiment by D). In one even further embodiment, in addition to or alternatively to the aforementioned substitutions, in the CH3 domain of the one heavy chain the amino acid K at position 439 and/or the amino acid K at position 370 (numbering according to EU index of Kabat) is substituted independently from each other by a negatively charged amino acid (in one preferred embodiment by E or D, in one preferred embodiment by D).

In one embodiment of a multispecific antibody according to the invention, the approach described in WO 2007/147901 is used to support heterodimerization of the first heavy chain and the second heavy chain of the multispecific antibody. In one embodiment of said CH3-engineered multispecific antibody according to the invention, in the CH3 domain of one heavy chain the amino acid K at position 253 (numbering according to EU index of Kabat) is substituted by E, the amino acid D at position 282 (numbering according to EU index of Kabat) is substituted by K and the amino acid K at position 322 (numbering according to EU index of Kabat) is substituted by D; and in the CH3 domain of the other heavy chain the amino acid D at position 239 (numbering according to EU index of Kabat) is substituted by K, the amino acid E at position 240 (numbering according to EU index of Kabat) is substituted by K and the amino acid K at position 292 (numbering according to EU index of Kabat) is substituted by D.

In one embodiment of a multispecific antibody according to the invention, the approach described in WO 2007/110205 is used to support heterodimerization of the first heavy chain and the second heavy chain of the multispecific antibody.

In one embodiment of the invention the multispecific antibody is a bispecific antibody or a trispecific antibody. In one preferred embodiment of the invention the multispecific antibody is a bispecific antibody.

In one embodiment of the invention, the antibody is a bivalent or trivalent antibody. In one embodiment of the invention, the antibody is a bivalent antibody.

In one embodiment of the invention, the multispecific antibody comprises immunoglobulin constant regions of one or more immunoglobulin classes. Immunoglobulin classes include IgG, IgM, IgA, IgD, and IgE isotypes and, in the case of IgG and IgA, their subtypes.

In one embodiment of the invention, the multispecific antibody has a constant domain structure of an IgG type antibody. In one further embodiment of the invention, the multispecific antibody is characterized in that said multispecific antibody is of human IgG1 subclass, or of human IgG1 subclass with the mutations L234A and L235A. In one further embodiment of the invention, the multispecific antibody is characterized in that said multispecific antibody is of human IgG2 subclass. In one further embodiment of the invention, the multispecific antibody is characterized in that said multispecific antibody is of human IgG3 subclass. In one further embodiment of the invention, the multispecific antibody is characterized in that said multispecific antibody is of human IgG4 subclass or, of human IgG4 subclass with the additional mutation S228P. In one further embodiment of the invention, the multispecific antibody is characterized in that said multispecific antibody is of human IgG1 or human IgG4 subclass. In one further embodiment of the invention, the multispecific antibody is characterized in that said multispecific antibody is of human IgG1 subclass with the mutations L234A and L235A (numbering according to EU index of Kabat). In one further embodiment of the invention, the multispecific antibody is characterized in that said multispecific antibody is of human IgG1 subclass with the mutations L234A, L235A and P329G (numbering according to EU index of Kabat). In one further embodiment of the invention, the multispecific antibody is characterized in that said multispecific antibody is of human IgG4 subclass with the mutations S228P and L235E (numbering according to EU index of Kabat). In one further embodiment of the invention, the multispecific antibody is characterized in that said multispecific antibody is of human IgG4 subclass with the mutations S228P, L235E and P329G (numbering according to EU index of Kabat).

In one embodiment, an antibody comprising a heavy chain including a CH3 domain as specified herein, comprises an additional C-terminal glycine-lysine dipeptide (G446 and K447, numbering according to EU index of Kabat). In one embodiment, an antibody comprising a heavy chain including a CH3 domain, as specified herein, comprises an additional C-terminal glycine residue (G446, numbering according to EU index of Kabat).

The multispecific antibody is prepared by recombinant methods. Thus, the invention also relates to a method for the preparation of a multispecific antibody according to the invention, comprising the steps of
- transforming a host cell with vectors comprising nucleic acids encoding
   a) the first light chain as defined for a multispecific antibody according to the invention derived from a first antibody which specifically binds to a first antigen;
   b) the first heavy chain as defined for a multispecific antibody according to the invention derived from a first antibody which specifically binds to a first antigen;
   c) the second light chain as defined for a multispecific antibody according to the invention derived from a second antibody which specifically binds to a second antigen; and
   d) the second heavy chain as defined for a multispecific antibody according to the invention derived from a second antibody which specifically binds to a second antigen,
- culturing said host cell under conditions that allow synthesis of said multispecific antibody; and
- recovering said multispecific antibody from said host cell culture.

In one embodiment of a method according to the invention, the host cell is transformed with vectors comprising nucleic acids encoding
a) the first light chain derived from a first antibody which specifically binds to a first antigen, wherein in the constant domain CL of the first light chain the amino acids at position 124 and 123 (numbering according to Kabat) are substituted independently from each other by an amino acid selected from K, R and H (in one preferred embodiment by K or R, in one preferred embodiment by K);
b) the first heavy chain derived from a first antibody which specifically binds to a first antigen, wherein in the constant domain CH1 of the first heavy chain the amino acids at position 147 and 213 (numbering according to EU index of Kabat) are substituted independently from each other by an amino acid selected from E or D (in one preferred embodiment by E);
c) the second light chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and
d) the second heavy chain derived from a second antibody which specifically binds to a second antigen, wherein in the second heavy chain the variable domain VH is replaced by the variable domain VL of the second light chain and the constant domain CH1 is replaced by the constant domain CL of the second light chain.

In one embodiment of a method according to the invention, the host cell is transformed with vectors comprising nucleic acids encoding
a) the first light chain derived from a first antibody which specifically binds to a first antigen;
b) the first heavy chain derived from a first antibody which specifically binds to a first antigen,;
c) the second light chain derived from a second antibody which specifically binds to a second antigen, wherein in the second light chain the variable domain VL is replaced by the variable domain VH of the second heavy chain and the constant domain CL is replaced by the constant domain CH1 of the second heavy chain,
   and wherein in the constant domain CL of the second heavy chain the amino acids at position 124 and 123 (numbering according to Kabat) are substituted independently from each other by an amino acid selected from K, R and H (in one preferred embodiment by K or R, in one preferred embodiment by K); and
d) the second heavy chain derived from a second antibody which specifically binds to a second antigen, wherein in the second heavy chain the variable domain VH is replaced by the variable domain VL of the second light chain and the constant domain CH1 is replaced by the constant domain CL of the second light chain,
   and wherein in the constant domain CH1 of the second light chain the amino acids at position 147 and 213 (numbering according to EU index of Kabat) are substituted independently from each other by an amino acid selected from E or D (in one preferred embodiment by E).

Another object of the invention is a multispecific antibody produced by a method according to the invention.

Another object of the invention is a host cell comprising
a) a vector comprising nucleic acids encoding the first light chain of an antibody of the invention;
b) a vector comprising nucleic acids encoding the first heavy chain of an antibody of the invention;
c) a vector comprising nucleic acids encoding the second light chain of an antibody of the invention; and
d) a vector comprising nucleic acids encoding the second heavy chain of an antibody of the invention.

Another object of the invention is a nucleic acid encoding the multispecific antibody according to the invention.

In one embodiment the nucleic acid encodes a first light chain as defined for a multispecific antibody according to the invention. In one embodiment the nucleic acid encodes a first light chain as defined for a multispecific antibody according to the invention, wherein in the constant domain CL of the first light chain the amino acids at position 124 and 123 (numbering according to Kabat) are substituted independently from each other by an amino acid selected from K, R and H (in one embodiment selected from K and R, in one embodiment the amino acid is K). In one embodiment the nucleic acid encodes a first light chain as defined for a multispecific antibody according to the invention, wherein in the constant domain CL of the first light chain the amino acid at position 124 (numbering according to Kabat) is substituted by an amino acid selected from E and D (in one embodiment the amino acid is E).

In one embodiment the nucleic acid encodes a first heavy chain as defined for a multispecific antibody according to the invention. In one embodiment the nucleic acid encodes a first heavy chain as defined for a multispecific antibody according to the invention, wherein in the constant domain CH1 of the first heavy chain the amino acids at position 147 and 213 (numbering according to EU index of Kabat) are substituted independently from each other by an amino acid selected from E or D (in one embodiment the amino acid is E).

In one embodiment the nucleic acid encodes a modified second light chain as defined for a multispecific antibody according to the invention. In one embodiment the nucleic acid encodes a modified second light chain as defined for a multispecific antibody according to the invention, wherein in the constant domain CH1 of the second light chain the amino acids at position 147 and 213 (numbering according to EU index of Kabat) are substituted independently from each other by an amino acid selected from E or D (in one embodiment the amino acid is E). In one further embodiment, the nucleic acid encodes a modified second light chain as defined for a multispecific antibody according to the invention, wherein in the variable domain VH the amino acid at position 39 (numbering according to Kabat) is substituted by an amino acid selected from E and D (in one preferred embodiment by E).

In one embodiment the nucleic acid encodes a modified second heavy chain as defined for a multispecific antibody according to the invention. In one embodiment the nucleic acid encodes a modified second heavy chain as defined for a multispecific antibody according to the invention, wherein in the constant domain CL of the second heavy chain the amino acids at position 124 and 123 (numbering according to Kabat) are substituted independently from each other by an amino acid selected from K, R and H (in one embodiment selected from K and R, in one embodiment the amino acid is K). In one further embodiment, the nucleic acid encondes a modified second heavy chain as defined for a multispecific antibody according to the invention, wherein in the constant domain CL the amino acid at position 124 (numbering according to Kabat) is substituted by an amino acid selected from E and D (in one embodiment the amino acid is E). In one further embodiment, the nucleic acid encondes a modified second heavy chain as defined for a multispecific antibody according to the invention, wherein in the variable domain VL of the second heavy chain the amino acid at position 38 (numbering according to Kabat) is substituted by an amino acid selected from K, R and H (in one preferred embodiment by K or R, in one preferred embodiment by K). In one further embodiment, the nucleic acid encondes a modified second heavy chain as defined for a multispecific antibody according to the invention, wherein in the constant domain CL the amino acid at position 124 (numbering according to Kabat) is substituted by an amino acid selected from E and D; and wherein in the variable domain VL of the second heavy chain the amino acid at position 38 (numbering according to Kabat) is substituted by an amino acid selected from E and D (in one preferred embodiment by E).

In one embodiment, the nucleic acid according to the invention is an isolated nucleic acid.

Another object of the invention is an expression vector comprising a nucleic acid according to the invention, wherein the vector is capable of expressing said nucleic acid in a host cell.

Another object of the invention is a host cell comprising a nucleic acid according to the invention. Another object of the invention is a host cell comprising an expression vector according to the invention. In one embodiment the host cell is a HEK293 cells or a CHO cell.

Another object of the invention is a method of producing an antibody comprising culturing said host cell of the invention so that the antibody is produced. In one embodiment of said method the method further comprises recovering the antibody from the host cell.

Another object of the invention is a pharmaceutical composition comprising a multispecific antibody according to the invention. One aspect of the invention is a pharmaceutical composition comprising a multispecific antibody according to the invention in combination with at least one pharmaceutically acceptable carrier.

In one embodiment, a composition (in one preferred embodiment a pharmaceutical composition) comprising a population of antibodies of the invention comprises an antibody comprising a heavy chain including a CH3 domain, as specified herein, with an additional C-terminal glycine-lysine dipeptide (G446 and K447, numbering according to EU index of Kabat). In one embodiment, a composition comprising a population of antibodies of the invention comprises an antibody comprising a heavy chain including a CH3 domain, as specified herein, with an additional C-terminal glycine residue (G446, numbering according to EU index of Kabat).

In one embodiment, such a composition comprises a population of antibodies comprised of antibodies comprising a heavy chain including a CH3 domain, as specified herein; antibodies comprising a heavy chain including a CH3 domain, as specified herein, with an additional C-terminal glycine residue (G446, numbering according to EU index of Kabat); and antibodies comprising a heavy chain including a CH3 domain, as specified herein, with an additional C-terminal glycine-lysine dipeptide (G446 and K447, numbering according to EU index of Kabat).

Also described herein is an immunoconjugate comprising the multispecific antibody according to the invention coupled to a cytotoxic agent.

Also described herein is the use of a multispecific antibody according to the invention for the manufacture of a pharmaceutical composition. Also described herein is a method for the manufacture of a pharmaceutical composition comprising a multispecific antibody according to the invention, including formulating the multispecific antibody according to the invention in combination with at least one pharmaceutically acceptable carrier.

Another object of the invention is the multispecific antibody according to the invention for use as a medicament. Another object of the invention is the multispecific antibody according to the invention for use in the treatment of cancer. Another object of the invention is the multispecific antibody according to the invention for use in the treatment of inflammatory diseases, autoimmune diseases, rheumatoid arthritis, psoratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

Another object of the invention is a pharmaceutical composition comprising a multispecific antibody according to the invention in combination with at least one pharmaceutically acceptable carrier for use as a medicament. Another object of the invention is a pharmaceutical composition comprising a multispecific antibody according to the invention in combination with at least one pharmaceutically acceptable carrier for use in the treatment of cancer. Another object of the invention is a pharmaceutical composition comprising a multispecific antibody according to the invention in combination with at least one pharmaceutically acceptable carrier for use in the treatment of inflammatory diseases, autoimmune diseases, rheumatoid arthritis, psoratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

Also described herein is an immunoconjugate comprising the multispecific antibody according to the invention coupled to a cytotoxic agent for use as a medicament. AAlso described herein is an immunoconjugate comprising the multispecific antibody according to the invention coupled to a cytotoxic agent for use in the treatment of cancer. Also described herein is an immunoconjugate comprising the multispecific antibody according to the invention coupled to a cytotoxic agent for use in the treatment of inflammatory diseases, autoimmune diseases, rheumatoid arthritis, psoratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

Also described herein is the use of a multispecific antibody according to the invention for the manufacture of a medicament. Also described herein is the use of a multispecific antibody according to the invention for the manufacture of a medicament for the treatment of cancer. Also described herein is the use of a multispecific antibody according to the invention for the manufacture of a medicament for the treatment of inflammatory diseases, autoimmune diseases, rheumatoid arthritis, psoratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

Also described herein is a method of treatment of a patient suffering from a disease by administering a multispecific antibody according to the invention to the patient in the need of such treatment. Also described herein is a method of treatment of a patient suffering from cancer by administering a multispecific antibody according to the invention to the patient in the need of such treatment. Also described herein is a method of treatment of a patient suffering from at least one of the following diseases including inflammatory diseases, autoimmune diseases, rheumatoid arthritis, psoratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and vascular injury; by administering a multispecific antibody according to the invention to the patient in the need of such treatment.

### III) SPECIFIC EMBODIMENTS OF THE INVENTION

In the following specific embodiments of the invention are listed.
1. A multispecific antibody, comprising
   a) a first light chain and a first heavy chain derived from a first antibody which specifically binds to a first antigen; and
   b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D; or
      ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.
2. The multispecific antibody according to embodiment 1, wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K), and wherein in the constant domain CH1 of the first heavy chain the amino acids at position 147 and 213 (numbering according to EU index of Kabat) are substituted independently from each other by an amino acid selected from E or D (in one preferred embodiment by E).
3. The multispecific antibody according to embodiment 1, wherein in the constant domain CL of the second heavy chain the amino acids at position 124 and 123 (numbering according to Kabat) are substituted independently from each other by an amino acid selected from K, R and H (in one preferred embodiment independently from each other by K or R; in one further preferred embodiment by K), and wherein in the constant domain CH1 of the second light chain the amino acids at position 147 and 213 (numbering according to EU index of Kabat) are substituted independently from each other by an amino acid selected from E or D (in one preferred embodiment by E).
4. The multispecific antibody according to embodiment 1, wherein
   i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D, and in the constant domain CL of the second heavy chain the amino acid at position 124 (numbering according to Kabat) is substituted by an amino acid selected from E and D (in one preferred embodiment by E); or
   ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D, and in the constant domain CL of the first light chain the amino acid at position 124 (numbering according to Kabat) is substituted by an amino acid selected from E and D (in one preferred embodiment by E).
5. The multispecific antibody according to embodiment 2, wherein in the constant domain CL of the second heavy chain the amino acid at position 124 (numbering according to Kabat) is substituted by an amino acid selected from E and D (in one preferred embodiment by E).
6. The multispecific antibody according to embodiment 3, wherein in the constant domain CL of the first light chain the amino acid at position 124 (numbering according to Kabat) is substituted by an amino acid selected from E and D (in one preferred embodiment by E).
7. The multispecific antibody according to any one of the preceding embodiments,
   iii) wherein in the variable domain VL of the first light chain the amino acid at position 38 (numbering according to Kabat) is substituted by an amino acid selected from K, R and H (in one preferred embodiment independently from each other by K or R; in one further preferred embodiment by K); and
      wherein in the variable domain VH of the first heavy chain the amino acid at position 39 (numbering according to Kabat) is substituted by an amino acid selected from E and D (in one preferred embodiment by E); and
   iv) wherein in the variable domain VL of the second heavy chain the amino acid at position 38 (numbering according to Kabat) is substituted by an amino acid selected from E and D (in one preferred embodiment E); and
      wherein in the variable domain VH of the second light chain the amino acid at position 39 (numbering according to Kabat) is substituted by an amino acid selected from K, R and H (in one preferred embodiment by K or R, in one embodiment by K).
8. The multispecific antibody according to any one of the preceding embodiments, wherein the constant domain CL of the first light chain and the constant domain CL of the second heavy chain are of kappa isotype.
9. The multispecific antibody according to any one embodiments 1 to 7, wherein the constant domain CL of the first light chain is of kappa isotype and the constant domain CL of the second heavy chain is of lambda isotype.
10. The multispecific antibody according to any one embodiments 1 to 7, wherein the constant domain CL of the first light chain is of lambda isotype and the constant domain CL of the second heavy chain is of kappa isotype.
11. The multispecific antibody according to any one embodiments 1 to 7, wherein the onstant domain CL of the first light chain and the constant domain CL of the second heavy chain are of lambda isotype.
12. The multispecific antibody according to any one of embodiments 1 to 9, wherein the multispecific antibody comprises a constant domain CL of kappa isotype in the first light chain, wherein in the constant domain CL of the first light chain the amino acid E at position 123 (numbering according to Kabat) is substituted by by an amino acid selected from K, R and H (in one preferred embodiment by K or R; in one further preferred embodiment by K) and the amino acid E at position 124 (numbering according to Kabat) is substituted by by an amino acid selected from K, R and H (in one preferred embodiment by K or R; in one further preferred embodiment by K).
13. The multispecific antibody according to any one of embodiments 1 to 8 and 10, wherein the multispecific antibody comprises a constant domain CL of kappa isotype in the second heavy chain, wherein in the constant domain CL of the second heavy chain the amino acid E at position 123 (numbering according to Kabat) is substituted by by an amino acid selected from K, R and H (in one preferred embodiment by K or R; in one further preferred embodiment by K) and the amino acid E at position 124 (numbering according to Kabat) is substituted by by an amino acid selected from K, R and H (in one preferred embodiment by K or R; in one further preferred embodiment by K).
14. The multispecific antibody according to any one of embodiments 1 to 7, 10 and 11, wherein the multispecific antibody comprises a constant domain CL of lambda isotype in the first light chain, wherein in the constant domain CL of the first light chain the amino acid E at position 123 (numbering according to Kabat) is substituted by by an amino acid selected from K, R and H (in one preferred embodiment independently from each other by K or R; in one further preferred embodiment by K) and the amino acid Q at position 124 (numbering according to Kabat) is substituted by by an amino acid selected from K, R and H (in one preferred embodiment independently from each other by K or R; in one further preferred embodiment by K).
15. The multispecific antibody according to any one of embodiments 1 to 7, 9 and 11, wherein the multispecific antibody comprises a constant domain CL of lambda isotype in the second heavy chain, wherein in the constant domain CL of the second heavy chain the amino acid E at position 123 (numbering according to Kabat) is substituted by by an amino acid selected from K, R and H (in one preferred embodiment independently from each other by K or R; in one further preferred embodiment by K) and the amino acid Q at position 124 (numbering according to Kabat) is substituted by by an amino acid selected from K, R and H (in one preferred embodiment independently from each other by K or R; in one further preferred embodiment by K).
16. The multispecific antibody according to any one of the preceding embodiments, wherein in the tertiary structure of the antibody the CH3 domain of the first heavy chain and the CH3 domain of the second heavy chain form an interface that is located between the respective antibody CH3 domains, wherein the respective amino acid sequences of the CH3 domain of the first heavy chain and the CH3 domain of the second heavy chain each comprise a set of amino acids that is located within said interface in the tertiary structure of the antibody,
   - wherein from the set of amino acids that is located in the interface in the CH3 domain of one heavy chain at least one amino acid residue is substituted by an amino acid residue having a larger side chain volume than the original amino acid residue, thereby generating a protuberance within the interface, wherein the protuberance is located in the CH3 domain of the one heavy chain, and wherein the protuberance is positionable in a cavity located in the CH3 domain of the other heavy chain within the interface; and
   - wherein from the set of amino acids that is located in the interface in the CH3 domain of the other heavy chain at least one amino acid residue is substituted by an amino acid residue having a smaller side chain volume than the original amino acid residue, thereby generating a cavity within the interface, wherein the cavity is located in the CH3 domain of the other heavy chain, and wherein in the cavity the protuberance within the interface located in the CH3 domain of the one heavy chain is positionable.
17. The multispecific antibody according to embodiment 16, wherein said amino acid residue having a larger side chain volume than the original amino acid residue is selected from R, F, Y and W; and/or wherein said amino acid residue having a smaller side chain volume than the original amino acid residue is selected from A, S, T and V.
18. The multispecific antibody according to any one of embodiments 16 or 17, wherein in the CH3 domain of the one heavy chain the amino acid T at position 366 (numbering according to EU index of Kabat) is substituted by W; and in the CH3 domain of the other heavy chain the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V.
19. The multispecific antibody according to any one of embodiments 16 to 18, wherein in the CH3 domain of the one heavy chain the amino acid T at position 366 (numbering according to EU index of Kabat) is substituted by W, the amino acid R at position 409 (numbering according to EU index of Kabat) is substituted by D, and the amino acid K at position 370 (numbering according to EU index of Kabat) is substituted by E; and in the CH3 domain of the other heavy chain the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V, the amino acid D at position 399 (numbering according to EU index of Kabat) is substituted by K, and the amino acid E at position 357 (numbering according to EU index of Kabat) is substituted by K.
20. The multispecific antibody according to any one of embodiments 16 to 19, wherein from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residus.
21. The multispecific antibody according to embodiment 20, wherein in the CH3 domain of the one heavy chain either the amino acid E at position 356 (numbering according to EU index of Kabat) or the amino acid S at position 354 (numbering according to EU index of Kabat) is substituted by C and in the CH3 domain of the other heavy chain the amino acid Y at position 349 (numbering according to EU index of Kabat) is substituted by C.
22. The multispecific antibody according to embodiment 20, wherein in the CH3 domain of the one heavy chain the amino acid Y at position 349 (numbering according to EU index of Kabat) is substituted by C; and in the CH3 domain of the other heavy chain the amino acid S at position 354 (numbering according to EU index of Kabat) is substituted by C.
23. The multispecific antibody according to any one of the preceding embodiments, wherein the antibody is bispecific, trispecific or tetraspecific.
24. The multispecific antibody according to any one of the preceding embodiments, wherein the antibody is bispecific or trispecific.
25. The multispecific antibody according to any one of the preceding embodiments, wherein the antibody is bispecific.
26. The multispecific antibody according to any one of the preceding embodiments, wherein the antibody is bivalent or trivalent.
27. The multispecific antibody according to any one of the preceding embodiments, wherein the antibody is bivalent.
28. The multispecific antibody according to any one of the preceding embodiments, wherein the antibody has a constant domain structure of an IgG type antibody.
29. The multispecific antibody according to any one of the preceding embodiments, wherein the antibody is of human IgG1 or human IgG4 subclass.
30. The multispecific antibody according to any one of the preceding embodiments, wherein the antibody is of human IgG1 subclass with the mutations L234A and L235A (numbering according to EU index of Kabat).
31. The multispecific antibody according to any one of the preceding embodiments, wherein the antibody is of human IgG4 subclass with the mutations S228P and L235E (numbering according to EU index of Kabat).
32. The multispecific antibody according to embodiment 30 or 31, wherein the antibody further comprises a P329G mutation (numbering according to EU index of Kabat).
33. The multispecific antibody according to any one of embodiments 1 to 15, 23 to 27, wherein the antibody is devoid of Fc domains.
34. The multispecific antibody according to any one of the preceding embodiments that specifically binds to human Angiopoietin-2 and human VEGF, wherein
   a) the antibody comprises a variable heavy chain domain (VH) according to SEQ ID NO: 32 (<VH Ang2>) and a variable light chain domain (VL) according to SEQ ID NO: 33 (<VL Ang2>); and
   b) the antibody comprises a variable heavy chain domain (VH) according to SEQ ID NO: 31 (<VH VEGF>) and a variable light chain domain (VL) according to SEQ ID NO: 30 (<VL VEGF>).
35. The multispecific antibody according to any one of the preceding embodiments that specifically binds to human Angiopoietin-2 and human VEGF, wherein
   a) the first antibody comprises a variable heavy chain domain (VH) according to SEQ ID NO: 32 (<VH Ang2>) and a variable light chain domain (VL) according to SEQ ID NO: 33 (<VL Ang2>); and
   b) the second antibody comprises a variable heavy chain domain (VH) according to SEQ ID NO: 31 (<VH VEGF>) and a variable light chain domain (VL) according to SEQ ID NO: 30 (<VL VEGF>).
36. A bispecific antibody specifically binding to human Angiopoietin-2 and human VEGF, comprising
   a) a first light chain and a first heavy chain derived from a first antibody which specifically binds to human Angiopoietin-2, which in one preferred embodiment comprises a variable heavy chain domain according to SEQ ID NO: 32 and a variable light chain domain according to SEQ ID NO: 33; and
   b) a second light chain and a second heavy chain derived from a second antibody which specifically binds to human VEGF, which in one preferred embodiment comprises a variable heavy chain domain according to SEQ ID NO: 31 and a variable light chain domain according to SEQ ID NO: 30, wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E); or
      ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
37. A bispecific antibody specifically binding to human Angiopoietin-2 and human VEGF, comprising
   a) a first light chain and a first heavy chain derived from a first antibody which specifically binds to human Angiopoietin-2, which in one preferred embodiment comprises a variable heavy chain domain according to SEQ ID NO: 32 and a variable light chain domain according to SEQ ID NO: 33; and
   b) a second light chain and a second heavy chain derived from a second antibody which specifically binds to human VEGF, which in one preferred embodiment comprises a variable heavy chain domain according to SEQ ID NO: 31 and a variable light chain domain according to SEQ ID NO: 30, wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
38. A bispecific antibody specifically binding to human Angiopoietin-2 and human VEGF, comprising
   a) a first light chain and a first heavy chain derived from a first antibody which specifically binds to human Angiopoietin-2, which in one preferred embodiment comprises a variable heavy chain domain according to SEQ ID NO: 32 and a variable light chain domain according to SEQ ID NO: 33; and
   b) a second light chain and a second heavy chain derived from a second antibody which specifically binds to human VEGF, which in one preferred embodiment comprises a variable heavy chain domain according to SEQ ID NO: 31 and a variable light chain domain according to SEQ ID NO: 30, wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
39. The bispecific antibody specifically binding to human Angiopoietin-2 and human VEGF according to embodiment 36,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the second heavy chain the amino acid at position 124 (numbering according to Kabat) is substituted by an amino acid selected from E and D (in one preferred embodiment by E); or
   ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the first light chain the amino acid at position 124 (numbering according to Kabat) is substituted by an amino acid selected from E and D (in one preferred embodiment by E).
40. The bispecific antibody specifically binding to human Angiopoietin-2 and human VEGF according to embodiment 37,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the second heavy chain the amino acid at position 124 (numbering according to Kabat) is substituted by an amino acid selected from E and D (in one preferred embodiment by E).
41. The bispecific antibody specifically binding to human Angiopoietin-2 and human VEGF according to embodiment 38,
   ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the first light chain the amino acid at position 124 (numbering according to Kabat) is substituted by an amino acid selected from E and D (in one preferred embodiment by E).
42. The bispecific antibody specifically binding to human Angiopoietin-2 and human VEGF according to any one of embodiments 36 to 41,
   - wherein in the variable domain VL of the first light chain the amino acid at position 38 (numbering according to Kabat) is substituted by an amino acid selected from K, R and H (in one preferred embodiment independently from each other by K or R; in one further preferred embodiment by K); and wherein in the variable domain VH of the first heavy chain the amino acid at position 39 (numbering according to Kabat) is substituted by an amino acid selected from E and D (in one preferred embodiment by E); and
   - wherein in the variable domain VL of the second heavy chain the amino acid at position 38 (numbering according to Kabat) is substituted by an amino acid selected from E and D (in one preferred embodiment E); and
      wherein in the variable domain VH of the second light chain the amino acid at position 39 (numbering according to Kabat) is substituted by an amino acid selected from K, R and H (in one preferred embodiment by K or R, in one embodiment by K).
43. The multispecific antibody according to any one of embodiments 1 to 33 that specifically binds to human TWEAK and human IL17, wherein
   a) the antibody comprises a variable heavy chain domain (VH) according to SEQ ID NO: 36 (<VH IL17>) and a variable light chain domain (VL) according to SEQ ID NO: 37 (<VL IL17>); and
   b) the antibody comprises a variable heavy chain domain (VH) according to SEQ ID NO: 35 (<VH TWEAK>) and a variable light chain domain (VL) according to SEQ ID NO: 34 (<VL TWEAK >).
44. The multispecific antibody according to any one of embodiments 1 to 33 or 43 that specifically binds to human TWEAK and human IL17, wherein
   a) the first antibody comprises a variable heavy chain domain (VH) according to SEQ ID NO: 36 (<VH IL17>) and a variable light chain domain (VL) according SEQ ID NO: 37 (<VL IL17>); and
   b) the second antibody comprises a variable heavy chain domain (VH) according to SEQ ID NO: 35 (<VH TWEAK>) and a variable light chain domain (VL) according to SEQ ID NO: 34 (<VL TWEAK >).
45. A bispecific antibody specifically binding to human TWEAK and human IL17, comprising
   a) a first light chain and a first heavy chain derived from a first antibody which specifically binds to human IL17, which in one preferred embodiment comprises a variable heavy chain domain according to SEQ ID NO: 35 and a variable light chain domain according to SEQ ID NO: 34; and
   b) a second light chain and a second heavy chain derived from a second antibody which specifically binds to human TWEAK, which in one preferred embodiment comprises a variable heavy chain domain according to SEQ ID NO: 36 and a variable light chain domain according to SEQ ID NO: 37, wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E); or
      ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
46. A bispecific antibody specifically binding to human TWEAK and human IL17, comprising
   a) a first light chain and a first heavy chain derived from a first antibody which specifically binds to human IL17, which in one preferred embodiment comprises a variable heavy chain domain according to SEQ ID NO: 35 and a variable light chain domain according to SEQ ID NO: 34; and
   b) a second light chain and a second heavy chain derived from a second antibody which specifically binds to human TWEAK, which in one preferred embodiment comprises a variable heavy chain domain according to SEQ ID NO: 36 and a variable light chain domain according to SEQ ID NO: 37, wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
47. A bispecific antibody specifically binding to human TWEAK and human IL17, comprising
   a) a first light chain and a first heavy chain derived from a first antibody which specifically binds to human IL17, which in one preferred embodiment comprises a variable heavy chain domain according to SEQ ID NO: 35 and a variable light chain domain according to SEQ ID NO: 34; and
   b) a second light chain and a second heavy chain derived from a second antibody which specifically binds to human TWEAK, which in one preferred embodiment comprises a variable heavy chain domain according to SEQ ID NO: 36 and a variable light chain domain according to SEQ ID NO: 37, wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
48. The bispecific antibody specifically binding to human TWEAK and human IL17 according to embodiment 45,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the second heavy chain the amino acid at position 124 (numbering according to Kabat) is substituted by an amino acid selected from E and D (in one preferred embodiment by E); or
   ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the first light chain the amino acid at position 124 (numbering according to Kabat) is substituted by an amino acid selected from E and D (in one preferred embodiment by E).
49. The bispecific antibody specifically binding to human TWEAK and human IL17 according to embodiment 46,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the second heavy chain the amino acid at position 124 (numbering according to Kabat) is substituted by an amino acid selected from E and D (in one preferred embodiment by E).
50. The bispecific antibody specifically binding to human TWEAK and human IL17 according to embodiment 47,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the first light chain the amino acid at position 124 (numbering according to Kabat) is substituted by an amino acid selected from E and D (in one preferred embodiment by E).
51. The bispecific antibody specifically binding to human TWEAK and human IL17 according to any one of embodiments 45 to 50,
   - wherein in the variable domain VL of the first light chain the amino acid at position 38 (numbering according to Kabat) is substituted by an amino acid selected from K, R and H (in one preferred embodiment independently from each other by K or R; in one further preferred embodiment by K); and wherein in the variable domain VH of the first heavy chain the amino acid at position 39 (numbering according to Kabat) is substituted by an amino acid selected from E and D (in one preferred embodiment by E); and
   - wherein in the variable domain VL of the second heavy chain the amino acid at position 38 (numbering according to Kabat) is substituted by an amino acid selected from E and D (in one preferred embodiment E); and
      wherein in the variable domain VH of the second light chain the amino acid at position 39 (numbering according to Kabat) is substituted by an amino acid selected from K, R and H (in one preferred embodiment by K or R, in one embodiment by K).
52. The multispecific antibody according to any one of embodiments 1 to 33 that specifically binds to human Her1, human Her3 and human cMet, wherein
   a) the antibody comprises a variable heavy chain domain (VH) according to SEQ ID NO: 40 (<VH Her1Her3>) and a variable light chain domain (VL) according to SEQ ID NO: 41 (<VL Her1Her3>); and
   b) the antibody comprises a variable heavy chain domain (VH) according to SEQ ID NO: 39 (<VH cMet>) and a variable light chain domain (VL) according to SEQ ID NO: 38 (<VL cMet>).
53. The multispecific antibody according to any one of embodiments 1 to 33 or 52 that specifically binds to human Her1, human Her3 and human cMet, wherein
   a) the first antibody comprises a variable heavy chain domain (VH) according to SEQ ID NO: 40 (<VH Her1Her3>) and a variable light chain domain (VL) according to SEQ ID NO: 41 (<VL Her1Her3>); and
   b) the second antibody comprises a variable heavy chain domain (VH) according to SEQ ID NO: 39 (<VH cMet>) and a variable light chain domain (VL) according to SEQ ID NO: 38 (<VL cMet>).
54. A trispecific antibody specifically binding to human Her1, human Her3 and human cMet, comprising
   a) a first light chain and a first heavy chain derived from a first antibody which specifically binds to human Her1 and human Her3, which in one preferred embodiment comprises a variable heavy chain domain according to SEQ ID NO: 40 and a variable light chain domain according to SEQ ID NO: 41; and
   b) a second light chain and a second heavy chain derived from a second antibody which specifically binds to human cMet, which in one preferred embodiment comprises a variable heavy chain domain according to SEQ ID NO: 39 and a variable light chain domain according to SEQ ID NO: 38,
      wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E); or
      ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
55. A trispecific antibody specifically binding to human Her1, human Her3 and human cMet, comprising
   a) a first light chain and a first heavy chain derived from a first antibody which specifically binds to human Her1 and human Her3, which in one preferred embodiment comprises a variable heavy chain domain according to SEQ ID NO: 40 and a variable light chain domain according to SEQ ID NO: 41; and
   b) a second light chain and a second heavy chain derived from a second antibody which specifically binds to human cMet, which in one preferred embodiment comprises a variable heavy chain domain according to SEQ ID NO: 39 and a variable light chain domain according to SEQ ID NO: 38,
      wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
56. A trispecific antibody specifically binding to human Her1, human Her3 and human cMet, comprising
   a) a first light chain and a first heavy chain derived from a first antibody which specifically binds to human Her1 and human Her3, which in one preferred embodiment comprises a variable heavy chain domain according to SEQ ID NO: 40 and a variable light chain domain according to SEQ ID NO: 41; and
   b) a second light chain and a second heavy chain derived from a second antibody which specifically binds to human cMet, which in one preferred embodiment comprises a variable heavy chain domain according to SEQ ID NO: 39 and a variable light chain domain according to SEQ ID NO: 38,
      wherein in the second light chain the variable domain VL is replaced by the variable domain VH of the second heavy chain and the constant domain CL is replaced by the constant domain CH1 of the second heavy chain; and
      in the second heavy chain the variable domain VH is replaced by the variable domain VL of the second light chain and the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
57. The trispecific antibody specifically binding to human Her1, human Her3 and human cMet according to embodiment 54,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the second heavy chain the amino acid at position 124 (numbering according to Kabat) is substituted by an amino acid selected from E and D (in one preferred embodiment by E); or
   ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the first light chain the amino acid at position 124 (numbering according to Kabat) is substituted by an amino acid selected from E and D (in one preferred embodiment by E).
58. The trispecific antibody specifically binding to human Her1, human Her3 and human cMet according to embodiment 55,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the second heavy chain the amino acid at position 124 (numbering according to Kabat) is substituted by an amino acid selected from E and D (in one preferred embodiment by E).
59. The trispecific antibody specifically binding to human Her1, human Her3 and human cMet according to embodiment 56,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the first light chain the amino acid at position 124 (numbering according to Kabat) is substituted by an amino acid selected from E and D (in one preferred embodiment by E).
60. The trispecific antibody specifically binding to human Her1, human Her3 and human cMet according to any one of embodiments 54 or 59,
   - wherein in the variable domain VL of the first light chain the amino acid at position 38 (numbering according to Kabat) is substituted by an amino acid selected from K, R and H (in one preferred embodiment independently from each other by K or R; in one further preferred embodiment by K); and
      wherein in the variable domain VH of the first heavy chain the amino acid at position 39 (numbering according to Kabat) is substituted by an amino acid selected from E and D (in one preferred embodiment by E); and
   - wherein in the variable domain VL of the second heavy chain the amino acid at position 38 (numbering according to Kabat) is substituted by an amino acid selected from E and D (in one preferred embodiment E); and
      wherein in the variable domain VH of the second light chain the amino acid at position 39 (numbering according to Kabat) is substituted by an amino acid selected from K, R and H (in one preferred embodiment by K or R, in one embodiment by K).
61. The antibody according to any one of the preceding embodiments, for use as a medicament.
62. The antibody according to any one of embodiments 1 to 60, for use in the treatment of cancer.
63. The antibody according to any one of embodiments 1 to 60, for use in the treatment of inflammatory diseases, autoimmune diseases, rheumatoid arthritis, psoratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.
64. Use of the antibody according to any one of embodiments 1 to 60 for the manufacture of a medicament, in one preferred embodiment for the treatment of cancer or for the treatment of inflammatory diseases, autoimmune diseases, rheumatoid arthritis, psoratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.
65. A method for the preparation of a multispecific antibody according to any one of embodiments 1 to 60, comprising the steps of
   - transforming a host cell with vectors comprising nucleic acids encoding
      a) the first light chain as defined in one of embodiments 1 to 60 derived from a first antibody which specifically binds to a first antigen;
      b) the first heavy chain as defined in one of embodiments 1 to 60 derived from a first antibody which specifically binds to a first antigen;
      c) the second light chain as defined in one of embodiments 1 to 60 derived from a second antibody which specifically binds to a second antigen; and
      d) the second heavy chain as defined in one of embodiments 1 to 60 derived from a second antibody which specifically binds to a second antigen,
   - culturing said host cell under conditions that allow synthesis of said multispecific antibody; and
   - recovering said multispecific antibody from said host cell culture.
66. A method for the reduction of side product formation during the preparation of a multispecific antibody, in one preferred embodiment according to any one of the preceding embodiments, comprising the steps of
   - transforming a host cell with vectors comprising nucleic acids encoding
      a) a first light chain and a first heavy chain derived from a first antibody which specifically binds to a first antigen; and
      b) a second light chain and a second heavy chain derived from a second antibody which specifically binds to a second antigen, wherein in the second light chain the variable domain VL is replaced by the variable domain VH of the second heavy chain and the constant domain CL is replaced by the constant domain CH1 of the second heavy chain; and in the second heavy chain the variable domain VH is replaced by the variable domain VL of the second light chain and the constant domain CH1 is replaced by the constant domain CL of the second light chain;
      and wherein in order to reduce the formation of side product of the multispecific antibody:
      i) in the constant domain CL of the first light chain the amino acids at position 124 and 123 (numbering according to Kabat) are substituted independently from each other by an amino acid selected from K, R and H; and in the constant domain CH1 of the first heavy chain the amino acids at position 147 and 213 (numbering according to EU index of Kabat) are substituted independently from each other by an amino acid selected from E or D; or
      ii) in the constant domain CL of the second heavy chain the amino acids at position 124 and 123 (numbering according to Kabat) are substituted independently from each other by an amino acid selected from K, R and H; and in the constant domain CH1 of the second light chain the amino acids at position 147 and 213 (numbering according to EU index of Kabat) are substituted independently from each other by an amino acid selected from E or D;
   - culturing said host cell under conditions that allow synthesis of said multispecific antibody; and
   - recovering said multispecific antibody from said host cell culture.
67. The method according to embodiment 66, wherein wherein in order to reduce the formation of side product of the multispecific antibody:
   i) in the constant domain CL of the first light chain the amino acids at position 124 and 123 (numbering according to Kabat) are substituted independently from each other by an amino acid selected from K, R and H; and in the constant domain CH1 of the first heavy chain the amino acids at position 147 and 213 (numbering according to EU index of Kabat) are substituted independently from each other by an amino acid selected from E or D.
68. The method according to embodiment 66, wherein wherein in order to reduce the formation of side product of the multispecific antibody:
   i) in the constant domain CL of the second heavy chain the amino acids at position 124 and 123 (numbering according to Kabat) are substituted independently from each other by an amino acid selected from K, R and H; and in the constant domain CH1 of the second light chain the amino acids at position 147 and 213 (numbering according to EU index of Kabat) are substituted independently from each other by an amino acid selected from E or D.
69. The method according to embodiment 66, wherein in order to reduce the formation of side product of the multispecific antibody
   i) in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and in the constant domain CH1 of the first heavy chain the amino acids at position 147 and 213 (numbering according to EU index of Kabat) are substituted independently from each other by an amino acid selected from E or D (in one preferred embodiment by E), and in the constant domain CL of the second heavy chain the amino acid at position 124 (numbering according to Kabat) is substituted by an amino acid selected from E and D (in one preferred embodiment by E); or
   ii) in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and in the constant domain CH1 of the first heavy chain the amino acids at position 147 and 213 (numbering according to EU index of Kabat) are substituted independently from each other by an amino acid selected from E or D (in one preferred embodiment by E), and in the constant domain CL of the first light chain the amino acid at position 124 (numbering according to Kabat) is substituted by an amino acid selected from E and D (in one preferred embodiment by E).
70. The method according to embodiment 67, wherein in order to reduce the formation of side product of the multispecific antibody
   i) in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and in the constant domain CH1 of the first heavy chain the amino acids at position 147 and 213 (numbering according to EU index of Kabat) are substituted independently from each other by an amino acid selected from E or D (in one preferred embodiment by E), and in the constant domain CL of the second heavy chain the amino acid at position 124 (numbering according to Kabat) is substituted by an amino acid selected from E and D (in one preferred embodiment by E).
71. The method according to embodiment 68, wherein in order to reduce the formation of side product of the multispecific antibody
   i) in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and in the constant domain CH1 of the first heavy chain the amino acids at position 147 and 213 (numbering according to EU index of Kabat) are substituted independently from each other by an amino acid selected from E or D (in one preferred embodiment by E), and in the constant domain CL of the first light chain the amino acid at position 124 (numbering according to Kabat) is substituted by an amino acid selected from E and D (in one preferred embodiment by E).
72. The method according to any one of embodiments 66 to 71, wherein in order to reduce the formation of side product of the multispecific antibody the following amino acid substitutions are included
   - in the variable domain VL of the first light chain the amino acid at position 38 (numbering according to Kabat) is substituted by an amino acid selected from K, R and H (in one preferred embodiment independently from each other by K or R; in one further preferred embodiment by K); and in the variable domain VH of the first heavy chain the amino acid at position 39 (numbering according to Kabat) is substituted by an amino acid selected from E and D (in one preferred embodiment by E); and
   - wherein in the variable domain VL of the second heavy chain the amino acid at position 38 (numbering according to Kabat) is substituted by an amino acid selected from E and D (in one preferred embodiment E); and
      wherein in the variable domain VH of the second light chain the amino acid at position 39 (numbering according to Kabat) is substituted by an amino acid selected from K, R and H (in one preferred embodiment by K or R, in one embodiment by K).
73. A multispecific antibody produced by a method according to embodiment 65.
74. A nucleic acid encoding the amino acid sequence of
   a) a first light chain as defined in one of embodiments 1 to 60,
   b) a second light chain as defined in one of embodiments 1 to 60,
   c) a first heavy chain as defined in one of embodiments 1 to 60; or
   d) a second heavy chain as defined in one of embodiments 1 to 60.
75. A vector comprising a nucleic acid according to embodiment 74, wherein the vector is capable of expressing said nucleic acid in a host cell.
76. An expression vector comprising a nucleic acid according to embodiment 74, wherein the vector is capable of expressing said nucleic acid in a host cell.
77. A host cell comprising a nucleic acid according to embodiment 74.
78. A host cell comprising a vector according to embodiment 75 or an expression vector according to embodiment 76.
79. A pharmaceutical composition comprising a multispecific antibody according to any one of embodiments 1 to 60 in combination with at least one pharmaceutically acceptable carrier.
80. The pharmaceutical composition according to embodiment 79, for use as a medicament.
81. The pharmaceutical composition according to embodiment 79, for use in the treatment of cancer.
82. The pharmaceutical composition according to embodiment 79, for use in the treatment of inflammatory diseases, autoimmune diseases, rheumatoid arthritis, psoratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

**DESCRIPTION OF THE AMINO ACID SEQUENCES**

| | |
|---|---|
| **SEQ ID NO:1** | heavy chain (HC) <VEGF> with VL-VH/CL-CH1 domain exchange wild type (wt) |
| **SEQ ID NO:2** | light chain (LC) <VEGF> with VL-VH/CL-CH1 domain exchange wild type (wt) |
| **SEQ ID NO:3** | heavy chain (HC) <Ang-2> wild type (wt) |
| **SEQ ID NO:4** | kappa light chain (LC) <Ang-2> wild type (wt) |
| **SEQ ID NO:5** | heavy chain (HC) <Ang-2> with K213E and K147E substitutions |
| **SEQ ID NO:6** | kappa light chain (LC) <Ang-2> with E123K and Q124K substitutions |
| **SEQ ID NO:7** | heavy chain (HC) <VEGF> with VL-VH/CL-CH1 domain exchange with Q124E substitution |
| **SEQ ID NO:8** | heavy chain (HC) <VEGF> with VL-VH/CL-CH1 domain exchange with Q124E and Q38E substitutions |
| **SEQ ID NO:9** | light chain (LC) <VEGF> with VL-VH/CL-CH1 domain exchange with Q39K substitution |
| **SEQ ID NO:10** | heavy chain (HC) <Ang-2> with K213E, K147E and Q39E substitutions |
| **SEQ ID NO:11** | kappa light chain (LC) <Ang-2> with E123K, Q124K and Q38K substitutions |
| **SEQ ID NO:12** | lambda light chain (LC) <Ang-2> wild type (wt) |
| **SEQ ID NO:13** | lambda light chain (LC) <Ang-2> with E123K and E124K substitutions |
| **SEQ ID NO:14** | heavy chain (HC) <TWEAK> with VL-VH/CL-CH1 domain exchange wild type (wt) |
| **SEQ ID NO:15** | light chain (LC) <TWEAK> with VL-VH/CL-CH1 domain exchange wild type (wt) |
| **SEQ ID NO:16** | heavy chain (HC) <IL17> wild type (wt) |
| **SEQ ID NO:17** | kappa light chain (LC) <IL17> wild type (wt) |
| **SEQ ID NO:18** | heavy chain (HC) <IL17> with K213E and K147E substitutions |
| **SEQ ID NO:19** | kappa light chain (LC) <IL17> with E123K and Q124K substitutions |
| **SEQ ID NO:20** | heavy chain (HC) <TWEAK> with VL-VH/CL-CH1 domain exchange with Q124E substitution |
| **SEQ ID NO:21** | heavy chain (HC) <cMet> with VL-VH/CL-CH1 domain exchange wild type (wt) |
| **SEQ ID NO:22** | light chain (LC) <cMet> with VL-VH/CL-CH1 domain exchange wild type (wt) |
| **SEQ ID NO:23** | heavy chain (HC) <Her1Her3> wild type (wt) |
| **SEQ ID NO:24** | kappa light chain (LC) <Her1Her3> wild type (wt) |
| **SEQ ID NO:25** | heavy chain (HC) <cMet> with VL-VH/CL-CH1 domain exchange with Q124E substitution |
| **SEQ ID NO:26** | heavy chain (HC) <Her1Her3> with K213E and K147E substitutions |
| **SEQ ID NO:27** | kappa light chain (LC) <Her1Her3> E123K and Q124K substitutions |
| **SEQ ID NO:28** | heavy chain (HC) <Her1Her3> with K213D and K147E substitutions |
| **SEQ ID NO:29** | kappa light chain (LC) <Her1Her3> E123R and Q124K substitutions |
| **SEQ ID NO:30** | variable heavy chain domain <VEGF> 289 |
| **SEQ ID NO:31** | variable light chain domain <VEGF> 289 |
| **SEQ ID NO:32** | variable heavy chain domain <Ang-2> 289 |
| **SEQ ID NO:33** | variable light chain domain <Ang-2> 289 |
| **SEQ ID NO:34** | variable heavy chain domain <TWEAK> |
| **SEQ ID NO:35** | variable light chain domain <TWEAK> |
| **SEQ ID NO:36** | variable heavy chain domain <IL17> |
| **SEQ ID NO:37** | variable light chain domain <IL17> |
| **SEQ ID NO:38** | variable heavy chain domain <cMet> |
| **SEQ ID NO:39** | variable light chain domain <cMet> |
| **SEQ ID NO:40** | variable heavy chain domain <Her1Her3> |
| **SEQ ID NO:41** | variable light chain domain <Her1Her3> |
| **SEQ ID NO:42** | heavy chain (HC) <TWEAK> with VL-VH/CL-CH1 domain exchange with Q38E and Q124E substitution |
| **SEQ ID NO:43** | light chain (LC) <TWEAK> with VL-VH/CL-CH1 domain exchange with Q39K substitution |
| **SEQ ID NO:44** | heavy chain (HC) <IL17> with K213E, K147E and and Q39E substitutions |
| **SEQ ID NO:45** | kappa light chain (LC) <IL17> with E123K, Q124K and Q38K substitutions |
| **SEQ ID NO:46** | heavy chain (HC) <VEGF> with VL-VH/CL-CH1 domain exchange with Q124E substitution with wild type CH3 |
| **SEQ ID NO:47** | heavy chain (HC) <Ang-2> with K231E, K147E substitution with wild type CH3 |
| **SEQ ID NO:48** | heavy chain (HC) <VEGF> with VL-VH/CL-CH1 domain exchange with wild type CH3 |
| **SEQ ID NO:49** | heavy chain (HC) <VEGF> with VL-VH/CL-CH1 domain exchange with E123K, Q124K substitution |
| **SEQ ID NO:50** | light chain (LC) <VEGF> with VL-VH/CL-CH1 domain exchange with K213E, K147E substitution |
| **SEQ ID NO:51** | kappa light chain (LC) <Ang-2> with Q124E substitution |

### EXAMPLES

The following examples are provided to aid the understanding of the present invention, the true scope of which is set forth in the appended claims.

In the following section, if not stated otherwise, LC* denotes the modified light light chain <CH1-VH> and HC* denotes the modified heavy chain <CL-VL> or <CH3-CH2-CL-VL>.

### Materials & general methods

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to numbering according to Kabat (Kabat, E.A., et al., Sequences of Proteins of Immunological Interest, 5th ed., Public Health Service, National Institutes of Health, Bethesda, MD (1991)).

### Recombinant DNA techniques

Standard methods were used to manipulate DNA as described in Sambrook, J. et al., Molecular Cloning: A laboratory manual; Cold Spring Harbor Laboratory Press, Cold Spring Harbor, New York, 1989. The molecular biological reagents were used according to the manufacturer's instructions.

### Gene synthesis

Desired gene segments were prepared from oligonucleotides made by chemical synthesis. The 600 - 1800 bp long gene segments, which were flanked by singular restriction endonuclease cleavage sites, were assembled by annealing and ligating oligonucleotides including PCR amplification and subsequently cloned via the indicated restriction sites e.g. KpnI/SacI or AscI/PacI into a pPCRScript (Stratagene) based pGA4 cloning vector. The DNA sequences of the subcloned gene fragments were confirmed by DNA sequencing. Gene synthesis fragments were ordered according to given specifications at Geneart (Regensburg, Germany).

### DNA sequence determination

DNA sequences were determined by double strand sequencing performed at MediGenomix GmbH (Martinsried, Germany) or Sequiserve GmbH (Vaterstetten, Germany).

### DNA and protein sequence analysis and sequence data management

The GCG's (Genetics Computer Group, Madison, Wisconsin) software package version 10.2 and Infomax's Vector NT1 Advance suite version 8.0 was used for sequence creation, mapping, analysis, annotation and illustration.

### Expression vectors

For the expression of the described antibodies, variants of expression plasmids for transient expression (e.g. in HEK293 EBNA or HEK293-F) cells based either on a cDNA organization with or without a CMV-Intron A promoter or on a genomic organization with a CMV promoter were applied.

Beside the antibody expression cassette the vectors contained:
- an origin of replication which allows replication of this plasmid in *E. coli,* and
- a β-lactamase gene which confers ampicillin resistance in *E. coli.*

The transcription unit of the antibody gene was composed of the following elements:
- unique restriction site(s) at the 5' end
- the immediate early enhancer and promoter from the human cytomegalovirus,
- followed by the Intron A sequence in the case of the cDNA organization,
- a 5'-untranslated region of a human antibody gene,
- an immunoglobulin heavy chain signal sequence,
- the human antibody chain (wildtype or with domain exchange) either as cDNA or as genomic organization with the immunoglobulin exon-intron organization
- a 3' untranslated region with a polyadenylation signal sequence, and
- unique restriction site(s) at the 3' end.

The fusion genes comprising the antibody chains as described below were generated by PCR and/or gene synthesis and assembled by known recombinant methods and techniques by connection of the according nucleic acid segments e.g. using unique restriction sites in the respective vectors. The subcloned nucleic acid sequences were verified by DNA sequencing. For transient transfections larger quantities of the plasmids were prepared by plasmid preparation from transformed *E. coli* cultures (Nucleobond AX, Macherey-Nagel).

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

Multispecific antibodies were expressed by transient co-transfection of the respective expression plasmids in adherently growing HEK293-EBNA or in HEK29-F cells growing in suspension as described below.

### Transient transfections in HEK293-EBNA system

Multispecific antibodies were expressed by transient co-transfection of the respective expression plasmids (e.g. encoding the heavy and modified heavy chain, as well as the corresponding light and modified light chain) in adherently growing HEK293-EBNA cells (human embryonic kidney cell line 293 expressing Epstein-Barr-Virus nuclear antigen; American type culture collection deposit number ATCC # CRL-10852, Lot. 959 218) cultivated in DMEM (Dulbecco's modified Eagle's medium, Gibco®) supplemented with 10% Ultra Low IgG FCS (fetal calf serum, Gibco®), 2 mM L-Glutamine (Gibco®), and 250 µg/ml Geneticin (Gibco®). For transfection FuGENE™ 6 Transfection Reagent (Roche Molecular Biochemicals) was used in a ratio of FuGENE™ reagent (µl) to DNA (µg) of 4:1 (ranging from 3:1 to 6:1). Proteins were expressed from the respective plasmids using an equimolar ratio of (modified and wildtype) light chain and (modified and wildtype) heavy chain encoding plasmids. Cells were fed at day 3 with L-Glutamine ad 4 mM, Glucose [Sigma] and NAA [Gibco®]. Multispecific antibody containing cell culture supernatants were harvested from day 5 to 11 after transfection by centrifugation and stored at -20°C. General information regarding the recombinant expression of human immunoglobulins in e.g. HEK293 cells is given in: Meissner, P. et al., Biotechnol. Bioeng. 75 (2001) 197-203.

### Transient transfections in HEK293-F system

Multispecific antibodies were generated by transient transfection with the respective plasmids (e.g. encoding the heavy and modified heavy chain, as well as the corresponding light and modified light chain) using the HEK293-F system (Invitrogen) according to the manufacturer's instruction. Briefly, HEK293-F cells (Invitrogen) growing in suspension either in a shake flask or in a stirred fermenter in serum-free FreeStyle™ 293 expression medium (Invitrogen) were transfected with a mix of the four expression plasmids and 293fectin™ or fectin (Invitrogen). For 2 L shake flask (Corning) HEK293-F cells were seeded at a density of 1.0E^{∗}6 cells/mL in 600 mL and incubated at 120 rpm, 8% CO2. The day after the cells were transfected at a cell density of ca. 1.5E^{∗}6 cells/mL with ca. 42 mL mix of A) 20 mL Opti-MEM (Invitrogen) with 600 µg total plasmid DNA (1 µg/mL) encoding the heavy or modified heavy chain, respectively and the corresponding light or modified light chain in an equimolar ratio and B) 20 ml Opti-MEM + 1.2 mL 293 fectin or fectin (2 µl/mL). According to the glucose consumption glucose solution was added during the course of the fermentation. The supernatant containing the secreted antibody was harvested after 5-10 days and antibodies were either directly purified from the supernatant or the supernatant was frozen and stored.

### Protein determination

The protein concentration of purified antibodies and derivatives was determined by determining the optical density (OD) at 280 nm, using the molar extinction coefficient calculated on the basis of the amino acid sequence according to Pace, et al., Protein Science, 1995, 4, 2411-1423.

### Antibody concentration determination in supernatants

The concentration of antibodies and derivatives in cell culture supernatants was estimated by immunoprecipitation with Protein A Agarose-beads (Roche). 60 µL Protein A Agarose beads were washed three times in TBS-NP40 (50 mM Tris, pH 7.5, 150 mM NaCl, 1% Nonidet-P40). Subsequently, 1-15 mL cell culture supernatant were applied to the Protein A Agarose beads pre-equilibrated in TBS-NP40. After incubation for at 1 hour at room temperature the beads were washed on an Ultrafree-MC-filter column (Amicon) once with 0.5 mL TBS-NP40, twice with 0.5 mL 2x phosphate buffered saline (2xPBS, Roche) and briefly four times with 0.5 mL 100 mM Na-citrate pH 5,0. Bound antibody was eluted by addition of 35 µl NuPAGE® LDS Sample Buffer (Invitrogen). Half of the sample was combined with NuPAGE® Sample Reducing Agent or left unreduced, respectively, and heated for 10 min at 70°C. Consequently, 5-30 µl were applied to a 4-12% NuPAGE® Bis-Tris SDS-PAGE (Invitrogen) (with MOPS buffer for non-reduced SDS-PAGE and MES buffer with NuPAGE® Antioxidant running buffer additive (Invitrogen) for reduced SDS-PAGE) and stained with Coomassie Blue.

The concentration of antibodies and derivatives in cell culture supernatants was quantitatively measured by affinity HPLC chromatography. Briefly, cell culture supernatants containing antibodies and derivatives that bind to Protein A were applied to an Applied Biosystems Poros A/20 column in 200 mM KH2PO4, 100 mM sodium citrate, pH 7.4 and eluted from the matrix with 200 mM NaCl, 100 mM citric acid, pH 2,5 on an Agilent HPLC 1100 system. The eluted protein was quantified by UV absorbance and integration of peak areas. A purified standard IgG1 antibody served as a standard.

Alternatively, the concentration of antibodies and derivatives in cell culture supernatants was measured by Sandwich-IgG-ELISA. Briefly, StreptaWell High Bind Strepatavidin A-96 well microtiter plates (Roche) are coated with 100 µL/well biotinylated anti-human IgG capture molecule F(ab')2<h-Fcy> BI (Dianova) at 0.1 µg/mL for 1 hour at room temperature or alternatively overnight at 4°C and subsequently washed three times with 200 µL/well PBS, 0.05% Tween (PBST, Sigma). 100 µL/well of a dilution series in PBS (Sigma) of the respective antibody containing cell culture supernatants was added to the wells and incubated for 1-2 hour on a microtiterplate shaker at room temperature. The wells were washed three times with 200 µL/well PBST and bound antibody was detected with 100 µl F(ab')2<hFcγ>POD (Dianova) at 0.1 µg/mL as the detection antibody for 1-2 hours on a microtiterplate shaker at room temperature. Unbound detection antibody was washed away three times with 200 µL/well PBST and the bound detection antibody was detected by addition of 100 µL ABTS/well. Determination of absorbance was performed on a Tecan Fluor Spectrometer at a measurement wavelength of 405 nm (reference wavelength 492 nm).

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

### SDS-PAGE

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

### CE-SDS

Purity and antibody integrity were analyzed by CE-SDS using microfluidic Labchip technology (PerkinElmer, USA). 5 µl of protein solution was prepared for CE-SDS analysis using the HT Protein Express Reagent Kit according manufacturers instructions and analysed on LabChip GXII system using a HT Protein Express Chip. Data were analyzed using LabChip GX Software.

### Analytical size exclusion chromatography

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM KH2PO4/K2HPO4, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

### Mass spectrometry

This section describes the characterization of the multispecific antibodies with VL-VH/CL-CH1 domain exchange (*CrossMAb^{Fab}*) with emphasis on their correct assembly. The expected primary structures were analyzed by electrospray ionization mass spectrometry (ESI-MS) of the deglycosylated intact CrossMAbs and in special cases of the deglycosylated/limited LysC digested CrossMabs.

The CrossMab^{Fab}s were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated CrossMab^{Fab}s in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Determination of binding and binding affinity of multispecific antibodies to the respective antigens using surface plasmon resonance (SPR) (BIACORE)

### VEGF binding was assessed according to the following procedure:

Binding of indicated antibodies to human VEGFA-121 was investigated by surface plasmon resonance using a BIACORE® T200 instrument (GE Healthcare). Around 10000 (RU) of anti His antibody (1 µg/ml anti His antibody; Order Code: 28995056; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

VEFGA-121-His was captured by injecting a 0.5 µg/ml solution for 30 sec at a flow of 5 µl/min. The association was measured by injection of the indicated antibodies in various concentrations in solution for 180 sec at a flow of 30 µl/min starting with 1000 nM in 1:3 serial dilutions. The dissociation phase was monitored for up to 600 sec and triggered by switching from the sample solution to running buffer. The surface was regenerated by 60 sec washing with a Glycine pH 1.5 solution at a flow rate of 30 µl/min. Bulk refractive index differences were corrected by subtracting the response obtained from a anti His antibody surface. Blank injections are also subtracted (= double referencing). For calculation of K_{D} and other kinetic parameters the Langmuir 1:1 model was used.

### Ang-2 binding was assessed according to the following procedure:

Binding of indicated antibodies to human Ang-2-RBD-Fc was investigated by surface plasmon resonance using a BIACORE® T200 instrument (GE Healthcare). Around 8000 (RU) of goat anti human F(ab')₂ (10 µg/ml anti human F(ab)'₂; Order Code: 28958325; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

The bispecific antibody was captured by injecting a 5 nM solution for 25 sec at a flow of 5 µl/min. The association was measured by injection of human Ang2-RBD-Fc in various concentrations in solution for 120 sec at a flow of 30 µl/min starting with 100 nM in 1:3 serial dilutions. The dissociation phase was monitored for up to 180 sec and triggered by switching from the sample solution to running buffer. The surface was regenerated by 60 sec washing with a Glycine pH 2.1 solution at a flow rate of 30 µl/min. Bulk refractive index differences were corrected by subtracting the response obtained from a goat anti human F(ab')₂ surface. Blank injections are also subtracted (= double referencing). For calculation of apparent K_{D} the Langmuir 1:1 model was used.

### IL-17 binding was assessed according to the following procedure:

Around 6000 resonance units (RU) of the capturing system (15 µg/ml goat anti human F(ab')2; Order Code: 28958325; GE Healthcare Bio-Sciences AB, Schweden) were coupled on a CM5 chip at pH 5.0 by using an amine coupling kit supplied by the GE Healthcare. The sample and system buffer was PBS-T (10 mM phosphate buffered saline including 0.05% Tween 20) pH 7.4. The bispecific antibody was captured by injecting a 50 nM solution for 90 sec at a flow of 10 µl/min. Association was measured by injection human IL17 in various concentrations in solution for 3 min at a flow of 30 µl/min starting with 50 nM in 1:1 serial dilutions. The dissociation phase was monitored for up to 10 min and triggered by switching from the sample solution to running buffer. The surface was regenerated twice by 60 sec wash with a 10 mM glycine pH 2.1 solution at a flow rate of 30 µl/min. Bulk refractive index differences were corrected by subtracting the response obtained from a goat anti human F(ab')2 surface. Blank injections are also subtracted (= double referencing). For calculation of apparent K_{D} and other kinetic parameters, the Langmuir 1:1 model was used.

### TWEAK binding was assessed according to the following procedure:

Due to strong unspecific binding of the TWEAK analyte to the sensor surface, a reverse setup - using TWEAK as ligand - was chosen. Around 100 RU of TWEAK was immobilized on the C1 chip surface at pH 5.0 using an amine coupling kit supplied by the GE Healthcare. The sample and system buffer was PBS-T (10 mM phosphate buffered saline including 0.05% Tween 20) pH 7.4. Association was measured by injection the bispecific antibody in various concentrations in solution for 3 min at a flow of 30 µl/min starting with 50 nM in 1:1 serial dilutions. The dissociation phase was monitored for up to 10 min and triggered by switching from the sample solution to running buffer. The surface was regenerated three times by 30 sec wash with a 3 M MgCl2 solution at a flow rate of 30 µl/min. Bulk refractive index differences were corrected by subtracting the response obtained from a blank-coupled surface. Blank injections are also subtracted (= double referencing). For calculation of K_{D} and other kinetic parameters, the Langmuir 1:1 model was used.

### Example 1A

### Production and expression of bivalent, bispecific antibodies which bind to Angiopoietin-2 (ANG2) and Vascular endothelial growth factor (VEGF), with VL-VH/CL-CH1 domain exchange (CrossMAb^{Fab}) in one binding arm and with two charged amino acid substitutions in the CH1/CL interface

In a first example bispecific antibodies which bind to human Angiopoietin-2 (ANG2) and human Vascular endothelial growth factor (VEGF) were generated as described in the general methods section by classical molecular biology techniques and expressed transiently in HEK293 cells as described above.

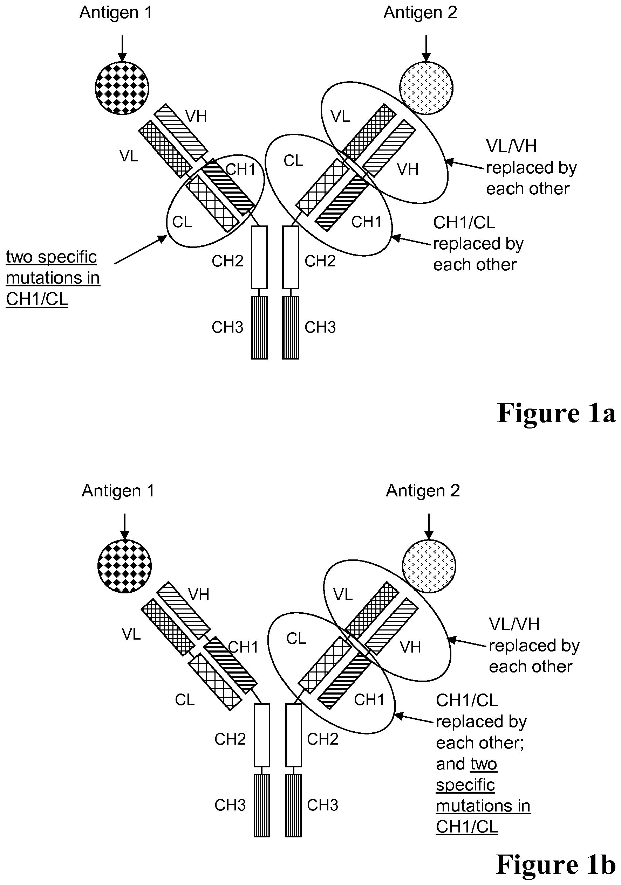

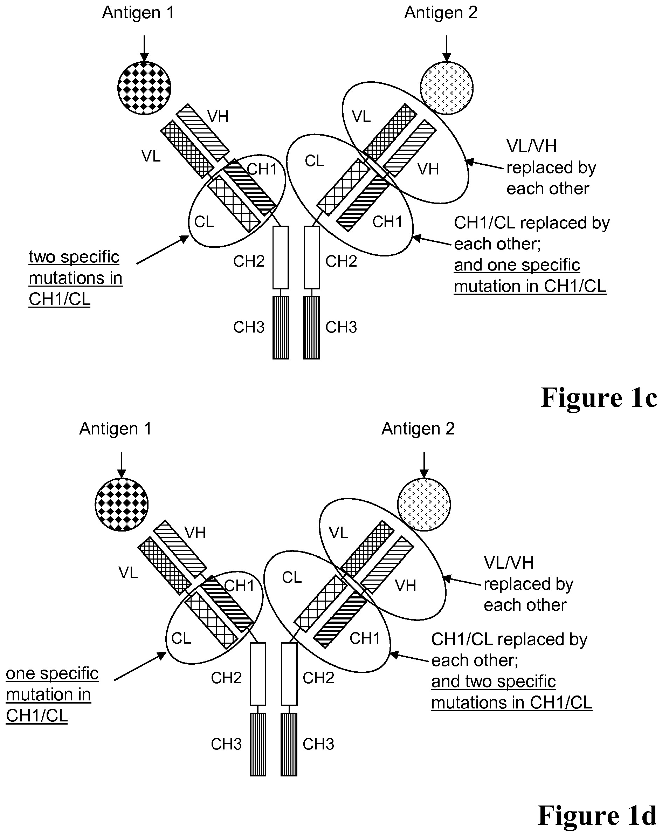

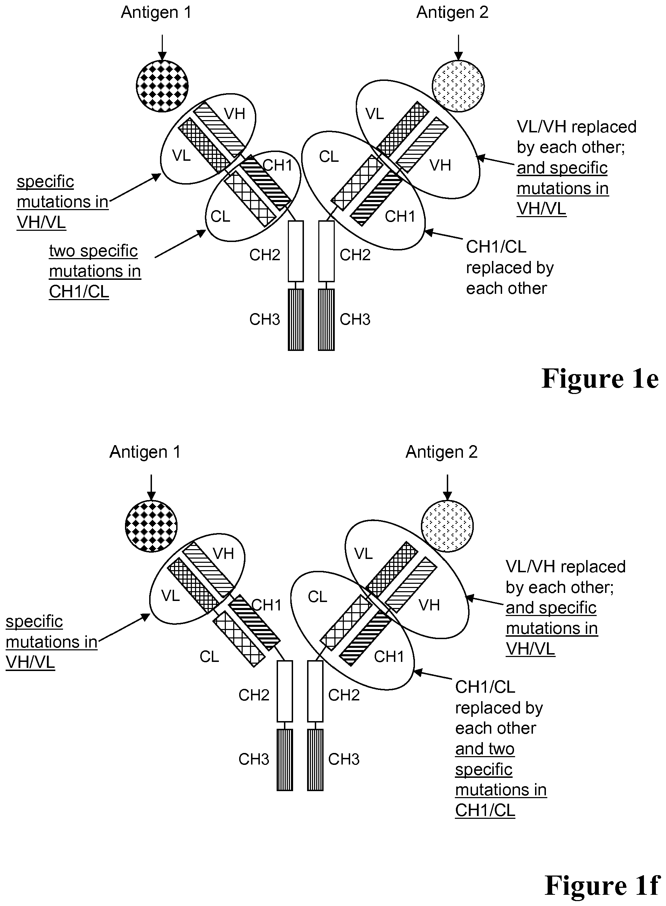

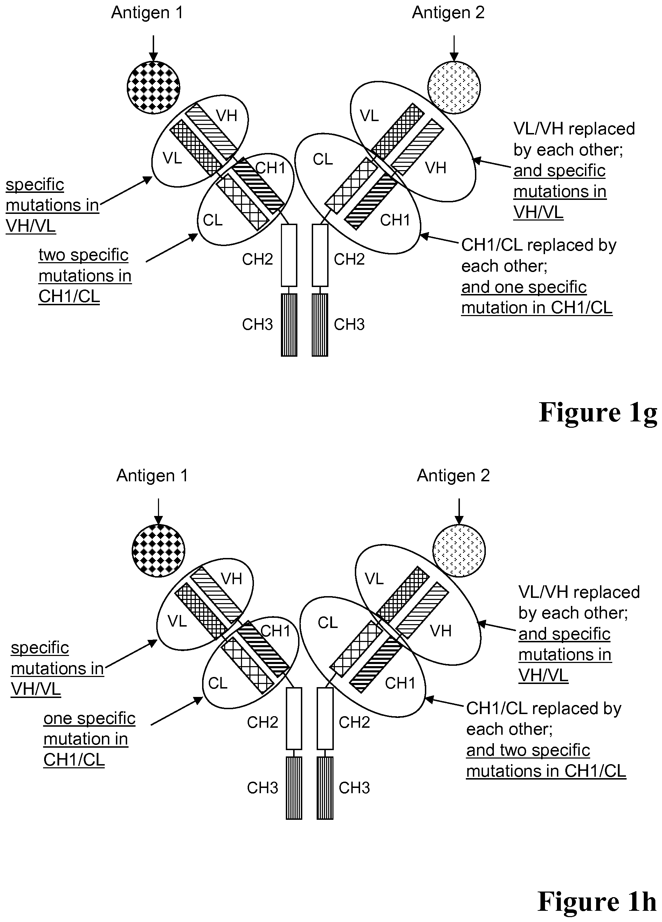

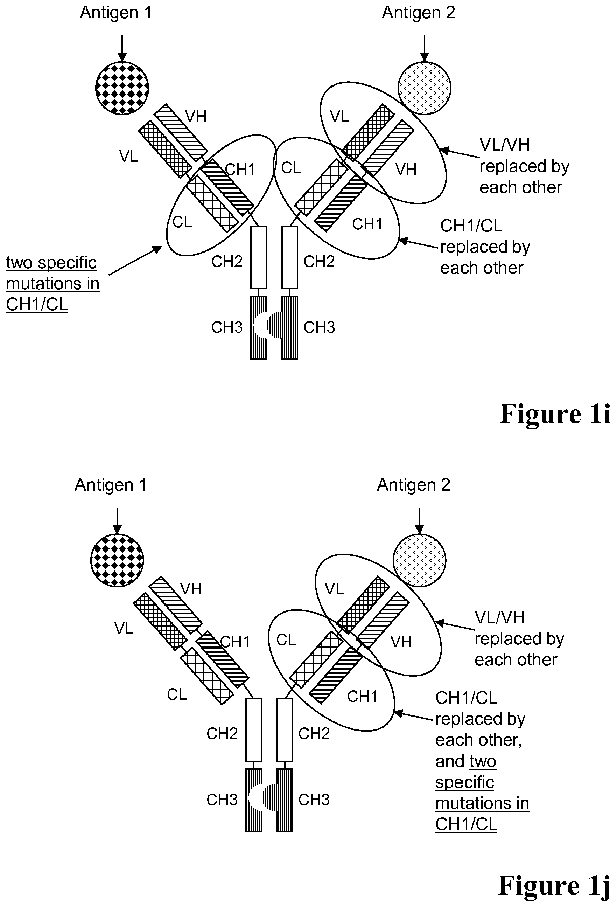

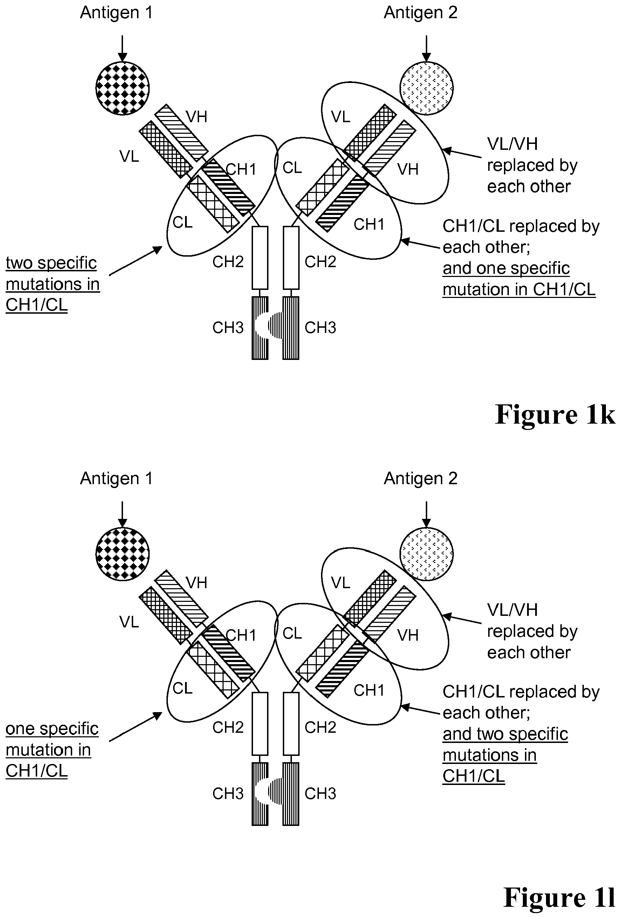

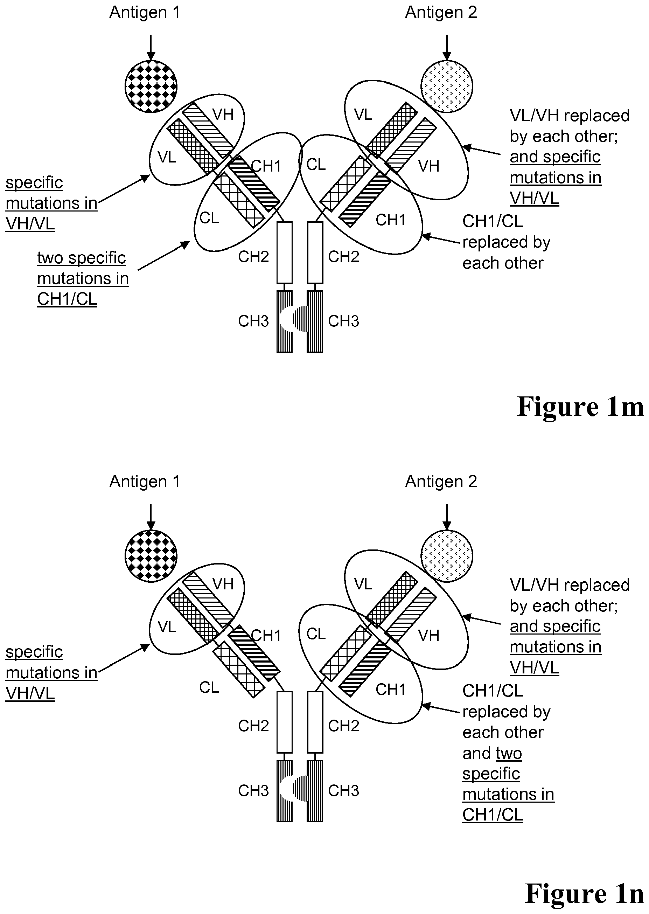

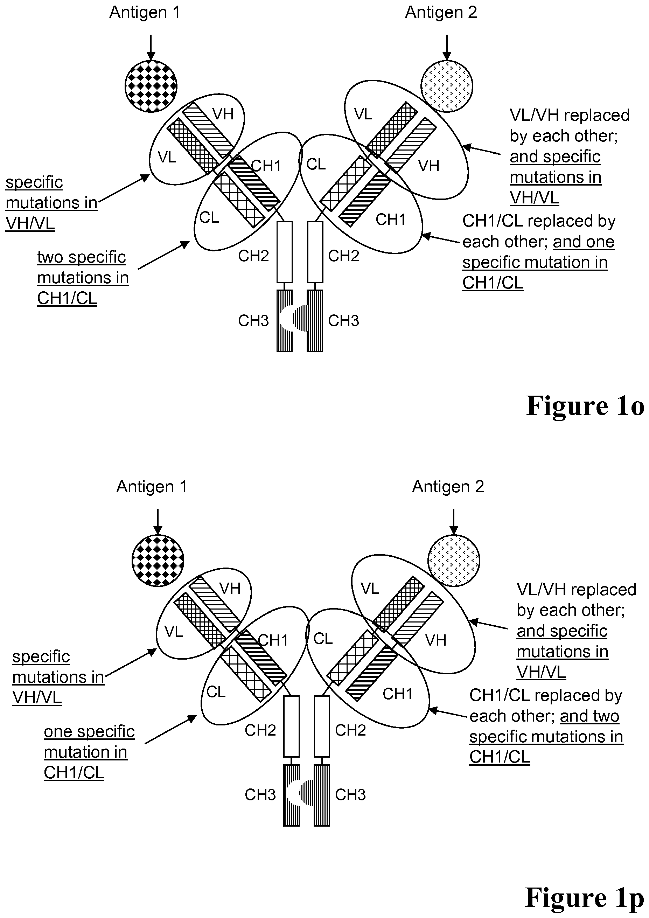

A general scheme of these respective bispecific antibodies is given in Figure 1. For comparative analyses the wild type (wt) VL-VH/CL-CH1 domain exchange antibodies without charged amino acid substitutions in the CH1/CL interface were prepared. The bispecific antibodies were expressed using expression plasmids containing the nucleic acids encoding the amino acid sequences as shown in Table **1.**

**Table 1: Amino acid sequences of light chains (LC) and heavy chains (HC) of anti-Ang2-VEGF bispecific antibodies Ang2VEGF-0289, Ang2VEGF-280, Ang2VEGF-111, Ang2VEGF_290 and Ang2VEGF_279 with VL-VH/CL-CH1 domain exchange (CrossMAbFab): wild type (wt) and different combinations of substitutions with charged amino acids**

| **Antibody** | **VEGF_HC*** | **VEGF_LC*** | **Ang2_HC** | **Ang2_LC** |
|---|---|---|---|---|
| Ang2VEGF_289 | SEQ ID NO: 1 | SEQ ID NO: 2 | SEQ ID NO: 3 | SEQ ID NO: 4 |
| Ang2VEGF_280 | SEQ ID NO: 1 | SEQ ID NO: 2 | SEQ ID NO: 5 | SEQ ID NO: 6 |
| Ang2VEGF_111 | SEQ ID NO: 7 | SEQ ID NO: 2 | SEQ ID NO: 5 | SEQ ID NO: 6 |
| Ang2VEGF_290^{†} | SEQ ID NO: 1 | SEQ ID NO: 2 | SEQ ID NO: 3 | SEQ ID NO: 12 |
| Ang2VEGF_279^{†} | SEQ ID NO: 7 | SEQ ID NO: 2 | SEQ ID NO: 5 | SEQ ID NO: 13 |

| | | | | |
|---|---|---|---|---|
| ^{†}... Ang2VEGF_290 and Ang2VEGF_279 include Ang2-specific antibody with lambda light chain, whereas all other listed anti-Ang2-VEGF bispecific antibodies include a kappa light chain | | | | |

For all constructs knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349'C) (contained in the respective corresponding heavy chain (HC) sequences depicted above).

### Example 1B

### Purification and characterization of bivalent, bispecific antibodies which bind to ANG2 and VEGF, with VL-VH/CL-CH1 domain exchange (CrossMAbFab) in one binding arm and with two charged amino acid substitutions in the CH1/CL interface

The bispecific antibodies expressed above in example 1A were purified from the supernatant by a combination of Protein A affinity chromatography and size exclusion chromatography. All bispecific antibodies can be produced in good yields and are stable. The obtained products were characterized for identity by mass spectrometry and analytical properties such as purity by CE-SDS, monomer content and stability.

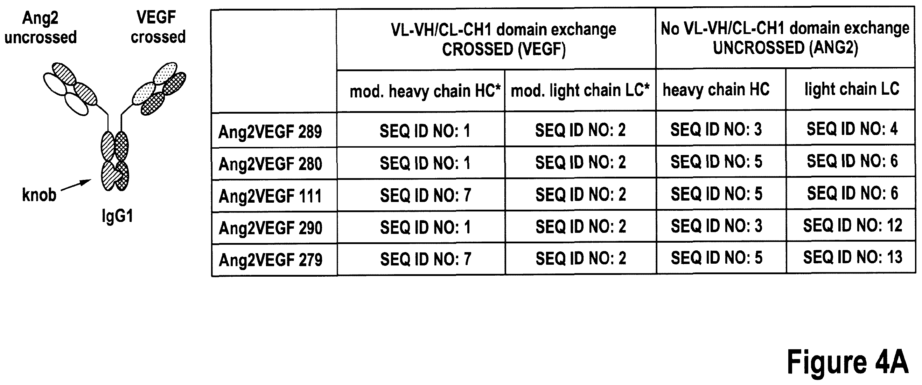

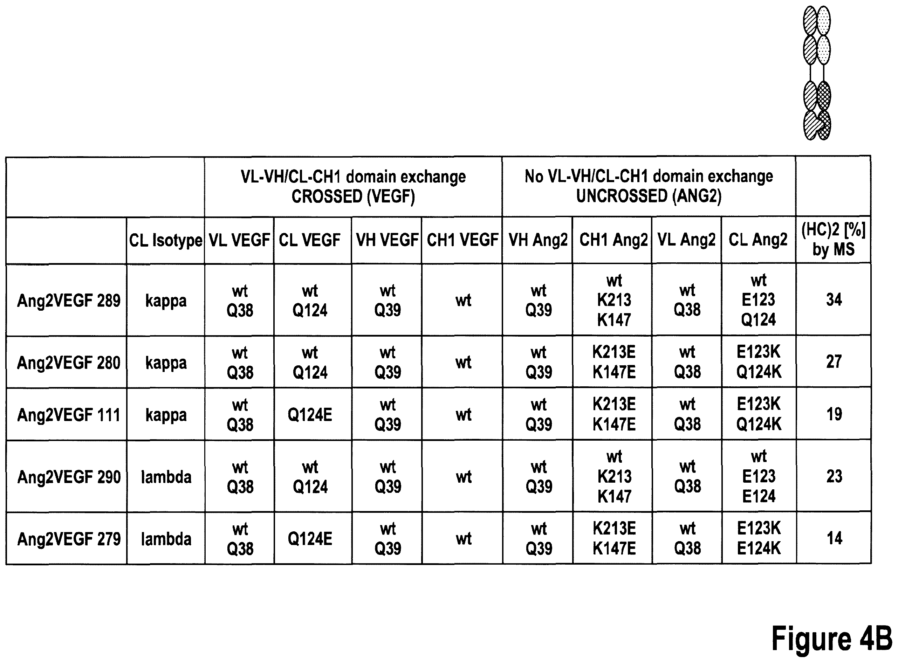

The expected primary structures were analyzed by electrospray ionization mass spectrometry (ESI-MS) of the deglycosylated intact CrossMAbs and deglycosylated/plasmin digested or alternatively deglycosylated/limited LysC digested CrossMAbs as described in the general methods section.
Results are shown in Table 2a and Figure 4.

**Table 2a: Reduction of non functional heavy chain dimer (HC)₂ formation as side product by charged amino acid substitutions in the CH1/CL interface**

| **Antibody** | **VEGF_HC*** | **VEGF_LC*** | **Ang2_HC** | **Ang2_LC** | **(HC)₂ [%] by MS^{‡}** |
|---|---|---|---|---|---|
| Ang2VEGF_289 | wt | wt | wt | wt | 34 |
| Ang2VEGF_280 | wt | wt | K213E | E123K | 27 |
| | | | K147E | Q124K | |
| Ang2VEGF_111 | Q124E | wt | K213E | E123K | 19 |
| | | | K147E | Q124K | |
| Ang2VEGF_290^{†} | wt | wt | wt | wt | 23 |
| Ang2VEGF_279^{†} | Q124E | wt | K213E | E123K | 14 |
| | | | K147E | E124K | |

| | | | | | |
|---|---|---|---|---|---|
| ^{†}... Ang2VEGF_290 and Ang2VEGF_279 include Ang2-specific antibody with lambda light chain, whereas all other listed anti-Ang2-VEGF bispecific antibodies include a kappa light chain. ^{‡}... after Protein A chromatography. | | | | | |

The repition of the described experiment is summarized in Table 2b:

**Table 2b: Reduction of non functional heavy chain dimer (HC)₂ formation as side product by charged amino acid substitutions in the CH1/CL interface (repetition)**

| **Antibody** | **Purity by CE-SDS [%]*** | **(HC)₂ [%] by CE-SDS*** | **(HC)₂ [%] by CE-SDS^{#}** | **(HC)₂[%] by MS^{#}** |
|---|---|---|---|---|
| Ang2VEGF_289 | 58 | - | - | 12* |
| Ang2VEGF_280 | 62 | - | 6 | 3 |
| Ang2VEGF_111 | 92 | - | - | 3* |
| Ang2VEGF_290^{†} | 53 | 43 | 9 | - |
| Ang2VEGF_279^{†} | 86 | 10 | 2 | - |

| | | | | |
|---|---|---|---|---|
| ^{†}... Ang2VEGF_290 and Ang2VEGF_279 include Ang2-specific antibody with lambda light chain, whereas all other listed anti-Ang2-VEGF bispecific antibodies include a kappa light chain; * after Protein A purification; # after Protein A and SEC purification. | | | | |

Results in Tables 2a and b and Figure 4 show that by substituting two charged amino acids with the opposite charge in the CH1 domain (K213E, K147E) as well as in the CL domain (E123K, Q124K) in the "uncrossed" side, as it is described for the invention in further detail above, formation of the main side product (a non functional heavy chain dimer) is strongly reduced as compared to the wild type bispecific antibody without such substitutions. Experiments demonstrate that the the charge substitution reduces the side product formation when carried out in kappa light chains (tested antibodies Ang2VEGF-0289 [wt], Ang2VEGF-280, Ang2VEGF-111) as well as in lambda light chains (tested antibodies Ang2VEGF_290 [wt] and Ang2VEGF_279).

By introducing an additional charged amino acid in the CL domain in the "crossed" side the formation of the side product can be further reduced (tested antibodies Ang2VEGF-0289 [wt], Ang2VEGF-280 [without additional charge in "crossed" side], Ang2VEGF-111[with additional charge in "crossed" side]).

### Antigen binding:

Binding of the multispecific antibodies to their respective target antigens, i.e. ANG2 and VEGF, was assessed by Biacore® as described in the general methods section.

As comparative example, a reference antibody specifically binding to Ang2 and VEGF comprising a VH/VL domain exchange/replacement but lacking charged amino acid substitutions (Ang2VEGF-0290 antibody) was assessed in parallel.

Affinity measurements were conducted with the purified material from the repition experiments summarized in **Table 2b.**

Results are indicated in **Tables 2c and 2d.**

**Table 2c: Affinity for VEGF of indicated antibodies**

| **Sample** | **KD (nM)** |
|---|---|
| Ang2VEGF-0280 | 7 |
| Ang2VEGF-0290 (wt) | 8 |
| Ang2VEGF-0279 | 6 |

**Table 2d: Affinity for Ang2 of indicated antibodies**

| **Sample** | **App.KD (nM)** |
|---|---|
| Ang2VEGF-0280 | 3 |
| Ang2VEGF-0290 (wt) | 3 |
| Ang2VEGF-0279 | 5 |

The tested antibodies specifically bind to both targets, Ang2 and VEGF, and exhibit an antigen affinity in the nanomolar range.

### Stability:

In order to assess stability of the antibody constructs, the aggregation onset temperatures was assessed. Thermal stability measurements were conducted with the purified material from the repition experiments summarized in **Table 2b.**

Results are shown in **Table 2e.**

**Table 2e: Aggregation onset temperature (T_{agg}) of indicated antibodies**

| **Sample** | **T_{agg} (°C)** |
|---|---|
| Ang2VEGF-0280 | 61.0 |
| Ang2VEGF-0290 (wt) | 62.0 |
| Ang2VEGF-0279 | 61.0 |

### Example 1C

### Production and expression of bivalent, bispecific antibodies which bind to TNF-related weak inducer of apoptosis (TWEAK) and Interleukin-17 (IL17), with VL-VH/CL-CH1 domain exchange (CrossMAb^{Fab}) in one binding arm and with two charged amino acid substitutions in the CH1/CL interface

In a second example bispecific antibodies which bind to human TNF-related weak inducer of apoptosis (TWEAK) and human Interleukin-17 (IL17) were generated as described in the general methods section by classical molecular biology techniques and expressed transiently in HEK293 cells as described above.

A general scheme of these respective bispecific antibodies is given in Figure 1a. For comparative analyses the wild type (wt) VL-VH/CL-CH1 domain exchange antibodies without charged amino acid substitutions in the CH1/CL interface were prepared. The bispecific antibodies were expressed using expression plasmids containing the nucleic acids encoding the amino acid sequences as shown in Table 3.

**Table 3: Amino acid sequences of light chains (LC) and heavy chains (HC) of anti-Tweak-IL17 bispecific antibodies TweakIL17_101, TweakIL17_102, TweakIL17_103 with VL-VH/CL-CH1 domain exchange (CrossMAb^{Fab}): wild type (wt) and different combinations of substitutions with charged amino acids**

| **Antibody** | **TWEAK_HC*** | **TWEAK_LC*** | **IL17_HC** | **IL-17_LC** |
|---|---|---|---|---|
| TweakIL17_101 | SEQ ID NO: 14 | SEQ ID NO: 15 | SEQ ID NO: 16 | SEQ ID NO: 17 |
| TweakIL17_102 | SEQ ID NO: 14 | SEQ ID NO: 15 | SEQ ID NO: 18 | SEQ ID NO: 19 |
| TweakIL17_103 | SEQ ID NO: 20 | SEQ ID NO: 15 | SEQ ID NO: 18 | SEQ ID NO: 19 |
| TweakIL17-0128^{*} | SEQ ID NO: 42 | SEQ ID NO: 43 | SEQ ID NO: 44 | SEQ ID NO: 45 |

| | | | | |
|---|---|---|---|---|
| *^{*} only data available from repetition experiment* | | | | |

For all constructs knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349'C) (contained in the respective corresponding heavy chain (HC) sequences depicted above).

### Example 1D

### Purification and characterization of bivalent, bispecific antibodies which bind to TWEAK and IL17, with VL-VH/CL-CH1 domain exchange (CrossMAb^{Fab}) in one binding arm and with two charged amino acid substitutions in the CH1/CL interface

The bispecific antibodies expressed above in example 1C were purified from the supernatant by a combination of Protein A affinity chromatography and size exclusion chromatography. All bispecific antibodies can be produced in good yields and are stable. The obtained products were characterized for identity by mass spectrometry and analytical properties such as purity by CE-SDS, monomer content and thermal stability.

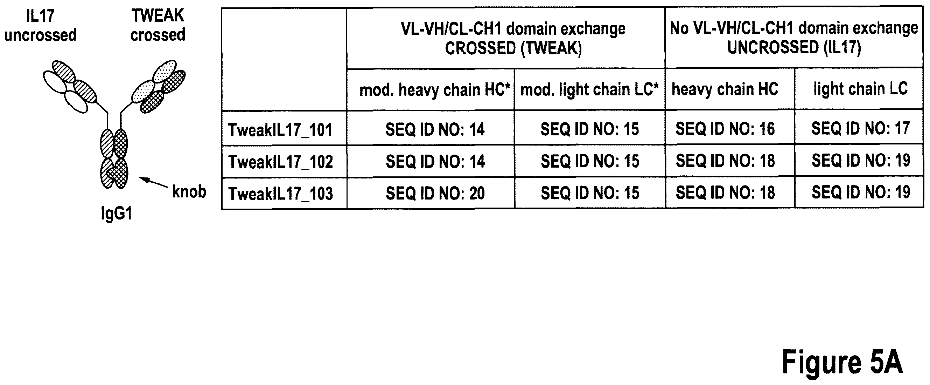

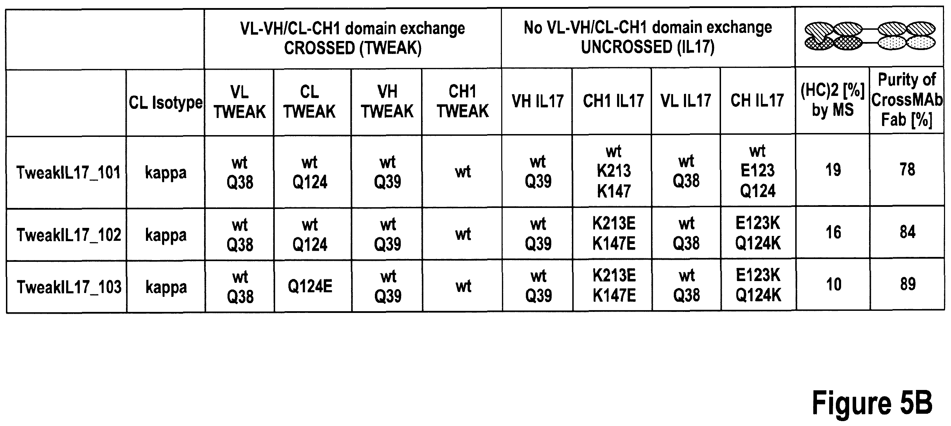

The expected primary structures were analyzed by electrospray ionization mass spectrometry (ESI-MS) of the deglycosylated intact CrossMAbs and in various cases of the deglycosylated/limited LysC digested CrossMAbs as described in the general methods section.
Results are shown in Table 4a and Figure 5.

**Table 4a: Reduction of side product formation by charged amino acid substitutions in the CH1/CL interface after Protein A chromatography**

| **Antibody** | **TWEAK_HC*** | **TWEAK_LC*** | **IL17_HC** | **IL17_LC** | **(HC)₂ [%] by MS** | **Purity by CE-SDS [%]** |
|---|---|---|---|---|---|---|
| TweakIL17_101 | wt | wt | wt | wt | 19 | 78 |
| TweakIL17_102 | wt | wt | K213E | E123K | 16 | 84 |
| | | | K147E | Q124K | | |
| TweakIL17_103 | Q124E | wt | K213E | E123K | 10 | 89 |
| | | | K147E | Q124K | | |

The data from the repetition of the example experiment are summarized in **Table 4b.**

**Table 4b: Reduction of side product formation by charged amino acid substitutions in the CH1/CL interface after Protein A chromatography**

| **Antibody** | **TWEAK_HC*** | **TWEAK_LC*** | **IL17_HC** | **IL17_LC** | **(HC)₂ [%] by CE-SDS** | **Purity by CE-SDS [%]*** |
|---|---|---|---|---|---|---|
| TweakIL17_101 | wt | wt | wt | wt | 25 | 72 |
| TweakIL17_102 | wt | wt | K213E | E123K | 12 | 84 |
| | | | K147E | Q124K | | |
| TweakIL17_103 | Q124E | wt | K213E | E123K | 16 | 78 |
| | | | K147E | Q124K | | |
| TweakIL17_0128 | Q38E | Q39K | Q39E | Q38K | 0 | 76 |
| | | | K213E | E123K | | |
| | | | K147E | Q124K | | |

| | | | | | | |
|---|---|---|---|---|---|---|
| ^{*} *purity of desired molecule by CE-SDS (main peak content)* | | | | | | |

**Table 4c: Reduction of side product formation by charged amino acid substitutions in the CH1/CL interface after Protein A and preparative size exclusion chromatography**

| **Antibody** | **Monomer [%] by SEC** | **(HC)₂ [%] by CE-SDS** |
|---|---|---|
| TweakIL17_101 | 98.2 | <1 |
| TweakIL17_102 | 99.4 | <1 |
| TweakIL17_0128 | >99 | 0 |

| | | |
|---|---|---|
| ^{*} *purity by CE-SDS (main peak content)* | | |

Results in Tables 4a, 4b and 4c and Figure 5 show that by substituting two charged amino acids with the opposite charge in the CH1 domain (K213E, K147E) as well as in the CL domain (E123K, Q124K) in the "uncrossed" side, as it is described for the invention in further detail above, formation of the main side product (a non functional heavy chain dimer) is strongly reduced as compared to the wild type bispecific antibody without such substitutions. By introducing an additional charged amino acid in the CL domain (kappa chain) in the "crossed" side the formation of the main side product (a non functional heavy chain dimer) can be further reduced. Remaining side products, i.e. the non functional heavy chain dimer, can be easily separated from the desired molecule via size exclusion chromatography.

### Antigen binding:

Binding of the multispecific antibodies to their respective target antigens, i.e. TWEAK and IL-17, was measured by Surface Plasmon Resonance (SPR) using a BIAcore® T200 instrument (GE Healthcare) at 25°C as described in the general methods section.

As comparative example, a reference antibody specifically binding to TWEAK and IL-17 comprising a VH/VL domain exchange/replacement but lacking charged amino acid substitutions (TweakIL17_101 antibody) was assessed in parallel.

Affinity measurements were conducted with the purified material from the repition experiments summarized in **Tables 4d and 4e.**

**Table 4d: Affinity for IL-17 of indicated antibodies**

| **Sample** | **KD (nM)** |
|---|---|
| TweakIL17_101 (wt) | 0.1 |
| TweakIL17_102 | 0.1 |
| TweakIL17_103 | 0.1 |
| TweakIL17_128 | 0.1 |

**Table 4e: Affinity for Tweak of indicated antibodies**

| **Sample** | **App.KD (nM)** |
|---|---|
| TweakIL17_101 (wt) | < 0.1 |
| TweakIL17_102 | < 0.1 |
| TweakIL17_103 | < 0.1 |
| TweakIL17_128 | <0.1 |

The tested antibodies specifically bind to both targets, TWEAK and IL-17, in a comparable manner as the corresponding antibody lacking the amino acid substitions.

### Example 1E

### Production and expression of trispecific antibodies which bind to Herl and Her3 (combined in a dual acting Fab [DAF] format) as well as Met proto-oncogene (cMet), with VL-VH/CL-CH1 domain exchange (CrossDAF^{Fab}) in one binding arm and with charged amino acid substitutions in the CH1/CL interface

In a third example trispecific antibodies which bind to ErbB family members Her1 and Her3 as well as Met proto-oncogene (cMet) were generated as described in the general methods section by classical molecular biology techniques and expressed transiently in HEK293 cells as described above. In this construct the specificities against Her1 and Her3 are combined in a so-called dual-acting-Fab (DAF) format as disclosed in WO2013/174873.

A general scheme of these respective multispecific antibodies is given in Figure 1. For comparative analyses the wild type (wt) VL-VH/CL-CH1 domain exchange trispecific antibodies without charged amino acid substitutions in the CH1/CL interface were prepared. The multispecific antibodies were expressed using expression plasmids containing the nucleic acids encoding the amino acid sequences as shown in Table 5.

**Table 5: Amino acid sequences of light chains (LC) and heavy chains (HC) of anti-[HerlHer3]-cMet trispecific antibodies MetHERl(3)DAF_0002, MetHERl(3)DAF_0001, MetHERl(3)DAF_0003 with VL-VH/CL-CH1 domain exchange (CrossDAF^{Fab}): wild type (wt) and different combinations of substitutions with charged amino acids**

| **Antibody** | **cMet_HC*** | **cMet_LC*** | **Her_HC** | **Her_LC** |
|---|---|---|---|---|
| MetHER1(3)DAF_0002 | SEQ ID NO: 21 | SEQ ID NO: 22 | SEQ ID NO: 23 | SEQ ID NO: 24 |
| MetHER1(3)DAF_0001 | SEQ ID NO: 25 | SEQ ID NO: 22 | SEQ ID NO: 26 | SEQ ID NO: 27 |
| MetHER1(3)DAF_0003 | SEQ ID NO: 25 | SEQ ID NO: 22 | SEQ ID NO: 28 | SEQ ID NO: 29 |

For all constructs knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349C) (contained in the respective corresponding heavy chain (HC) sequences depicted above).

### Example 1F

### Purification and characterization of trispecific antibodies which bind to HerlHer3 and cMet, with VL-VH/CL-CH1 domain exchange (CrossDAF^{Fab}) in one binding arm and with charged amino acid substitutions in the CH1/CL interface

The multispecific antibodies expressed above in example IE were purified from the supernatant by a combination of Protein A affinity chromatography and size exclusion chromatography. All multispecific antibodies can be produced in good yields and are stable. The obtained products were characterized for identity by mass spectrometry and analytical properties such as purity by SDS-PAGE, monomer content and stability.

The expected primary structures were analyzed by electrospray ionization mass spectrometry (ESI-MS) of the deglycosylated intact CrossDAFs and in special cases of the deglycosylated/limited LysC digested CrossDAFs as described in the general methods section.

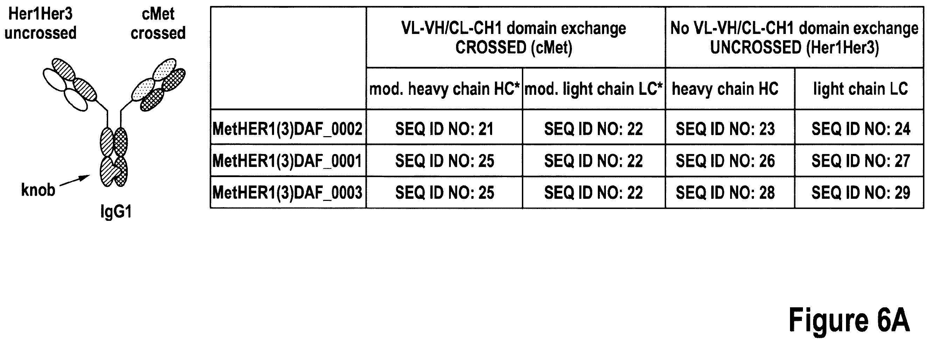

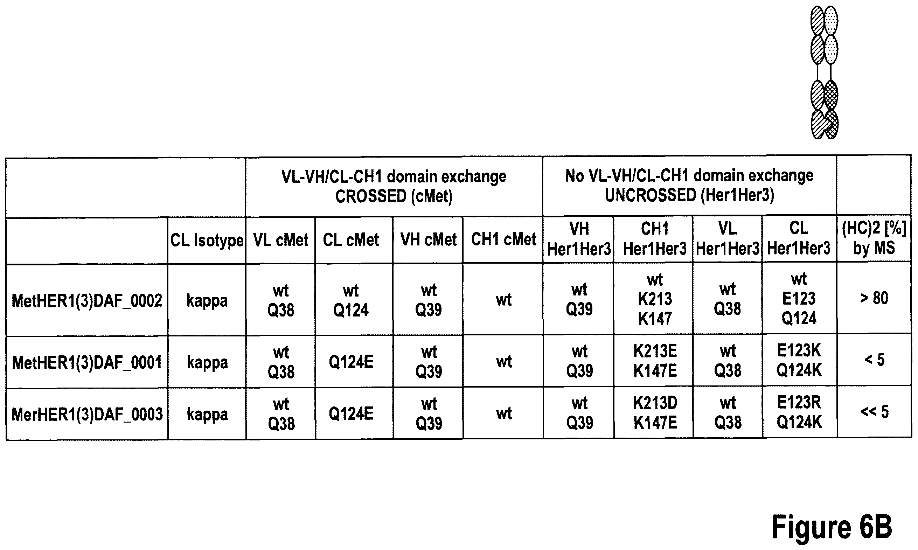

Results are shown in Table 6 and Figure 6.

**Table 6: Reduction of non functional heavy chain dimer (HC)₂ formation as side product by charged amino acid substitutions in the CH1/CL interface**

| **Antibody** | **cMet_HC*** | **cMet_LC*** | **Her_HC** | **Her_LC** | **(HC)₂ [%] by MS** |
|---|---|---|---|---|---|
| MetHERl(3)DAF_0002 | wt | wt | wt | wt | > 80 |
| MetHERl(3)DAF_0001 | Q124E | wt | K213E | E123K | <5 |
| | | | K147E | Q124K | |
| MetHERl(3)DAF_0003 | Q124E | wt | K213D | E123R | << 5 |
| | | | K147E | Q124K | |

Results in Table 6 and Figure 6 show that by substituting two charged amino acids with the opposite charge in the CH1 domain (K213E or K213D, K147E) as well as in the CL domain (E123K or E123R, Q124K) in the "uncrossed" side, as it is described for the invention in further detail above, and introducing an additional charged amino acid in the CL domain in the "crossed" side, formation of the main side product (a non functional heavy chain dimer) is strongly reduced as compared to the wild type trispecific antibody without such substitutions.

### Example 2A

### Production and expression of bivalent, bispecific antibodies which bind to Angiopoietin-2 (ANG2) and Vascular endothelial growth factor (VEGF), with VL-VH/CL-CH1 domain exchange (CrossMAb^{Fab}) in one binding arm, with charged amino acid substitutions in the CH1/CL interface and with charged amino acid introduction in the VH/VL interface

Bispecific antibodies which bind to human Angiopoietin-2 (ANG2) and human Vascular endothelial growth factor (VEGF) were generated as described in Example 1A. The bispecific antibodies were expressed using expression plasmids containing the nucleic acids encoding the amino acid sequences as shown in Table 7.

**Table 7: Amino acid sequences of light chains (LC) and heavy chains (HC) of anti-Ang2-VEGF bispecific antibody Ang2VEGF-044 which includes additional charged amino acids introduced in both VH/VL interfaces**

| **Antibody** | **VEGF_HC*** | **VEGF_LC*** | **Ang2_HC** | **Ang2_LC** |
|---|---|---|---|---|
| Ang2VEGF_044 | SEQ ID NO: 8 | SEQ ID NO: 9 | SEQ ID NO: 5 | SEQ ID NO: 6 |

In this construct knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349'C) (contained in the respective corresponding heavy chain (HC) sequences depicted above).

### Example 2B

### Purification and characterization of bivalent, bispecific antibodies which bind to ANG2 and VEGF, with VL-VH/CL-CH1 domain exchange (CrossMAb^{Fab}) in one binding arm, with two charged amino acid substitutions in the CH1/CL interface and with charged amino acid introduction in the VH/VL interface

The bispecific antibodies expressed above in example 1A (Ang2VEGF-0289 [wt], Ang2VEGF-280, Ang2VEGF-111) and example 2A (Ang2VEGF_044) were purified from the supernatant by a combination of Protein A affinity chromatography and size exclusion chromatography. All bispecific antibodies can be produced in good yields and are stable. The obtained products were characterized for identity by mass spectrometry and analytical properties such as purity by SDS-PAGE, monomer content and stability.

The expected primary structures were analyzed by electrospray ionization mass spectrometry (ESI-MS) of the deglycosylated intact CrossMAbs and in special cases of the deglycosylated/limited LysC digested CrossMAbs as described in the general methods section.

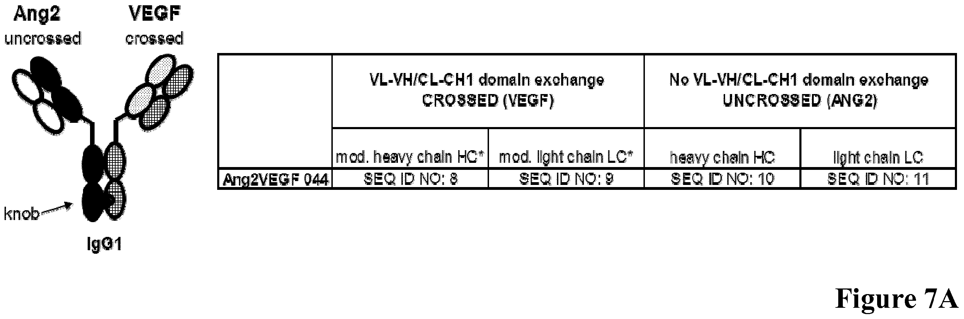

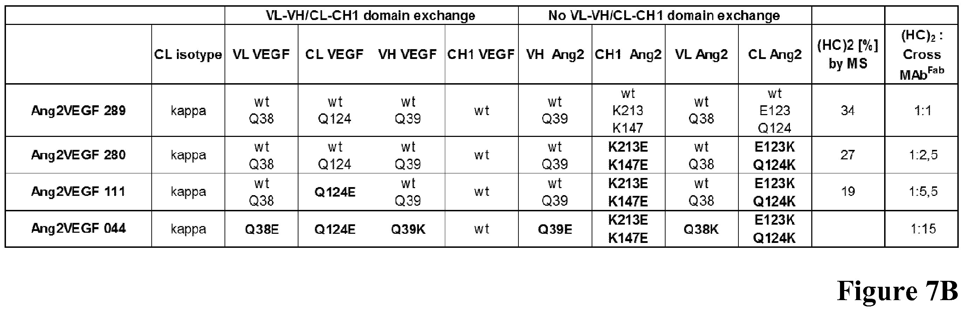

Results are shown in Table 8 and Figure 7.

**Table 8: Reduction of non functional heavy chain dimer (HC)₂ formation as side product by charged amino acid substitutions in the CH1/CL interface with additional charged amino acid introduction in the VH/VL interface**

| **Antibody** | **VEGF_HC*** | **VEGFLC*** | **Ang2_HC** | **Ang2_LC** | **(HC)₂ [%] by MS** | **(HC)₂ : CrossMAb^{Fab}** |
|---|---|---|---|---|---|---|
| Ang2VEGF_289 | wt | wt | wt | wt | 34 | 1:1 |
| Ang2VEGF_280 | wt | wt | K213E | E123K | 27 | 1:2,5 |
| | | | K147E | Q124K | | |
| Ang2VEGF_111 | Q124E | wt | K213E | E123K | 19 | 1:5,5 |
| | | | K147E | Q124K | | |
| Ang2VEGF_044 | Q124E | Q39K | K213E | E123K | *n.d. ** | 1:15 |
| | Q38E | | K147E | Q124K | | |
| | | | Q39E | Q38K | | |

| | | | | | | |
|---|---|---|---|---|---|---|
| * ... *n.d. = not determined* | | | | | | |

Results in Table 8 and Figure 7 show that by introducing a charged amino acid in the VH domain and an amino acid with the opposite charge in the VL domain in the "crossed" side as well as in the "uncrossed" side the formation of the side product can be reduced even further (tested antibodies Ang2VEGF-0289 [wt], Ang2VEGF-111[without additional charges VH-VL domains], Ang2VEGF-044 [with additional charges VH-VL domains]).

### Example 3A

### Production and expression of bivalent, bispecific antibodies which bind to TWEAK and IL-17, with VL-VH/CL-CH1 domain exchange (CrossMAb^{Fab}) in one binding arm, with charged amino acid substitutions in the CH1/CL interface and with charged amino acid introduction in the VH/VL interface

Bispecific antibodies which bind to human TWEAK and human IL-17 were generated as described in Example 1C. The bispecific antibodies were expressed using expression plasmids containing the nucleic acids encoding the amino acid sequences as shown in **Table 9.**

**Table 9: Amino acid sequences of light chains (LC) and heavy chains (HC) of anti-TWEAK-IL17 bispecific antibody TweakIL17_0128 which includes charged amino acids introduced in both VH/VL interfaces**

| **Antibody** | **IL-17_HC*** | **IL-17_LC*** | **Tweak_HC** | **Tweak_LC** |
|---|---|---|---|---|
| TweakIL17_0128 | SEQ ID NO: 42 | SEQ ID NO:43 | SEQ ID NO:44 | SEQ ID NO:45 |

In this construct knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349C) (contained in the respective corresponding heavy chain (HC) sequences depicted above).

### Example 3B

### Purification and characterization of bivalent, bispecific antibodies which bind to TWEAK and IL-17, with VL-VH/CL-CH1 domain exchange (CrossMAb^{Fab}) in one binding arm, with charged amino acid substitutions in the CH1/CL interface and with charged amino acid introduction in the VH/VL interface

The bispecific antibodies expressed above in example 1C and example 3A were purified from the supernatant by a combination of Protein A affinity chromatography and size exclusion chromatography. All bispecific antibodies can be produced in good yields and are stable. Results are shown in Table 10.

**Table 10: Yield and fraction of desired antibody after Protein A Purification**

| **Antibody** | **Tweak_HC** | **Tweak_LC** | **IL-17_HC** | **IL-17_LC** | **Yield [mg]** | **monomer [%]*** | **HC₂ [%] by CE-SDS** |
|---|---|---|---|---|---|---|---|
| TweakIL17_0101 | wt | wt | wt | wt | 12,2 | 62,1 | 25 |
| TweakIL17_0102 | wt | wt | K213E | E123K | 11,1 | 73,6 | 12 |
| | | | K147E | Q124K | | | |
| TweakIL17_0103 | Q124E | wt | K213E | E123K | 18,5 | 73,8 | 16 |
| | | | K147E | Q124K | | | |
| TweakIL17_0128 | Q124E | Q39K | K213E | E123K | 11,4 | 83,4 | 0 |
| | Q38E | | K147E | Q124K | | | |
| | | | Q39E | Q38K | | | |

| | | | | | | | |
|---|---|---|---|---|---|---|---|
| * ... *fraction of desired bispecific antibody as analyzed via analytical SEC after Protein A purification* | | | | | | | |

In order to assess stability of the antibody constructs, the aggregation onset temperatures was assessed.

Results are shown in **Table 11.**

**Table 11: Aggregation onset temperature (T_{agg}) of indicated antibodies**

| **Sample** | **T_{agg} (°C)** |
|---|---|
| TweakIL17_ 0101 | 54.8 |
| TweakIL17_0102 | 52.4 |
| TweakIL17_0103 | 53.0 |
| TweakIL17_0128 | 51.6 |

Antigen binding of antibody TweakIL17_0128 was assessed as described in Example 1D:
KD (TWEAK) = < 0.1 nM
KD (IL-17) = 0.1 nM

### Example 4A

### Production and expression of bivalent, bispecific antibodies which bind to Angiopoietin-2 (ANG2) and Vascular endothelial growth factor (VEGF) that comprise a wild type CH3 domain, with VL-VH/CL-CH1 domain exchange (CrossMAb^{Fab}) in one binding arm, with charged amino acid substitutions in the CH1/CL interface

To assess the influence of heavy chain heterodimerization strategies on the side product profile of indicated antibodies, antibodies lacking CH3-modifications were generated.

Bispecific antibodies which bind to human Angiopoietin-2 (ANG2) and human Vascular endothelial growth factor (VEGF) were generated as described in Example 1A. The bispecific antibodies were expressed using expression plasmids containing the nucleic acids encoding the amino acid sequences as shown in Table 12.

**Table 12: Amino acid sequences of light chains (LC) and heavy chains (HC) of indicated anti-Ang2-VEGF bispecific antibodies**

| **Antibody** | **VEGF_HC*** | **VEGF_LC*** | **Ang2_HC** | **Ang2_LC** |
|---|---|---|---|---|
| Ang2VEGF_467 | SEQ ID NO: 46 | SEQ ID NO: 2 | SEQ ID NO: 47 | SEQ ID NO: 6 |
| Ang2VEGF_468 | SEQ ID NO: 48 | SEQ ID NO: 2 | SEQ ID NO: 47 | SEQ ID NO: 6 |

In these constructs wild type CH3 domains were used.

### Example 4B

### Purification and characterization of bivalent, bispecific antibodies which bind to Angiopoietin-2 (ANG2) and Vascular endothelial growth factor (VEGF) that comprise a wild type CH3 domain, with VL-VH/CL-CH1 domain exchange (CrossMAb^{Fab}) in one binding arm, with charged amino acid substitutions in the CH1/CL interface

The bispecific antibodies expressed above in example 4A were purified from the supernatant by a combination of Protein A affinity chromatography and size exclusion chromatography. The antibodies can be produced in good yields and are stable. Heavy chain dimer side product was analyzed via CE-SDS indicating that comparable ratios of heavy chain dimer are formed in the constructs with and without knobs-into-holes modifications within the CH3/CH3 interface, indicating that the effect of the introduction of charged amino acids in the CH1/CL interface on reduction of side products formed due to light chain mispairing (i.e. mispairing of the wrong light chain with the wrong heavy chain) occurs independently of the presence of mutations supporting the correct assembly of heavy chains (here: knobs-into-holes modifications within the CH3/CH3 interface were used to suppress heavy chain - heavy chain mispairing, i.e. undesired pairing of two identical heavy chains).

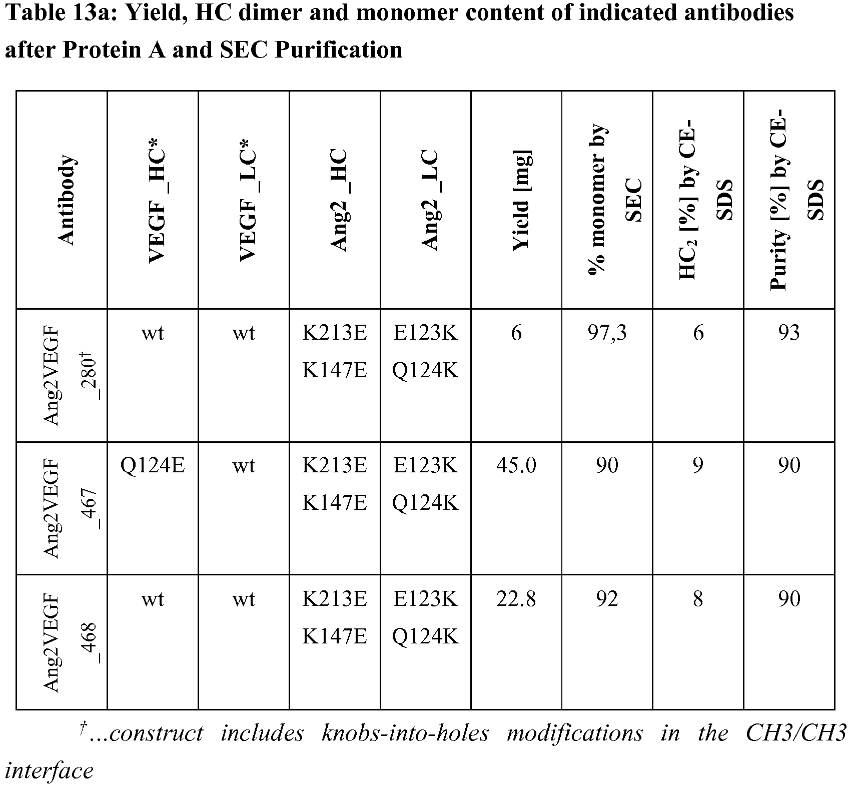

As expected, constructs lacking CH3-modifications give rise to side products caused by heavy chain - heavy chain mispairing, such as the monospecific anti-Ang2 antibody and heavy chain homodimers. The side product profile of the constructs was quantified after enzymatic deglycosylation by MS under denaturing and non reducing conditions. The relative content of an Ang2 or a VEGF specific monospecific antibody, the heavy chain homo- and hetero-dimer, as well as the content of the desired bispecific full size antibody was measured. Samples were analyzed after Protein A and SEC purification.

**Table 13b: Influence of heavy chain heterodimerization strategies on side product profile of indicated bispecific antibodies of the invention**

| **Antibody** | **Ang2 MAb by MS [%]** | **VEGF MAb by MS [%]** | **desired molecule by MS [%]** | **HC hetero-dimer [%]** | **HC-VEGF dimer [%]** |
|---|---|---|---|---|---|
| Ang2VEGF_280 | 2 | not detected | 95 | 3 | - |
| Ang2VEGF_467 | 52 | not detected | 36 | 7 | 5 |
| Ang2VEGF_468 | 56 | not detected | 33 | 6 | 5 |

**Antigen binding** was assessed via BIACORE® as described above in the material and methods section.

**Table 14a: Affinity for VEGF of indicated antibodies**

| **Sample** | **KD (nM)** |
|---|---|
| Ang2VEGF_280 | 7 |
| Ang2VEGF_467 | 17 |
| Ang2VEGF_468 | 22 |

**Table 14b: Affinity for ANG2 of indicated antibodies**

| **Sample** | **App.KD (nM)** |
|---|---|
| Ang2VEGF_280 | 3 |
| Ang2VEGF_467 | 0.5 |
| Ang2VEGF_468 | 0.5 |

In order to assess thermal **stability** of the antibody constructs, the aggregation onset temperatures was assessed.

Results are shown in **Table 14c.**

**Table 14c: Aggregation onset temperature (T_{agg}) of indicated antibodies**

| **Sample** | **T_{agg} (°C)** |
|---|---|
| Ang2VEGF_280 | 61.0 |
| Ang2VEGF_467 | 61.0 |
| Ang2VEGF_468 | 61.0 |

### Example 5A

### Production and expression of bivalent, bispecific antibodies which bind to Angiopoietin-2 (ANG2) and Vascular endothelial growth factor (VEGF), with VL-VH/CL-CH1 domain exchange (CrossMAb^{Fab}) and charged amino acid substitutions in the CH1/CL interface in the same binding arm

Bispecific antibodies which bind to human Angiopoietin-2 (ANG2) and human Vascular endothelial growth factor (VEGF) were generated as described in Example 1A. The bispecific antibodies were expressed using expression plasmids containing the nucleic acids encoding the amino acid sequences as shown in Table 15.

**Table 15: Amino acid sequences of light chains (LC) and heavy chains (HC) of indicated anti-Ang2-VEGF bispecific antibodies**

| **Antibody** | **VEGF_HC*** | **VEGF_LC*** | **Ang2_HC** | **Ang2_LC** |
|---|---|---|---|---|
| Ang2VEGF_469 | SEQ ID NO: 49 | SEQ ID NO: 50 | SEQ ID NO: 3 | SEQ ID NO: 4 |
| Ang2VEGF_470 | SEQ ID NO: 49 | SEQ ID NO: 50 | SEQ ID NO: 3 | SEQ ID NO: 51 |

For all constructs knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349'C) (contained in the respective corresponding heavy chain (HC) sequences depicted above).

### Example 5B

### Purification and characterization of bivalent, bispecific antibodies which bind to Angiopoietin-2 (ANG2) and Vascular endothelial growth factor (VEGF), with VL-VH/CL-CH1 domain exchange (CrossMAb^{Fab}) and charged amino acid substitutions in the CH1/CL interface in the same binding arm

The bispecific antibodies expressed above in example 5A were purified from the supernatant by a combination of Protein A affinity chromatography and size exclusion chromatography. The antibodies can be produced in good yields and are stable.

**Table 16a: Yield and monomer content of indicated antibodies after Protein A and SEC Purification**

| **Antibody** | **VEGF_HC*** | **VEGF_LC*** | **Ang2_HC** | **Ang2_LC** | **Yield [mg]** | **% monomer by SEC** |
|---|---|---|---|---|---|---|
| Ang2VEGF_469 | E123K | K213E | wt | wt | 46.4 | 90 |
| | Q124K | K147E | | | | |
| Ang2VEGF_470 | E123K | K213E | wt | Q124E | 46.4 | 90 |
| | Q124K | K147E | | | | |

**Table 16b: Side products of indicated antibodies after Protein A and SEC Purification**

| **Antibody** | **Purity oft he desired product by CE-SDS [%]** | **HC₂ [%] by CE-SDS** | **HC₂ [%] by MS** |
|---|---|---|---|
| Ang2VEGF_469 | 86 | 11 | 4 (3^{#}) |
| Ang2VEGF_470 | 89 | 7 | 3 (2^{#}) |

| | | | |
|---|---|---|---|
| # ... *in addition to the HC hetero-dimer about x % VEGF-HC homo-dimer were observed.* | | | |

**Antigen binding** was assessed via BIACORF® as described above.

**Table 17a: Affinity for VEGF of indicated antibodies**

| **Sample** | **KD (nM)** |
|---|---|
| Ang2VEGF_469 | 13 |
| Ang2VEGF_470 | 12 |

**Table 17b: Affinity for ANG2 of indicated antibodies**

| **Sample** | **App.KD (nM)** |
|---|---|
| Ang2VEGF_469 | 3 |
| Ang2VEGF_470 | 3 |

In order to assess **stability** of the antibody constructs, the aggregation onset temperatures was assessed.

Results are shown in **Table 17c.**

**Table 17c: Aggregation onset temperature (T_{agg}) of indicated antibodies**

| **Sample** | **T_{agg} (°C)** |
|---|---|
| Ang2VEGF_469 | 59 |
| Ang2VEGF_470 | 59 |

## Claims

1. A multispecific antibody, comprising
a) a first light chain and a first heavy chain derived from a first antibody which specifically binds to a first antigen; and
b) a second light chain and a second heavy chain derived from a second antibody which specifically binds to a second antigen, wherein
in the second light chain the variable domain VL is replaced by the variable domain VH of the second heavy chain and the constant domain CL is replaced by the constant domain CH1 of the second heavy chain; and
in the second heavy chain the variable domain VH is replaced by the variable domain VL of the second light chain and the constant domain CH1 is replaced by the constant domain CL of the second light chain; and
i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D; or
ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.

2. The multispecific antibody according to claim 1, wherein
i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D, and
in the constant domain CL of the second heavy chain the amino acid at position 124 (numbering according to Kabat) is substituted by an amino acid selected from E and D; or
ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D, and
in the constant domain CL of the first light chain the amino acid at position 124 (numbering according to Kabat) is substituted by an amino acid selected from E and D.

3. The multispecific antibody according to any one of the preceding claims,
iii) wherein in the variable domain VL of the first light chain the amino acid at position 38 (numbering according to Kabat) is substituted by an amino acid selected from K, R and H; and
wherein in the variable domain VH of the first heavy chain the amino acid at position 39 (numbering according to Kabat) is substituted by an amino acid selected from E and D; and
iv) wherein in the variable domain VL of the second heavy chain the amino acid at position 38 (numbering according to Kabat) is substituted by an amino acid selected from E and D (in one preferred embodiment E); and wherein in the variable domain VH of the second light chain the amino acid at position 39 (numbering according to Kabat) is substituted by an amino acid selected from K, R and H (in one preferred embodiment by K or R, in one embodiment by K).

4. The multispecific antibody according to any one of the preceding claims, wherein
- in the CH3 domain of one heavy chain the amino acid T at position 366 is substituted by W; and in the CH3 domain of the other heavy chain the amino acid T at position 366 is substituted by S, the amino acid L at position 368 is substituted by A and the amino acid Y at position 407 is substituted by V (numberings according to EU index of Kabat); or
- in the CH3 domain of one heavy chain the amino acid T at position 366 is substituted by W, the amino acid R at position 409 is substituted by D, and the amino acid K at position 370 is substituted by E; and in the CH3 domain of the other heavy chain the amino acid T at position 366 is substituted by S, the amino acid L at position 368 is substituted by A and the amino acid Y at position 407 is substituted by V, the amino acid D at position 399 is substituted by K, and the amino acid E at position 357 is substituted by K (numberings according to EU index of Kabat).

5. The multispecific antibody according to claim 4, wherein in the CH3 domain of one heavy chain either the amino acid E at position 356 or the amino acid S at position 354 is substituted by C and in the CH3 domain of the other heavy chain the amino acid Y at position 349 is substituted by C (numberings according to EU index of Kabat).

6. The multispecific antibody according to any one of the preceding claims that specifically binds to human Angiopoietin-2 and human VEGF, wherein
a) the antibody comprises a variable heavy chain domain according to SEQ ID NO: 32 and a variable light chain domain according to SEQ ID NO: 33; and
b) the antibody comprises a variable heavy chain domain according to SEQ ID NO: 31 and a variable light chain domain according to SEQ ID NO: 30.

7. The multispecific antibody according to any one of claims 1 to 5 that specifically binds to human TWEAK and human IL17, wherein
a) the antibody comprises a variable heavy chain domain according to SEQ ID NO: 36 and a variable light chain domain according to SEQ ID NO: 37; and
b) the antibody comprises a variable heavy chain domain according to SEQ ID NO: 35 and a variable light chain domain according to SEQ ID NO: 34.

8. The multispecific antibody according to any one of claims 1 to 5 that specifically binds to human Herl, human Her3 and human cMet, wherein
a) the antibody comprises a variable heavy chain domain according to SEQ ID NO: 40 and a variable light chain domain according to SEQ ID NO: 41; and
b) the antibody comprises a variable heavy chain domain according to SEQ ID NO: 39 and a variable light chain domain according to SEQ ID NO: 38.

9. A method for the preparation of a multispecific antibody according to any one of claims 1 to 8, comprising the steps of
- transforming an isolated host cell with vectors comprising nucleic acids encoding
a) the first light chain as defined in one of claims 1 to 8 derived from a first antibody which specifically binds to a first antigen;
b) the first heavy chain as defined in one of claims 1 to 8 derived from a first antibody which specifically binds to a first antigen;
c) the second light chain as defined in one of claims 1 to 8 derived from a second antibody which specifically binds to a second antigen; and
d) the second heavy chain as defined in one of claims 1 to 8 derived from a second antibody which specifically binds to a second antigen,
- culturing said host cell under conditions that allow synthesis of said multispecific antibody; and
- recovering said multispecific antibody from said host cell culture.

10. A nucleic acid encoding the antibody according to any one of claims 1 to 8.

11. A vector comprising a nucleic acid according to claim 10, wherein the vector is capable of expressing said nucleic acid in a host cell.

12. An isolated host cell comprising
a) a vector comprising nucleic acids encoding the first light chain as defined in one of claims 1 to 8 derived from a first antibody which specifically binds to a first antigen;
b) a vector comprising nucleic acids encoding the first heavy chain as defined in one of claims 1 to 8 derived from a first antibody which specifically binds to a first antigen;
c) a vector comprising nucleic acids encoding the second light chain as defined in one of claims 1 to 8 derived from a second antibody which specifically binds to a second antigen; and
d) a vector comprising nucleic acids encoding the second heavy chain as defined in one of claims 1 to 8 derived from a second antibody which specifically binds to a second antigen.

13. A pharmaceutical composition comprising a multispecific antibody according to any one of claims 1 to 8 in combination with at least one pharmaceutically acceptable carrier.

14. A multispecific antibody according to according to any one of claims 1 to 8 for use as a medicament.

## Patentansprüche

1. Multispezifischer Antikörper, umfassend
a) eine erste leichte Kette und eine erste schwere Kette, abgeleitet von einem ersten Antikörper, der spezifisch an ein erstes Antigen bindet; und
b) eine zweite leichte Kette und eine zweite schwere Kette, abgeleitet von einem zweiten Antikörper, der spezifisch an ein zweites Antigen bindet, wobei
in der zweiten leichten Kette die variable Domäne VL durch eine variable Domäne VH der zweiten schweren Kette ersetzt ist und die konstante Domäne CL durch die konstante Domäne CH1 der zweiten schweren Kette ersetzt ist; und
in der zweiten schweren Kette die variable Domäne VH durch die variable Domäne VL der zweiten leichten Kette ersetzt ist und die konstante Domäne CH1 durch die konstante Domäne CL der zweiten leichten Kette ersetzt ist; und
i) wobei in der konstanten Domäne CL der ersten leichten Kette die Aminosäuren an Position 124 und 123 (Nummerierung nach Kabat) unabhängig voneinander substituiert sind durch eine Aminosäure, ausgewählt aus K, R und H; und wobei in der konstanten Domäne CH1 der ersten schweren Kette die Aminosäuren an Position 147 und 213 (Nummerierung nach EU-Index von Kabat) unabhängig voneinander substituiert sind durch eine Aminosäure, ausgewählt aus E oder D; oder
ii) wobei in der konstanten Domäne CL der zweiten schweren Kette die Aminosäuren an Position 124 und 123 (Nummerierung nach Kabat) unabhängig voneinander substituiert sind durch eine Aminosäure, ausgewählt aus K, R und H; und wobei in der konstanten Domäne CH1 der zweiten leichten Kette die Aminosäuren an Position 147 und 213 (Nummerierung nach EU-Index von Kabat) unabhängig voneinander substituiert sind durch eine Aminosäure, ausgewählt aus E oder D.

2. Multispezifischer Antikörper nach Anspruch 1, wobei
i) wobei in der konstanten Domäne CL der ersten leichten Kette die Aminosäuren an Position 124 und 123 (Nummerierung nach Kabat) unabhängig voneinander substituiert sind durch eine Aminosäure, ausgewählt aus K, R und H; und wobei in der konstanten Domäne CH1 der ersten schweren Kette die Aminosäuren an Position 147 und 213 (Nummerierung nach EU-Index von Kabat) unabhängig voneinander substituiert sind durch eine Aminosäure, ausgewählt aus E oder D, und
in der konstanten Domäne CL der zweiten schweren Kette die Aminosäure an Position 124 (Nummerierung nach Kabat) substituiert ist durch eine Aminosäure, ausgewählt aus E und D; oder
ii) wobei in der konstanten Domäne CL der zweiten schweren Kette die Aminosäuren an Position 124 und 123 (Nummerierung nach Kabat) unabhängig voneinander substituiert sind durch eine Aminosäure, ausgewählt aus K, R und H; und wobei in der konstanten Domäne CH1 der zweiten leichten Kette die Aminosäuren an Position 147 und 213 (Nummerierung nach EU-Index von Kabat) unabhängig voneinander substituiert sind durch eine Aminosäure, ausgewählt aus E oder D, und
in der konstanten Domäne CL der ersten leichten Kette die Aminosäure an Position 124 (Nummerierung nach Kabat) substituiert ist durch eine Aminosäure, ausgewählt aus E und D.

3. Multispezifischer Antikörper nach einem der vorstehenden Ansprüche,
iii) wobei in der variablen Domäne VL der ersten leichten Kette die Aminosäure an Position 38 (Nummerierung nach Kabat) substituiert ist durch eine Aminosäure, ausgewählt aus K, R und H; und
wobei in der variablen Domäne VH der ersten schweren Kette die Aminosäure an Position 39 (Nummerierung nach Kabat) substituiert ist durch eine Aminosäure, ausgewählt aus E und D; und
iv) wobei in der variablen Domäne VL der zweiten schweren Kette die Aminosäure an Position 38 (Nummerierung nach Kabat) substituiert ist durch eine Aminosäure, ausgewählt aus E und D (in einer bevorzugten Ausführungsform E); und
wobei in der variablen Domäne VH der zweiten leichten Kette die Aminosäure an Position 39 (Nummerierung nach Kabat) substituiert ist durch eine Aminosäure, ausgewählt aus K, R und H (in einer bevorzugten Ausführungsform durch K oder R, in einer Ausführungsform durch K).

4. Multispezifischer Antikörper nach einem der vorstehenden Ansprüche, wobei
- in der CH3-Domäne einer schweren Kette die Aminosäure T an Position 366 substituiert ist durch W; und in der CH3-Domäne der anderen schweren Kette die Aminosäure T an Position 366 substituiert ist durch S, die Aminosäure L an Position 368 substituiert ist durch A und die Aminosäure Y an Position 407 substituiert ist durch V (Nummerierungen nach EU-Index von Kabat); oder
- in der CH3-Domäne einer schweren Kette die Aminosäure T an Position 366 substituiert ist durch W, die Aminosäure R an Position 409 substituiert ist durch D und die Aminosäure K an Position 370 substituiert ist durch E; und in der CH3-Domäne der anderen schweren Kette die Aminosäure T an Position 366 substituiert ist durch S, die Aminosäure L an Position 368 substituiert ist durch A und die Aminosäure Y an Position 407 substituiert ist durch V, die Aminosäure D an Position 399 substituiert ist durch K und die Aminosäure E an Position 357 substituiert ist durch K (Nummerierungen nach EU-Index von Kabat).

5. Multispezifischer Antikörper nach Anspruch 4, wobei in der CH3-Domäne einer schweren Kette entweder die Aminosäure E an Position 356 oder die Aminosäure S an Position 354 substituiert ist durch C und in der CH3-Domäne der anderen schweren Kette die Aminosäure Y an Position 349 substituiert ist durch C (Nummerierungen nach EU-Index von Kabat).

6. Multispezifischer Antikörper nach einem der vorstehenden Ansprüche, der spezifisch an humanes Angiopoetin-2 und humanen VEGF bindet, wobei
a) der Antikörper eine variable Domäne der schweren Kette gemäß SEQ ID NO:32 und eine variable Domäne der leichten Kette gemäß SEQ ID NO:33 umfasst; und
b) der Antikörper eine variable Domäne der schweren Kette gemäß SEQ ID NO:31 und eine variable Domäne der leichten Kette gemäß SEQ ID NO:30 umfasst.

7. Multispezifischer Antikörper nach einem der Ansprüche 1 bis 5, der spezifisch an humanes TWEAK und humanes IL17 bindet, wobei
a) der Antikörper eine variable Domäne der schweren Kette gemäß SEQ ID NO:36 und eine variable Domäne der leichten Kette gemäß SEQ ID NO:37 umfasst; und
b) der Antikörper eine variable Domäne der schweren Kette gemäß SEQ ID NO:35 und eine variable Domäne der leichten Kette gemäß SEQ ID NO:34 umfasst.

8. Multispezifischer Antikörper nach einem der Ansprüche 1 bis 5, der spezifisch an humanes Her1, humanes Her3 und humanes cMet bindet, wobei
a) der Antikörper eine variable Domäne der schweren Kette gemäß SEQ ID NO:40 und eine variable Domäne der leichten Kette gemäß SEQ ID NO:41 umfasst; und
b) der Antikörper eine variable Domäne der schweren Kette gemäß SEQ ID NO:39 und eine variable Domäne der leichten Kette gemäß SEQ ID NO:38 umfasst.

9. Verfahren zur Herstellung eines multispezifischen Antikörpers nach einem der Ansprüche 1 bis 8, umfassend die folgenden Schritte
- Transformieren einer isolierten Wirtszelle mit Vektoren, umfassend Nukleinsäuren, die Folgendes codieren
a) die erste leichte Kette, wie in einem der Ansprüche 1 bis 8 definiert, abgeleitet von einem ersten Antikörper, der spezifisch an ein erstes Antigen bindet;
b) die erste schwere Kette, wie in einem der Ansprüche 1 bis 8 definiert, abgeleitet von einem ersten Antikörper, der spezifisch an ein erstes Antigen bindet;
c) die zweite leichte Kette, wie in einem der Ansprüche 1 bis 8 definiert, abgeleitet von einem zweiten Antikörper, der spezifisch an ein zweites Antigen bindet; und
d) die zweite schwere Kette, wie in einem der Ansprüche 1 bis 8 definiert, abgeleitet von einem zweiten Antikörper, der spezifisch an ein zweites Antigen bindet;
- Kultivieren der Wirtszelle unter Bedingungen, die die Synthese des multispezifischen Antikörpers ermöglichen; und
- Gewinnen des multispezifischen Antikörpers aus der Wirtszellkultur.

10. Nukleinsäure, die den Antikörper nach einem der Ansprüche 1 bis 8 codiert.

11. Vektor, umfassend eine Nukleinsäure nach Anspruch 10, wobei der Vektor die Nukleinsäure in einer Wirtszelle exprimieren kann.

12. Isolierte Wirtszelle, umfassend
a) einen Vektor, umfassend Nukleinsäuren, die die erste leichte Kette, wie in einem der Ansprüche 1 bis 8 definiert, abgeleitet von einem ersten Antikörper, der spezifisch an ein erstes Antigen bindet, codieren;
b) einen Vektor, umfassend Nukleinsäuren, die die erste schwere Kette, wie in einem der Ansprüche 1 bis 8 definiert, abgeleitet von einem ersten Antikörper, der spezifisch an ein erstes Antigen bindet, codieren;
c) einen Vektor, umfassend Nukleinsäuren, die die zweite leichte Kette, wie in einem der Ansprüche 1 bis 8 definiert, abgeleitet von einem zweiten Antikörper, der spezifisch an ein zweites Antigen bindet, codieren; und
d) einen Vektor, umfassend Nukleinsäuren, die die zweite schwere Kette, wie in einem der Ansprüche 1 bis 8 definiert, abgeleitet von einem zweiten Antikörper, der spezifisch an ein zweites Antigen bindet, codieren.

13. Pharmazeutische Zusammensetzung, umfassend einen multispezifischen Antikörper nach einem der Ansprüche 1 bis 8 in Kombination mit mindestens einem pharmazeutisch unbedenklichen Träger.

14. Multispezifischer Antikörper nach einem der Ansprüche 1 bis 8 zur Verwendung als ein Medikament.

## Revendications

1. Anticorps multi-spécifique, comprenant
a) une première chaîne légère et une première chaîne lourde dérivées d'un premier anticorps qui se lie spécifiquement à un premier antigène ; et
b) une seconde chaîne légère et une seconde chaîne lourde dérivées d'un second anticorps qui se lie spécifiquement à un second antigène, dans lequel
dans la seconde chaîne légère le domaine variable VL est remplacé par le domaine variable VH de la seconde chaîne lourde et le domaine constant CL est remplacé par le domaine constant CH1 de la seconde chaîne lourde ; et
dans la seconde chaîne lourde le domaine variable VH est remplacé par le domaine variable VL de la seconde chaîne légère et le domaine constant CH1 est remplacé par le domaine constant CL de la seconde chaîne légère ; et
i) dans lequel dans le domaine constant CL de la première chaîne légère les acides aminés en positions 124 et 123 (numérotation selon Kabat) sont substitués indépendamment l'un de l'autre par un acide aminé choisi parmi K, R et H ; et dans lequel dans le domaine constant CH1 de la première chaîne lourde les acides aminés en positions 147 et 213 (numérotation selon l'indice EU de Kabat) sont substitués indépendamment l'un de l'autre par un acide aminé choisi parmi E ou D ; ou
ii) dans lequel dans le domaine constant CL de la seconde chaîne lourde les acides aminés en positions 124 et 123 (numérotation selon Kabat) sont substitués indépendamment l'un de l'autre par un acide aminé choisi parmi K, R et H ; et dans lequel dans le domaine constant CH1 de la seconde chaîne légère les acides aminés en positions 147 et 213 (numérotation selon l'indice EU de Kabat) sont substitués indépendamment l'un de l'autre par un acide aminé choisi parmi E ou D.

2. Anticorps multi-spécifique selon la revendication 1, dans lequel
i) dans lequel dans le domaine constant CL de la première chaîne légère les acides aminés en positions 124 et 123 (numérotation selon Kabat) sont substitués indépendamment l'un de l'autre par un acide aminé choisi parmi K, R et H ; et dans lequel dans le domaine constant CH1 de la première chaîne lourde les acides aminés en positions 147 et 213 (numérotation selon l'indice EU de Kabat) sont substitués indépendamment l'un de l'autre par un acide aminé choisi parmi E ou D, et
dans le domaine constant CL de la seconde chaîne lourde l'acide aminé en position 124 (numérotation selon Kabat) est substitué par un acide aminé choisi parmi E et D ; ou
ii) dans lequel dans le domaine constant CL de la seconde chaîne lourde les acides aminés en positions 124 et 123 (numérotation selon Kabat) sont substitués indépendamment l'un de l'autre par un acide aminé choisi parmi K, R et H ; et dans lequel dans le domaine constant CH1 de la seconde chaîne légère les acides aminés en positions 147 et 213 (numérotation selon l'indice EU de Kabat) sont substitués indépendamment l'un de l'autre par un acide aminé choisi parmi E ou D, et
dans le domaine constant CL de la première chaîne légère l'acide aminé en position 124 (numérotation selon Kabat) est substitué par un acide aminé choisi parmi E et D.

3. Anticorps multi-spécifique selon l'une quelconque des revendications précédentes,
iii) dans lequel dans le domaine variable VL de la première chaîne légère l'acide aminé en position 38 (numérotation selon Kabat) est substitué par un acide aminé choisi parmi K, R et H ; et
dans lequel dans le domaine variable VH de la première chaîne lourde l'acide aminé en position 39 (numérotation selon Kabat) est substitué par un acide aminé choisi parmi E et D ; et
iv) dans lequel dans le domaine variable VL de la seconde chaîne lourde l'acide aminé en position 38 (numérotation selon Kabat) est substitué par un acide aminé choisi parmi E et D (dans un mode de réalisation préféré E) ; et
dans lequel dans le domaine variable VH de la seconde chaîne légère l'acide aminé en position 39 (numérotation selon Kabat) est substitué par un acide aminé choisi parmi K, R et H (dans un mode de réalisation préféré par K ou R, dans un mode de réalisation par K).

4. Anticorps multi-spécifique selon l'une quelconque des revendications précédentes, dans lequel
- dans le domaine CH3 d'une chaîne lourde l'acide aminé T en position 366 est substitué par W ; et dans le domaine CH3 de l'autre chaîne lourde l'acide aminé T en position 366 est substitué par S, l'acide aminé L en position 368 est substitué par A et l'acide aminé Y en position 407 est substitué par V (numérotations selon l'indice EU de Kabat) ; ou
- dans le domaine CH3 d'une chaîne lourde l'acide aminé T en position 366 est substitué par W, l'acide aminé R en position 409 est substitué par D et l'acide aminé K en position 370 est substitué par E ; et dans le domaine CH3 de l'autre chaîne lourde l'acide aminé T en position 366 est substitué par S, l'acide aminé L en position 368 est substitué par A et l'acide aminé Y en position 407 est substitué par V, l'acide aminé D en position 399 est substitué par K et l'acide aminé E en position 357 est substitué par K (numérotations selon l'indice EU de Kabat).

5. Anticorps multi-spécifique selon la revendication 4, dans lequel dans le domaine CH3 d'une chaîne lourde soit l'acide aminé E en position 356 soit l'acide aminé S en position 354 est substitué par C et dans le domaine CH3 de l'autre chaîne lourde l'acide aminé Y en position 349 est substitué par C (numérotations selon l'indice EU de Kabat).

6. Anticorps multi-spécifique selon l'une quelconque des revendications précédentes qui se lie spécifiquement à l'angiopoïétine-2 humaine et au VEGF humain, dans lequel
a) l'anticorps comprend un domaine variable de chaîne lourde selon la SEQ ID NO : 32 et un domaine variable de chaîne légère selon la SEQ ID NO : 33 ; et
b) l'anticorps comprend un domaine variable de chaîne lourde selon la SEQ ID NO : 31 et un domaine variable de chaîne légère selon la SEQ ID NO : 30.

7. Anticorps multi-spécifique selon l'une quelconque des revendications 1 à 5 qui se lie spécifiquement au TWEAK humain et à l'IL17 humaine, dans lequel
a) l'anticorps comprend un domaine variable de chaîne lourde selon la SEQ ID NO : 36 et un domaine variable de chaîne légère selon la SEQ ID NO : 37 ; et
b) l'anticorps comprend un domaine variable de chaîne lourde selon la SEQ ID NO : 35 et un domaine variable de chaîne légère selon la SEQ ID NO : 34.

8. Anticorps multi-spécifique selon l'une quelconque des revendications 1 à 5 qui se lie spécifiquement au Herl humain, au Her3 humain et au cMet humain, dans lequel
a) l'anticorps comprend un domaine variable de chaîne lourde selon la SEQ ID NO : 40 et un domaine variable de chaîne légère selon la SEQ ID NO : 41 ; et
b) l'anticorps comprend un domaine variable de chaîne lourde selon la SEQ ID NO : 39 et un domaine variable de chaîne légère selon la SEQ ID NO : 38.

9. Procédé de préparation d'un anticorps multi-spécifique selon l'une quelconque des revendications 1 à 8, comprenant les étapes de
- transformation d'une cellule hôte isolée avec des vecteurs comprenant des acides nucléiques codant pour
a) la première chaîne légère telle que définie dans l'une des revendications 1 à 8 dérivée d'un premier anticorps qui se lie spécifiquement à un premier antigène ;
b) la première chaîne lourde telle que définie dans l'une des revendications 1 à 8 dérivée d'un premier anticorps qui se lie spécifiquement à un premier antigène ;
c) la seconde chaîne légère telle que définie dans l'une des revendications 1 à 8 dérivée d'un second anticorps qui se lie spécifiquement à un second antigène ; et
d) la seconde chaîne lourde telle que définie dans l'une des revendications 1 à 8 dérivée d'un second anticorps qui se lie spécifiquement à un second antigène,
- culture de ladite cellule hôte dans des conditions qui permettent la synthèse dudit anticorps multi-spécifique ; et
- récupération dudit anticorps multi-spécifique de ladite culture de cellule hôte.

10. Acide nucléique codant pour l'anticorps selon l'une quelconque des revendications 1 à 8.

11. Vecteur comprenant un acide nucléique selon la revendication 10, dans lequel le vecteur est capable d'exprimer ledit acide nucléique dans une cellule hôte.

12. Cellule hôte isolée comprenant
a) un vecteur comprenant des acides nucléiques codant pour la première chaîne légère telle que définie dans l'une des revendications 1 à 8 dérivée d'un premier anticorps qui se lie spécifiquement à un premier antigène ;
b) un vecteur comprenant des acides nucléiques codant pour la première chaîne lourde telle que définie dans l'une des revendications 1 à 8 dérivée d'un premier anticorps qui se lie spécifiquement à un premier antigène ;
c) un vecteur comprenant des acides nucléiques codant pour la seconde chaîne légère telle que définie dans l'une des revendications 1 à 8 dérivée d'un second anticorps qui se lie spécifiquement à un second antigène ; et
d) un vecteur comprenant des acides nucléiques codant pour la seconde chaîne lourde telle que définie dans l'une des revendications 1 à 8 dérivée d'un second anticorps qui se lie spécifiquement à un second antigène.

13. Composition pharmaceutique comprenant un anticorps multi-spécifique selon l'une quelconque des revendications 1 à 8 en combinaison avec au moins un véhicule pharmaceutiquement acceptable.

14. Anticorps multi-spécifique selon l'une quelconque des revendications 1 à 8 pour une utilisation comme médicament.
